# EUROPEAN PATENT SPECIFICATION

(11) **EP 2 992 086 B1**
(45) Date of publication and mention of the grant of the patent: **30.09.2020**
(21) Application number: 14791378.4
(22) Date of filing: 30.04.2014
(51) Int. Cl.: C12N 5/00, C12N 5/02, A61K 38/00, A61K 38/16, C12N 15/11, A61K 39/39, C12N 9/12

(54) **MODULATION OF REGULATORY T CELL FUNCTION VIA PROTEIN KINASE C-ETA**
MODULATION DER FUNKTION REGULATORISCHER T-ZELLEN MITHILFE VON PROTEINKINASE-C-ETA
MODULATION DE LA FONCTION RÉGULATRICE DES CELLULES T PAR L'INTERMÉDIAIRE D'UNE PROTÉINE KINASE C-ETA

(30) Priority: 30.04.2013 US 201361817754 P
(43) Date of publication of application: 09.03.2016
(73) Proprietor: La Jolla Institute for Allergy and Immunology, La Jolla, CA 92037 (US)
(72) Inventor: ALTMAN, Amnon, La Jolla California 92037 (US); KONG, Kok-Fai, San Diego California 92122 (US)
(74) Representative: Boult Wade Tennant LLP
(86) International application number: PCT/US2014/036237
(87) International publication number: WO 2014/179491

(56) References cited:
- WO-A1-2005/014612
- WO-A1-2005/014612
- WO-A1-2010/026565
- WO-A1-2010/117055
- WO-A2-2011/083481
- WO-A2-2011/083481
- WO-A2-2012/127240
- T. FISCHER ET AL: "Phase IIB Trial of Oral Midostaurin (PKC412), the FMS-Like Tyrosine Kinase 3 Receptor (FLT3) and Multi-Targeted Kinase Inhibitor, in Patients With Acute Myeloid Leukemia and High-Risk Myelodysplastic Syndrome With Either Wild-Type or Mutated FLT3", JOURNAL OF CLINICAL ONCOLOGY, vol. 28, no. 28, 23 August 2010 (2010-08-23), pages 4339-4345, XP055290517, US ISSN: 0732-183X, DOI: 10.1200/JCO.2010.28.9678
- M. A. CURRAN ET AL: "PD-1 and CTLA-4 combination blockade expands infiltrating T cells and reduces regulatory T and myeloid cells within B16 melanoma tumors", PROCEEDINGS OF THE NATIONAL ACADEMY OF SCIENCES, vol. 107, no. 9, 2 March 2010 (2010-03-02) , pages 4275-4280, XP055067204, ISSN: 0027-8424, DOI: 10.1073/pnas.0915174107
- KOK-FAI KONG ET AL: "Protein kinase C-[eta] controls CTLA-4-mediated regulatory T cell function", NATURE IMMUNOLOGY, vol. 15, no. 5, 6 April 2014 (2014-04-06), pages 465-472, XP055290520, GB ISSN: 1529-2908, DOI: 10.1038/ni.2866
- FU GUO ET AL: "Protein kinase C [eta] is required for T cell activation and homeostatic proliferation.", SCIENCE SIGNALING 06 DEC 2011, vol. 4, no. 202, 6 December 2011 (2011-12-06), page ra84, XP008182073, ISSN: 1937-9145, DOI: 10.1126/scisignal.2002058
- EMILY J QUANN ET AL: "A cascade of protein kinase C isozymes promotes cytoskeletal polarization in T cells", NATURE IMMUNOLOGY, vol. 12, no. 7, 22 May 2011 (2011-05-22), pages 647-654, XP055313295, GB ISSN: 1529-2908, DOI: 10.1038/ni.2033
- FISCHER ET AL.: 'Phase IIB Trial of Oral Midostaurin (PKC412), the FMS-Like Tyrosine Kinase 3 Receptor (FLT3) and Multi-Targeted Kinase Inhibitor, in Patients With Acute Myeloid Leukemia and High-Risk Myelodysplastic Syndrome With Either Wild-Type or Mutated FLT3.' J CLIN ONCOLOGY vol. 28, no. 28, 2010, pages 4339 - 4345, XP055290517
- CURRAN ET AL.: 'PD-1 and CTLA-4 combination blockade expands infiltrating T cells and reduces regulatory T and myeloid cells within B16 melanoma tumors.' PROC NATL ACAD SCI USA vol. 107, no. 9, 2010, pages 4275 - 80, XP055067204
- KONG ET AL.: 'Protein kinase C-[eta] controls CTLA-4-mediated regulatory T cell function.' NAT IMMUNOL. vol. 15, no. 5, 06 April 2014, pages 465 - 72, XP055290520
- CHRISTOPHE PEDROS ET AL: "Requirement of Treg-intrinsic CTLA4/PKC[eta] signaling pathway for suppressing tumor immunity", JCI INSIGHT, vol. 2, no. 23, 7 December 2017 (2017-12-07), XP055492306, ISSN: 2379-3708, DOI: 10.1172/jci.insight.95692

### SEQUENCE LISTING

<110> La Jolla Institute for Allergy and Immunology
<120> Modulation of Regulatory T Cell Function via Protein Kinase C-eta
<130> 2013-106-02
<150> US 61/817,754
   <151> 2013-04-30
<160> 10
<170> PatentIn version 3.5
<210> 1
   <211> 683
   <212> PRT
   <213> Homo sapiens
<400> 1
   Met Ser Ser Gly Thr Met Lys Phe Asn Gly Tyr Leu Arg Val Arg Ile
   1               5                   10                  15
   Gly Glu Ala Val Gly Leu Gln Pro Thr Arg Trp Ser Leu Arg His Ser
               20                  25                  30
   Leu Phe Lys Lys Gly His Gln Leu Leu Asp Pro Tyr Leu Thr Val Ser
           35                  40                  45
   Val Asp Gln Val Arg Val Gly Gln Thr Ser Thr Lys Gln Lys Thr Asn
       50                  55                  60
   Lys Pro Thr Tyr Asn Glu Glu Phe Cys Ala Asn Val Thr Asp Gly Gly
   65                  70                  75                  80
   His Leu Glu Leu Ala Val Phe His Glu Thr Pro Leu Gly Tyr Asp His
                   85                  90                  95
   Phe Val Ala Asn Cys Thr Leu Gln Phe Gln Glu Leu Leu Arg Thr Thr
               100                 105                 110
   Gly Ala Ser Asp Thr Phe Glu Gly Trp Val Asp Leu Glu Pro Glu Gly
           115                 120                 125
   Lys Val Phe Val Val Ile Thr Leu Thr Gly Ser Phe Thr Glu Ala Thr
       130                 135                 140
   Leu Gln Arg Asp Arg Ile Phe Lys His Phe Thr Arg Lys Arg Gln Arg
   145                 150                 155                 160
   Ala Met Arg Arg Arg Val His Gln Ile Asn Gly His Lys Phe Met Ala
                   165                 170                 175
   Thr Tyr Leu Arg Gln Pro Thr Tyr Cys Ser His Cys Arg Glu Phe Ile
               180                 185                 190
   Trp Gly Val Phe Gly Lys Gln Gly Tyr Gln Cys Gln Val Cys Thr Cys
           195                 200                 205
   Val Val His Lys Arg Cys His His Leu Ile Val Thr Ala Cys Thr Cys
       210                 215                 220
   Gln Asn Asn Ile Asn Lys Val Asp Ser Lys Ile Ala Glu Gln Arg Phe
   225                 230                 235                 240
   Gly Ile Asn Ile Pro His Lys Phe Ser Ile His Asn Tyr Lys Val Pro
                   245                 250                 255
   Thr Phe Cys Asp His Cys Gly Ser Leu Leu Trp Gly Ile Met Arg Gln
               260                 265                 270
   Gly Leu Gln Cys Lys Ile Cys Lys Met Asn Val His Ile Arg Cys Gln
           275                 280                 285
   Ala Asn Val Ala Pro Asn Cys Gly Val Asn Ala Val Glu Leu Ala Lys
       290                 295                 300
   Thr Leu Ala Gly Met Gly Leu Gln Pro Gly Asn Ile Ser Pro Thr Ser
   305                 310                 315                 320
   Lys Leu Val Ser Arg Ser Thr Leu Arg Arg Gln Gly Lys Glu Ser Ser
                   325                 330                 335
   Lys Glu Gly Asn Gly Ile Gly Val Asn Ser Ser Asn Arg Leu Gly Ile
               340                 345                 350
   Asp Asn Phe Glu Phe Ile Arg Val Leu Gly Lys Gly Ser Phe Gly Lys
           355                 360                 365
   Val Met Leu Ala Arg Val Lys Glu Thr Gly Asp Leu Tyr Ala Val Lys
       370                 375                 380
   Val Leu Lys Lys Asp Val Ile Leu Gln Asp Asp Asp Val Glu Cys Thr
   385                 390                 395                 400
   Met Thr Glu Lys Arg Ile Leu Ser Leu Ala Arg Asn His Pro Phe Leu
                   405                 410                 415
   Thr Gln Leu Phe Cys Cys Phe Gln Thr Pro Asp Arg Leu Phe Phe Val
               420                 425                 430
   Met Glu Phe Val Asn Gly Gly Asp Leu Met Phe His Ile Gln Lys Ser
           435                 440                 445
   Arg Arg Phe Asp Glu Ala Arg Ala Arg Phe Tyr Ala Ala Glu Ile Ile
       450                 455                 460
   Ser Ala Leu Met Phe Leu His Asp Lys Gly Ile Ile Tyr Arg Asp Leu
   465                 470                 475                 480
   Lys Leu Asp Asn Val Leu Leu Asp His Glu Gly His Cys Lys Leu Ala
                   485                 490                 495
   Asp Phe Gly Met Cys Lys Glu Gly Ile Cys Asn Gly Val Thr Thr Ala
               500                 505                 510
   Thr Phe Cys Gly Thr Pro Asp Tyr Ile Ala Pro Glu Ile Leu Gln Glu
           515                 520                 525
   Met Leu Tyr Gly Pro Ala Val Asp Trp Trp Ala Met Gly Val Leu Leu
       530                 535                 540
   Tyr Glu Met Leu Cys Gly His Ala Pro Phe Glu Ala Glu Asn Glu Asp
   545                 550                 555                 560
   Asp Leu Phe Glu Ala Ile Leu Asn Asp Glu Val Val Tyr Pro Thr Trp
                   565                 570                 575
   Leu His Glu Asp Ala Thr Gly Ile Leu Lys Ser Phe Met Thr Lys Asn
               580                 585                 590
   Pro Thr Met Arg Leu Gly Ser Leu Thr Gln Gly Gly Glu His Ala Ile
           595                 600                 605
   Leu Arg His Pro Phe Phe Lys Glu Ile Asp Trp Ala Gln Leu Asn His
       610                 615                 620
   Arg Gln Ile Glu Pro Pro Phe Arg Pro Arg Ile Lys Ser Arg Glu Asp
   625                 630                 635                 640
   Val Ser Asn Phe Asp Pro Asp Phe Ile Lys Glu Glu Pro Val Leu Thr
                   645                 650                 655
   Pro Ile Asp Glu Gly His Leu Pro Met Ile Asn Gln Asp Glu Phe Arg
               660                 665                 670
   Asn Phe Ser Tyr Val Ser Pro Glu Leu Gln Pro
               675                 680
<210> 2
   <211> 223
   <212> PRT
   <213> Homo sapiens
<400> 2
<210> 3
   <211> 17
   <212> PRT
   <213> Artificial Sequence
<220>
   <223> Pseudosubstrate Peptide
<400> 3
   Thr Arg Lys Arg Gln Arg Ala Met Arg Arg Arg Val His Gln Ile Asn
   1 5 10 15
   Gly
<210> 4
   <211> 16
   <212> PRT
   <213> Drosophila pseudoobscura
<400> 4
   Arg Gln Ile Lys Ile Trp Phe Gln Asn Arg Arg Met Lys Trp Lys Lys
   1 5 10 15
<210> 5
   <211> 9
   <212> PRT
   <213> Artificial Sequence
<220>
   <223> Cell Penetrating Peptide
<400> 5
   Arg Arg Arg Arg Arg Arg Arg Arg Arg
   1 5
<210> 6
   <211> 9
   <212> PRT
   <213> Artificial Sequence
<220>
   <223> Cell Penetrating Peptide
<400> 6
   Lys Lys Lys Lys Lys Lys Lys Lys Lys
   1 5
<210> 7
   <211> 12
   <212> PRT
   <213> Artificial Sequence
<220>
   <223> Cell Penetrating Peptide
<400> 7
   Arg Arg Gln Arg Arg Thr Ser Lys Leu Met Lys Arg
   1               5                   10
<210> 8
   <211> 27
   <212> PRT
   <213> Artificial Sequence
<220>
   <223> Cell Penetrating Peptide
<400> 8
   Gly Trp Thr Leu Asn Ser Ala Gly Tyr Leu Leu Gly Lys Ile Asn Leu
   1               5                   10                  15
   Lys Ala Leu Ala Ala Leu Ala Lys Lys Ile Leu
               20                  25
<210> 9
   <211> 30
   <212> PRT
   <213> Artificial Sequence
<220>
   <223> Cell Penetrating Peptide
<400> 9
   Trp Glu Ala Lys Leu Ala Lys Ala Leu Ala Lys Ala Leu Ala Lys His
   1               5                   10                  15
   Leu Ala Lys Ala Leu Ala Lys Ala Leu Lys Ala Cys Glu Ala
               20                  25                  30
<210> 10
   <211> 21
   <212> DNA
   <213> Mus musculus
<400> 10
   actcatgtac ccaccgccat a 21

## Description

The discovery and recognition of CD4+Foxp3+ Tregs as a distinct subset of T cells with immunoregulatory function represents a major advance in understanding of the immune system. Tregs actively maintain immune homeostasis and self-tolerance, and one prominent Treg-mediated suppressive mechanism is dependent upon its contact with antigen presenting cells (APCs). This physical contact promotes the formation of a specialized signaling platform, known as the immunological synapse (IS), at the Treg-APC interface. However, the Treg IS is distinguishable from the "conventional" IS formed between naïve/effector T cells and APCs in several respects. First, although the TCR is present in the central supramolecular activation cluster (cSMAC) in both types of IS, the costimulatory CD28 receptor is recruited to the Teff IS, whereas CTLA-4 is present at the Treg IS. Second, PKCθ is absent from the Treg IS and, moreover, in contrast to Teff cells, it negatively regulates the function of Tregs. Physical association of PKCθ, mediated by its V3 domain, with the costimulatory CD28 receptor underlies its cSMAC recruitment and essential functions in driving the activation, proliferation and differentiation of Teff cells. Hence, the absence of PKCθ in the Treg IS suggests that TCR signaling events in these cells are significantly different from those of Teff cells.

WO2011/083481 describes a method, composition and kit for treatment of inflammatory disease and disorder using PKC isoform modulators.

Fischer et al., Journal of Clinical Oncology, 2010, vol. 28, pages 4339-4345 describes a Phase IIB trial of oral Midostaurin (PKC412), the FMS-Like Tyrosine Kinase 3 Receptor (FLT3) and Multi-Targeted Kinase Inhibitor, in patients with acute myeloid leukemia and high-risk myelodysplastic syndrome with either wild-type or mutated FLT3.

Curran et al., PNAS, 2010, vol. 107, pages 4275-4280 describes the use of a PD-1 and CTLA-4 combination blockade to expand infiltrating T cells and reduce regulatory T and myeloid cells within B16 melanoma tumors.

WO2005/014612 describes methods and compositions related to a naturally-occurring alternative splice variant of CTLA-4, termed ligand-independent CTLA-4 (liCTLA-4).

Kong et al., Nat Immunol., 2014, vol. 15, pages 465-472 describes how Protein Kinase C-η may control CTLA-4-Mediated Regulatory T Cell function.

WO2010/026565 describes a method and kit for the assessment of prognosis of a subject having or suspected of having cancer, by determination of the cellular localization of PKCη.

WO2010/117055 describes an inhibitor capable of inhibiting protein kinase Cα or η selectively.

Fu et al., Sci. Signal., 2011, vol. 4, pages ra84 describes how Protein Kinase Cη is required for T cell activation and homeostatic proliferation.

WO2012/127240 describes a method of identifying a modulator of the immune response system, comprising assaying for the effect of a compound on the intemalisation and recycling of CTLA-4 in a cell, wherein an increase in cycling of CTLA-4 compared to a control without the compound indicates that the compound suppresses the immune response, and a decrease in the cycling of CTLA-4 compared to a control without the compound indicates that the compound increases the immune response.

### SUMMARY

This disclosure is based in part on the surprising discovery that PKCη is an important modulator in the immune response. Accordingly, there are disclosed, among other things, compounds, uses and methods for modulating an immune response by modulating the interaction of PKCη with CTLA-4. In various embodiments and examples, compounds, methods and uses can be used to modulate the function or activity of T cells, such as T regs.

The invention is defined in the claims. Thus, the invention provides an agent for use in a method of treating cancer in a subject wherein the method comprises decreasing, reducing, inhibiting, suppressing or limiting the direct physical interaction between Protein kinase C eta type (PKCη) and Cytotoxic T-lymphocyte protein 4 (CTLA-4) to decrease, reduce, inhibit, suppress or limit regulatory T cell function; wherein the agent inhibits the direct physical interaction of PKCη with CTLA-4, further wherein the agent is a protein or peptide comprising or consisting of the PKCη amino acid sequence:
MSSGTMKFNGYLRVRIGEAVGLQPTRWSLRHSLFKKGHQLLDPYLTVSVDQVRVGQTSTKQKTNKPTYNEEFCANVTDGGHLELAVFHETPLGYDHFVANCTLQFQELLRTTGASDTFEGWVDLEPEGKVFVVITLTGSFTEATLQRDRIFKHFTRKRQRAMRRRVHQINGHKFMATYLRQPTYCSHCREFIWGVFGKQGYQCQVCTCVVHKRCHHLIVTACTCQNNINKVDSKIAEQRFGINIPHKFSIHNYKVPTFCDHCGSLLWGIMRQGLQCKICKMNVHIRCQANVAPNCGVNAVELAKTLAGMGLQPGNISPTSKLVSRSTLRRQGKESSKEGNGIGVNSSNRLGIDNFEFIRVLGKGSFGKVMLARVKETGDLYAVKVLKKDVILQDDDVECTMTEKRILSLARNHPFLTQLFCCFQTPDRLFFVMEFVNGGDLMFHIQKSRRFDEARARFYAAEIISALMFLHDKGIIYRDLKLDNVLLDHEGHCKLADFGMCKEGICNGVTTATFCGTPDYIAPEILQEMLYGPAVDWWAMGVLLYEMLCGHAPFEAENEDDLFEAILNDEVVYPTWLHEDATGILKSFMTKNPTMRLGSLTQGGEHAILRHPFFKEIDWAQLNHRQIEPPFRPRIKSREDVSNFDPDFIKEEPVLTPIDEGHLPMINQDEFRNFSYVSPELQP (SEQ ID NO: 1), or subsequence thereof, that binds directly to CTLA-4, and the method comprises contacting CTLA-4 with the agent.

Also as defined in the claims, the invention further provides an *in vitro* method of modulating regulatory T cell function comprising modulating interaction of PKCη with CTLA-4, the method comprising contacting CTLA-4 with an agent that inhibits the direct physical interaction of CTLA-4 with PKCη wherein the agent is a protein or peptide comprising or consisting of the PKCη amino acid sequence:
MSSGTMKFNGYLRVRIGEAVGLQPTRWSLRHSLFKKGHQLLDPYLTVSVDQVRVGQTSTKQKTNKPTYNEEFCANVTDGGHLELAVFHETPLGYDHFVANCTLQFQELLRTTGASDTFEGWVDLEPEGKVFVVITLTGSFTEATLQRDRIFKHFTRKRQRAMRRRVHQINGHKFMATYLRQPTYCSHCREFIWGVFGKQGYQCQVCTCVVHKRCHHLIVTACTCQNNINKVDSKIAEQRFGINIPHKFSIHNYKVPTFCDHCGSLLWGIMRQGLQCKICKMNVHIRCQANVAPNCGVNAVELAKTLAGMGLQPGNISPTSKLVSRSTLRRQGKESSKEGNGIGVNSSNRLGIDNFEFIRVLGKGSFGKVMLARVKETGDLYAVKVLKKDVILQDDDVECTMTEKRILSLARNHPFLTQLFCCFQTPDRLFFVMEFVNGGDLMFHIQKSRRFDEARARFYAAEIISALMFLHDKGIIYRDLKLDNVLLDHEGHCKLADFGMCKEGICNGVTTATFCGTPDYIAPEILQEMLYGPAVDWWAMGVLLYEMLCGHAPFEAENEDDLFEAILNDEVVYPTWLHEDATGILKSFMTKNPTMRLGSLTQGGEHAILRHPFFKEIDWAQLNHRQIEPPFRPRIKSREDVSNFDPDFIKEEPVLTPIDEGHLPMINQDEFRNFSYVSPELQP (SEQ ID NO: 1), or subsequence thereof, that binds directly to CTLA-4; thereby decreasing, reducing, inhibiting, suppressing, limiting or controlling interaction between PKCη and CTLA-4 to decrease, reduce, inhibit, suppress, limit or control regulatory T cell function.

The disclosure also describes a method of modulating an immune response comprising modulating activity or expression of PKCη. Examples described herein also provide methods of modulating an immune response comprising modulating interaction of PKCη with CTLA-4. In particular examples, the method comprises decreasing, reducing, inhibiting, suppressing, limiting or controlling interaction between PKCη and CTLA-4 to increase, stimulate, enhance, promote, induce or activate the immune response. In additional examples, the method comprises increasing, stimulating, enhancing, promoting, inducing or activating the immune response to a hyperproliferative cell, tumor cell, cancer cell or metastatic cell or pathogen. In further examples, the method comprises increasing, stimulating, enhancing, promoting, inducing or activating interaction between PKCη and CTLA-4 to decrease, reduce, inhibit, suppress, limit or control the immune response. In particular examples, PKCη is phosphorylated at S28, S32 and S317. In further examples, the method comprises modulating effector cell cytokine secretion. In particular examples, the effector cell cytokines comprise IL-2, IFNg, IL-4 and IL-17A.

In another example, there is provided a method of modulating regulatory T cell function comprising modulating activity or expression of PKCη. As defined in the claims, embodiments disclosed herein provide in vitro methods of modulating a regulatory T cell function comprising modulating interaction of PKCη with CTLA-4. In particular examples, the method comprises increasing, stimulating, enhancing, promoting, inducing or activating interaction between PKCη and CTLA-4 to increase, stimulate, enhance, promote, induce or activate the regulatory T cell function. In additional aspects, the method comprises decreasing, reducing, inhibiting, suppressing, limiting or controlling interaction between PKCη and CTLA-4 to decrease, reduce, inhibit, suppress, limit or control the regulatory T cell function. In further examples, the method comprises decreasing, reducing, inhibiting, suppressing, limiting or controlling regulatory T cell activity in order to increase, stimulate, enhance, promote, induce or activate an immune response to a hyperproliferative cell, tumor cell, cancer cell or metastatic cell or pathogen. In additional examples, the method comprises modulating effector cell cytokine secretion. In particular examples, the effector cell cytokines comprise IL-2, IFNg, IL-4 and IL-17A.

The disclosure also provides a method comprising contacting PKCη with an agent that modulates binding of PKCη to CTLA-4. Examples disclosed herein also provide a method comprising contacting CTLA-4 with an agent that modulates binding of CTLA-4 to PKCη. In particular aspects, the agent decreases, reduces, inhibits, suppresses or disrupts binding of PKCη to CTLA-4. In additional examples, the agent increases, enhances, stimulates or promotes binding of PKCη to CTLA-4. In further examples, the agent binds to one or both of PKCη and CTLA-4. In additional examples, the agent binds to a PKCη amino acid sequence that comprises, consists or consists essentially of from about residue 28 to residue 317 of PKCη or a subsequence, portion, homologue, variant or derivative thereof. In further aspects, the agent binds to a CTLA-4 amino acid sequence that comprises, consists or consists essentially of from about residue 182 to residue 223 of CTLA-4 or a subsequence, portion, homologue, variant or derivative thereof. In additional aspects, the agent binds to a CTLA-4 amino acid sequence that comprises K188, K191, K192 or R193 of CTLA-4. In further examples, the agent comprises a protein or peptide comprising, consisting of or consisting essentially of a PKCη amino acid sequence, or subsequence, portion, homologue, variant or derivative thereof, that binds to CTLA-4. In particular aspects, the peptide comprises, consists or consists essentially of an amino acid sequence of PKCη set forth as:
MSSGTMKFNGYLRVRIGEAVGLQPTRWSLRHSLFKKGHQLLDPYLTVSVDQVRVGQTSTKQKTNKPTYNEEFCANVTDGGHLELAVFHETPLGYDHFVANCTLQFQELLRTTGASDTFEGWVDLEPEGKVFVVITLTGSFTEATLQRDRIFKHFTRKRQRAMRRRVHQINGHKFMATYLRQPTYCSHCREFIWGVFGKQGYQCQVCTCVVHKRCHHLIVTACTCQNNINKVDSKIAEQRFGINIPHKFSIHNYKVPTFCDHCGSLLWGIMRQGLQCKICKMNVHIRCQANVAPNCGVNAVELAKTLAGMGLQPGNISPTSKLVSRSTLRRQGKESSKEGNGIGVNSSNRLGIDNFEFIRVLGKGSFGKVMLARVKETGDLYAVKVLKKDVILQDDDVECTMTEKRILSLARNHPFLTQLFCCFQTPDRLFFVMEFVNGGDLMFHIQKSRRFDEARARFYAAEIISALMFLHDKGIIYRDLKLDNVLLDHEGHCKLADFGMCKEGICNGVTTATFCGTPDYIAPEILQEMLYGPAVDWWAMGVLLYEMLCGHAPFEAENEDDLFEAILNDEVVYPTWLHEDATGILKSFMTKNPTMRLGSLTQGGEHAILRHPFFKEIDWAQLNHRQIEPPFRPRIKSREDVSNFDPDFIKEEPVLTPIDEGHLPMINQDEFRNFSYVSPELQP (SEQ ID NO: 1), or a subsequence, portion, homologue, variant or derivative thereof. In further aspects, the peptide comprises, consists or consists essentially of from about residue 28 to residue 317 of PKCη or a subsequence, portion, homologue, variant or derivative thereof. In additional aspects, the PKCη amino acid sequence, or subsequence, portion, homologue, variant or derivative thereof, is phosphorylated at S28, S32 and S317 of PKCη. In additional examples, the agent comprises a protein or peptide comprising, consisting of or consisting essentially of a CTLA-4 amino acid sequence, or subsequence, portion, homologue, variant or derivative thereof, that binds to PKCη. In particular examples, the peptide comprises, consists or consists essentially of an amino acid sequence of CTLA-4 set forth as:
MACLGFQRHKAQLNLATRTWPCTLLFFLLFIPVFCKAMHVAQPAVVLASSRGIASFVCEYASPGKATEVRVTVLRQADSQVTEVCAATYMMGNELTFLDDSICTGTSSGNQVNLTIQGLRAMDTGLYICKVELMYPPPYYLGIGNGTQIYVIDPEPCPDSDFLLWILAAVSSGLFFYSFLLTAVSLSKMLKKRSPLTTGVYVKMPPTEPECEKQFQPYFIPIN (SEQ ID NO: 2), or a subsequence, portion, homologue, variant or derivative thereof. In additional examples, the peptide comprises, consists or consists essentially of from about residue 182 to residue223 of CTLA-4 or a subsequence, portion, homologue, variant or derivative thereof. In further examples, the CTLA-4 amino acid sequence, or subsequence, portion, homologue, variant or derivative thereof comprises K188, K191, K192 or R193 of CTLA-4. In particular examples, the agent comprises a fusion polypeptide or chimeric polypeptide. In additional examples, the agent comprises a small molecule.

In further examples, the agent comprises an antibody or an antibody fragment thereof that binds to PKCη or CTLA-4. In particular examples, an agent comprises a bi-specific antibody or fragment of a bi-specific antibody that binds to PKCη and CTLA-4.

In various examples, an agent comprises a contiguous amino acid sequence having a length of about 5-500 amino acids. In particular examples, an agent comprises a contiguous amino acid sequence having a length of about 10-20, 20-30, 30-40, 40-50, 50-60, 60-70, 70-80, 80-90, 90-100, 100-110, 110-120, 120-130, 130-140, 140-150, 150-175 or 175-200 amino acid residues.

In more particular examples, an agent that binds to PKCη comprises or consists of: Rottlerin ((E)-1-[6-[(3-acetyl-2,4,6-trihydroxy-5-methylphenyl)methyl]-5,7-dihydroxy-2,2-dimethylchromen-8-yl]-3-phenylprop-2-en-1-one); Midostaurin ((9S,10R,11R,13R)-2,3,10,11,12,13-Hexahydro-10-methoxy-9-methyl-11-(methylamino)-9,13-epoxy-1H,9H-diindolo[1,2,3-gh:3',2',1r-lm]pyrrolo[3,4-j][1,7]benzodiamzonine-1-one), or a peptide pseudosubstrate sequence, for example, as set forth as: Thr-Arg-Lys-Arg-Gln-Arg-Ala-Met-Arg-Arg-Arg-Val-His-Gln-Ile-Asn-Gly (SEQ ID NO: 3).

In another example, there is provided a method of modulating an immune response in a subject, comprising administering an agent that modulates activity or expression of PKCη. Examples disclosed herein also provide a method of modulating an immune response in a subject, comprising administering an agent that modulates binding of PKCη to CTLA-4 in the subject, thereby modulating the immune response in the subject. In particular examples, the method comprises decreasing, reducing, inhibiting, suppressing, limiting or controlling in the subject an undesirable or aberrant immune response, disorder or disease, an inflammatory response, disorder or disease, inflammation, or an autoimmune response, disorder or disease, or an adverse symptom of an undesirable or aberrant immune response, disorder or disease, an inflammatory response, disorder or disease, inflammation or an autoimmune response, disorder or disease. In additional examples, the method comprises increasing, stimulating, enhancing, promoting, inducing or activating in a subject an immune response, inflammatory response or inflammation. In further examples, the subject has or has had an undesirable or aberrant immune response, disorder or disease, an inflammatory response, disorder or disease, inflammation, or an autoimmune response, disorder or disease or an adverse symptom of an undesirable or aberrant immune response, disorder or disease, an inflammatory response, disorder or disease, inflammation, or an autoimmune response, disorder or disease. In additional examples, the subject is in need of treatment for an undesirable or aberrant immune response, disorder or disease, an inflammatory response, disorder or disease, inflammation, or an autoimmune response, disorder or disease or an adverse symptom of an undesirable or aberrant immune response, disorder or disease, an inflammatory response, disorder or disease, inflammation, or an autoimmune response, disorder or disease. In further examples, the subject is at risk of an undesirable or aberrant immune response, disorder or disease, an inflammatory response, disorder or disease, inflammation, or an autoimmune response, disorder or disease or an adverse symptom of an undesirable or aberrant immune response, disorder or disease, an inflammatory response, disorder or disease, inflammation, or an autoimmune response, disorder or disease. In particular examples, the immune response or inflammatory response is an anti-cancer or anti-pathogen immune response or inflammatory response. In additional aspects, the subject has or has had cancer. In further aspects, the subject is in need of treatment for cancer. In additional aspects, the subject is at risk of developing cancer.

In particular aspects, the cancer comprises Acute lymphoblastic leukemia (ALL); Acute myeloid leukemia; Adrenocortical carcinoma; AIDS-related cancers; AIDS-related lymphoma; Anal cancer; Appendix cancer; Astrocytoma; childhood cerebellar or cerebral; Basal-cell carcinoma; Bile duct cancer; extrahepatic (see Cholangiocarcinoma); Bladder cancer; Bone tumor; Osteosarcoma/Malignant fibrous histiocytoma; Brainstem glioma; Brain cancer; Brain tumor; cerebellar astrocytoma; Brain tumor; cerebral astrocytoma/malignant glioma; Brain tumor; ependymoma; Brain tumor; medulloblastoma; Brain tumor; supratentorial primitive neuroectodermal tumors; Brain tumor; visual pathway and hypothalamic glioma; Breast cancer; Bronchial adenomas/carcinoids; Burkitt's lymphoma; Carcinoid tumor, childhood; Carcinoid tumor, gastrointestinal; Carcinoma of unknown primary; Central nervous system lymphoma, primary; Cerebellar astrocytoma, childhood; Cerebral astrocytoma/Malignant glioma, childhood; Cervical cancer; Childhood cancers; Chronic lymphocytic leukemia; Chronic myelogenous leukemia; Chronic myeloproliferative disorders; Colon Cancer; Cutaneous T-cell lymphoma; Desmoplastic small round cell tumor; Endometrial cancer; Ependymoma; Esophageal cancer; Ewing's sarcoma in the Ewing family of tumors; Extracranial germ cell tumor, Childhood; Extragonadal Germ cell tumor; Extrahepatic bile duct cancer; Eye Cancer; Intraocular melanoma; Eye Cancer, Retinoblastoma; Gallbladder cancer; Gastric (Stomach) cancer; Gastrointestinal Carcinoid Tumor; Gastrointestinal stromal tumor (GIST); Germ cell tumor: extracranial, extragonadal, or ovarian; Gestational trophoblastic tumor; Glioma of the brain stem; Glioma, Childhood Cerebral Astrocytoma; Glioma, Childhood Visual Pathway and Hypothalamic; Gastric carcinoid; Hairy cell leukemia; Head and neck cancer; Heart cancer; Hepatocellular (liver) cancer; Hodgkin lymphoma; Hypopharyngeal cancer; Hypothalamic and visual pathway glioma, childhood; Intraocular Melanoma; Islet Cell Carcinoma (Endocrine Pancreas); Kaposi sarcoma; Kidney cancer (renal cell cancer); Laryngeal Cancer; Leukemias; Leukemia, acute lymphoblastic (also called acute lymphocytic leukemia); Leukemia, acute myeloid (also called acute myelogenous leukemia); Leukemia, chronic lymphocytic (also called chronic lymphocytic leukemia); Leukemia, chronic myelogenous (also called chronic myeloid leukemia); Leukemia, hairy cell; Lip and Oral Cavity Cancer; Liposarcoma; Liver Cancer (Primary); Lung Cancer, Non-Small Cell; Lung Cancer, Small Cell; Lymphomas; Lymphoma, AIDS-related; Lymphoma, Burkitt; Lymphoma, cutaneous T-Cell; Lymphoma, Hodgkin; Lymphomas, Non-Hodgkin (an old classification of all lymphomas except Hodgkin's); Lymphoma, Primary Central Nervous System; Macroglobulinemia, Waldenström; Malignant Fibrous Histiocytoma of Bone/Osteosarcoma; Medulloblastoma, Childhood; Melanoma; Melanoma, Intraocular (Eye); Merkel Cell Carcinoma; Mesothelioma, Adult Malignant; Mesothelioma, Childhood; Metastatic Squamous Neck Cancer with Occult Primary; Mouth Cancer; Multiple Endocrine Neoplasia Syndrome, Childhood; Multiple Myeloma/Plasma Cell Neoplasm; Mycosis Fungoides; Myelodysplastic Syndromes; Myelodysplastic/Myeloproliferative Diseases; Myelogenous Leukemia, Chronic; Myeloid Leukemia, Adult Acute; Myeloid Leukemia, Childhood Acute; Myeloma, Multiple (Cancer of the Bone-Marrow); Myeloproliferative Disorders, Chronic; Nasal cavity and paranasal sinus cancer; Nasopharyngeal carcinoma; Neuroblastoma; Non-Hodgkin lymphoma; Non-small cell lung cancer; Oral Cancer; Oropharyngeal cancer; Osteosarcoma/malignant fibrous histiocytoma of bone; Ovarian cancer; Ovarian epithelial cancer (Surface epithelial-stromal tumor); Ovarian germ cell tumor; Ovarian low malignant potential tumor; Pancreatic cancer; Pancreatic cancer, islet cell; Paranasal sinus and nasal cavity cancer; Parathyroid cancer; Penile cancer; Pharyngeal cancer; Pheochromocytoma; Pineal astrocytoma; Pineal germinoma; Pineoblastoma and supratentorial primitive neuroectodermal tumors, childhood; Pituitary adenoma; Plasma cell neoplasia/Multiple myeloma; Pleuropulmonary blastoma; Primary central nervous system lymphoma; Prostate cancer; Rectal cancer; Renal cell carcinoma (kidney cancer); Renal pelvis and ureter, transitional cell cancer; Retinoblastoma; Rhabdomyosarcoma, childhood; Salivary gland cancer; Sarcoma, Ewing family of tumors; Sarcoma, Kaposi; Sarcoma, soft tissue; Sarcoma, uterine; Sézary syndrome; Skin cancer (nonmelanoma); Skin cancer (melanoma); Skin carcinoma, Merkel cell; Small cell lung cancer; Small intestine cancer; Soft tissue sarcoma; Squamous cell carcinoma; Squamous neck cancer with occult primary, metastatic; Stomach cancer; Supratentorial primitive neuroectodermal tumor, childhood; T-Cell lymphoma, cutaneous; Testicular cancer; Throat cancer; Thymoma, childhood; Thymoma and Thymic carcinoma; Thyroid cancer; Thyroid cancer, childhood; Transitional cell cancer of the renal pelvis and ureter; Trophoblastic tumor, gestational; Unknown primary site, carcinoma of, adult; Unknown primary site, cancer of, childhood; Ureter and renal pelvis, transitional cell cancer; Urethral cancer; Uterine cancer, endometrial; Uterine sarcoma; Vaginal cancer; Visual pathway and hypothalamic glioma, childhood; Vulvar cancer; Waldenström macroglobulinemia or Wilms tumor (kidney cancer), childhood.

In additional examples, non-limiting aberrant or undesirable immune responses, disorders and diseases, inflammatory responses, disorders and diseases, inflammation, autoimmune responses, disorders and diseases, treatable in accordance with examples herein include: rheumatoid arthritis, juvenile rheumatoid arthritis, osteoarthritis, psoriatic arthritis, multiple sclerosis (MS), encephalomyelitis, myasthenia gravis, systemic lupus erythematosus (SLE), asthma, allergic asthma, autoimmune thyroiditis, atopic dermatitis, eczematous dermatitis, psoriasis, Sjögren's Syndrome, Crohn's disease, aphthous ulcer, iritis, conjunctivitis, keratoconjunctivitis, ulcerative colitis (UC), inflammatory bowel disease (IBD), cutaneous lupus erythematosus, scleroderma, vaginitis, proctitis, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, interstitial lung fibrosis, Hashimoto's thyroiditis, autoimmune polyglandular syndrome, insulin-dependent diabetes mellitus (1DDM, type I diabetes), insulin-resistant diabetes mellitus (type 11 diabetes), immune-mediated infertility, autoimmune Addison's disease, pemphigus vulgaris, pemphigus foliaceus, dermatitis herpetiformis, autoimmune alopecia, vitiligo, autoimmune hemolytic anemia, autoimmune thrombocytopenic purpura, pernicious anemia, Guillain-Barre syndrome, stiff-man syndrome, acute rheumatic fever, sympathetic ophthalmia, Goodpasture's syndrome, systemic necrotizing vasculitis, antiphospholipid syndrome or an allergy, Behcet's disease, severe combined immunodeficiency (SCID), recombinase activating gene (RAG 1/2) deficiency, adenosine deaminase (ADA) deficiency, interleukin receptor common γ chain (γc) deficiency, Janus-associated kinase 3 (JAK3) deficiency and reticular dysgenesis; primary T cell immunodeficiency such as DiGcorge syndrome, Nude syndrome, T cell receptor deficiency, MHC class II deficiency, T AP-2 deficiency (MHC class I deficiency), ZAP70 tyrosine kinase deficiency and purine nucleotide phosphorylase (PNP) deficiency, antibody deficiencies, X-linked agammaglobulinemia (Bruton's tyrosine kinase deficiency), autosomal recessive agammaglobulinemia, Mu heavy chain deficiency, surrogate light chain (γ5/14 .1) deficiency, Hyper-lgM syndrome: X-linked (CD40 ligand deficiency) or non-X-Iinked, Ig heavy chain gene deletion, IgA deficiency, deficiency of IgG subclasses (with or without IgA deficiency), common variable immunodeficiency (CVID), antibody deficiency with normal immunoglobulins; transient hypogammaglobulinemia of infancy, interferon γ receptor (IFNGR1, IFNGR2) deficiency, interleukin 12 or interleukin 12 receptor deficiency, immunodeficiency with thymoma, Wiskott-Aldrich syndrome (WAS protein deficiency), ataxia telangiectasia (ATM deficiency), X-linked lymphoproliferative syndrome (SH2D1A/SAP deficiency), hyper IgE syndrome or Graft vs. Host Disease (GVHD).

The disclosure also describes a peptide, comprising, consisting or consisting essentially of a subsequence of PKCη or a portion, homologue, variant or derivative thereof that modulates PKCη expression, activity or signaling. Examples disclosed herein also provide a peptide, comprising, consisting or consisting essentially of a subsequence of PKCη or a portion, homologue, variant or derivative thereof that modulates binding of PKCη to CTLA-4. In particular examples, the sequence of PKCη comprises, consists or consists essentially of the amino acid sequence: MSSGTMKFNGYLRVRIGEAVGLQPTRWSLRHSLFKKGHQLLDPYLTVSVDQVRVGQTS TKQKTNKPTYNEEFCANVTDGGHLELAVFHETPLGYDHFVANCTLQFQELLRTTGASDT FEGWVDLEPEGKVFVVITLTGSFTEATLQRDRIFKHFTRKRQRAMRRRVHQINGHKFMA TYLRQPTYCSHCREFIWGVFGKQGYQCQVCTCVVHKRCHHLIVTACTCQNNINKVDSKI AEQRFGINIPHKFSIHNYKVPTFCDHCGSLLWGIMRQGLQCKICKMNVHIRCQANVAPNC GVNAVELAKTLAGMGLQPGNISPTSKLVSRSTLRRQGKESSKEGNGIGVNSSNRLGIDNF EFIRVLGKGSFGKVMLARVKETGDLYAVKVLKKDVILQDDDVECTMTEKRILSLARNHP FLTQLFCCFQTPDRLFFVMEFVNGGDLMFHIQKSRRFDEARARFYAAEIISALMFLHDKGI IYRDLKLDNVLLDHEGHCKLADFGMCKEGICNGVTTATFCGTPDYIAPEILQEMLYGPA VDWWAMGVLLYEMLCGHAPFEAENEDDLFEAILNDEVVYPTWLHEDATGILKSFMTK NPTMRLGSLTQGGEHAILRHPFFKEIDWAQLNHRQIEPPFRPRIKSREDVSNFDPDFIKEEP VLTPIDEGHLPMINQDEFRNFSYVSPELQP (SEQ ID NO: 1), or a subsequence, portion, homologue, variant or derivative thereof. In additional examples, the peptide comprises, consists or consists essentially of residues 28-317 of PKCη or a subsequence, portion, homologue, variant or derivative thereof. In further examples, the subsequence of PKCη or a portion, homologue, variant or derivative thereof is phosphorylated at S28, S32 and S317 of PKCη. In additional examples, the peptide is not phosphorylated.

In another example, there is provided a peptide, comprising, consisting or consisting essentially of a subsequence of CTLA-4 or a portion, homologue, variant or derivative thereof that modulates binding of CTLA-4 to PKCη. In particular examples, the sequence of CTLA-4 comprises, consists or consists essentially of the amino acid sequence: MACLGFQRHKAQLNLATRTWPCTLLFFLLFIPVFCKAMHVAQPAVVLASSRGIASFVCE YASPGKATEVRVTVLRQADSQVTEVCAATYMMGNELTFLDDSICTGTSSGNQVNLTIQG LRAMDTGLYICKVELMYPPPYYLGIGNGTQIYVIDPEPCPDSDFLLWILAAVSSGLFFYSF LLTAVSLSKMLKKRSPLTTGVYVKMPPTEPECEKQFQPYFIPIN (SEQ ID NO: 2), or a subsequence, portion, homologue, variant or derivative thereof. In further examples, the peptide comprises, consists or consists essentially of residues 182-223 of CTLA-4 or a subsequence, portion, homologue, variant or derivative thereof. In additional examples, the peptide comprises, consists or consists essentially of residues K188, K191, K192 or R193 of CTLA-4.

### BRIEF DESCRIPTION OF THE DRAWINGS

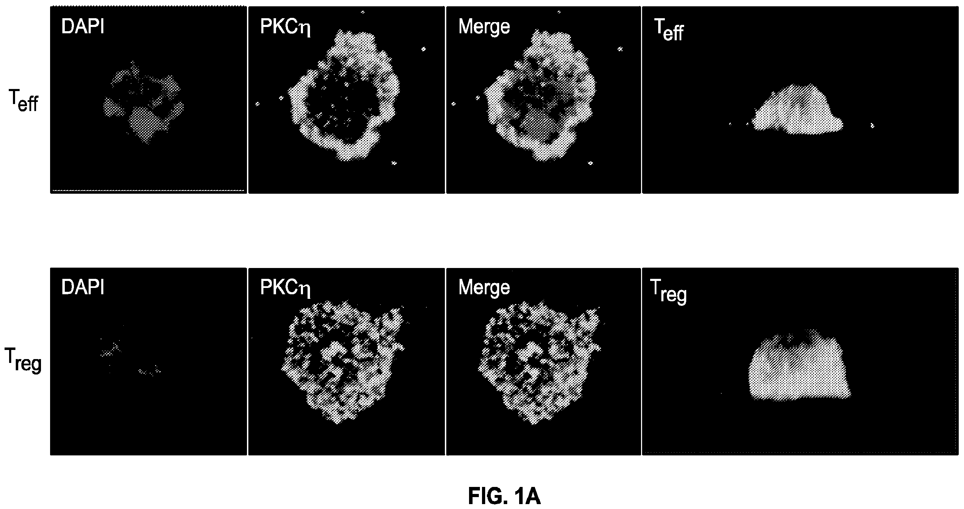

**Figures 1A-1D** **show that PKOη, but not PKCθ, is recruited to the immunological synapse of Tregs.** CD4+CD25- and CD4+CD25+ Treg cells were FACS-sorted from WT (A, B), Prkch-/- (C) or Prkcq-/- (D) mice. Cells were incubated with lipid bilayers containing anti-CD3 plus -CD28 mAbs for 6 min, fixed, and stained with rabbit antibodies against PKCη (A) or PKCθ (B), followed by AlexaFluor 488-labeled anti-rabbit Ig antibody, and counterstained with DAPI. In the merged image panel, PKC staining is shown in green, and the contact site is shown in red. In (A) and (B), the right most panels show side views, whereas the other panels show top views, of the cells. Images are representative of at least 50 cells collected from three independent studies.

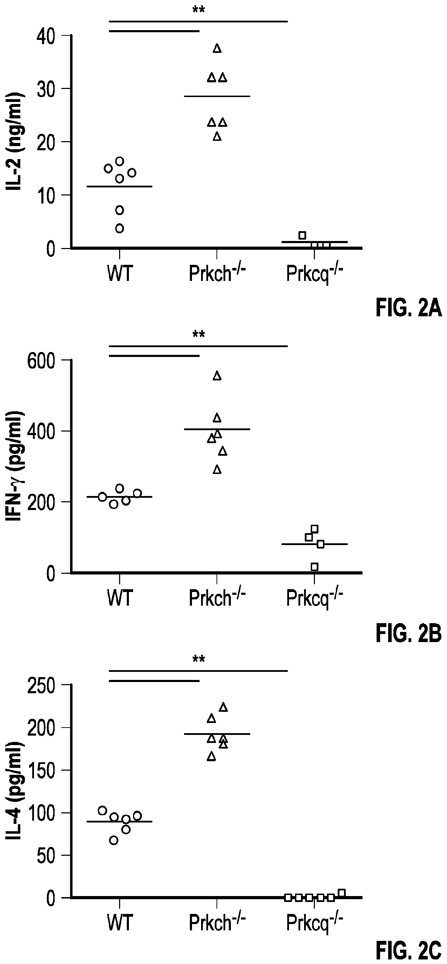

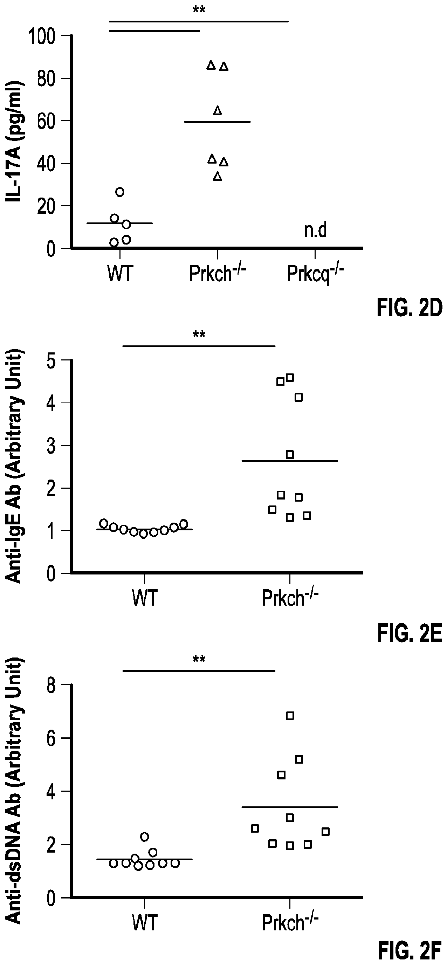

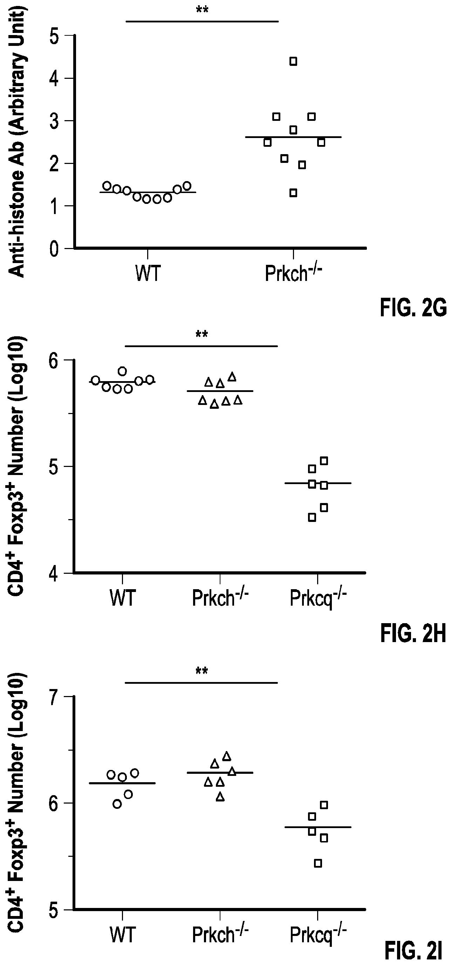

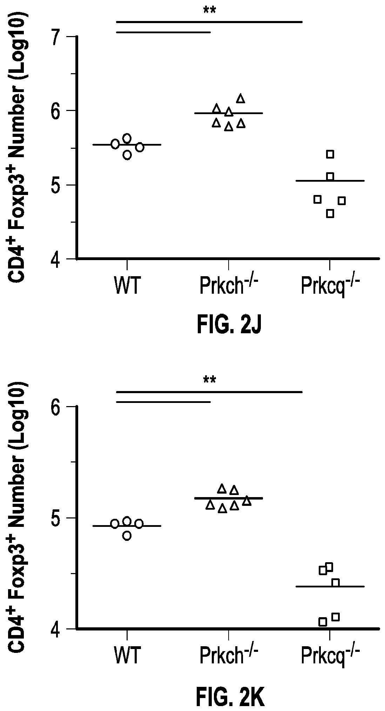

**Figures 2A-2M** **show phenotypic and functional characterization of Prkch-/- and Prkcq-/- mice.** Equal numbers of FACS-sorted CD44hi cells were stimulated with anti-CD3 plus -CD28 mAbs for 24 hours and the culture supernatant levels of IL-2 (A), IFNγ (B), IL-4 (C) and IL-17A (D) were determined by ELISA. (E to G) Serum levels of IgE (E), anti-double stranded DNA (F) or anti-histone (G) of 8-12 week-old WT and Prkch-/- mice were determined using ELISA. (H to K) Numbers of CD4+Foxp3+ cells from thymi (H), spleens (I), peripheral lymph nodes (J) and mesenteric lymph nodes (K) of 8-12 week-old WT, Prkcq-/- and Prkch-/- mice were determined by intracellular Foxp3 staining. (L) Expression of Foxp3, TCRβ, CTLA-4, CD25, CD44 and GITR were determined on CD4+Foxp3+-gated cells. (M) Naive CD4+CD62L+ cells from 8-12 week-old WT, Prkcq-/- and Prkch-/- mice were stimulated with anti-CD3 plus -CD28 mAbs in the presence of TGF-β and IL-2 for 3 days. iTreg differentiation was determined by intracellular Foxp3 staining. Each data point represents a single mouse. n.d. = not detected; **p < 0.05.

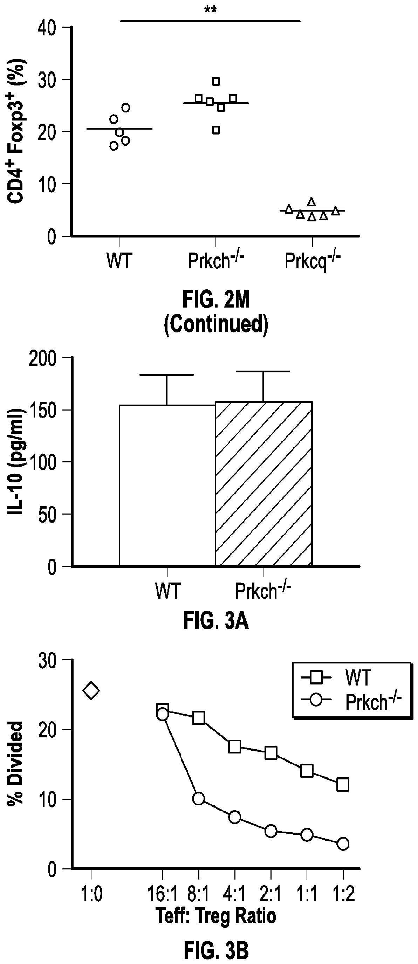

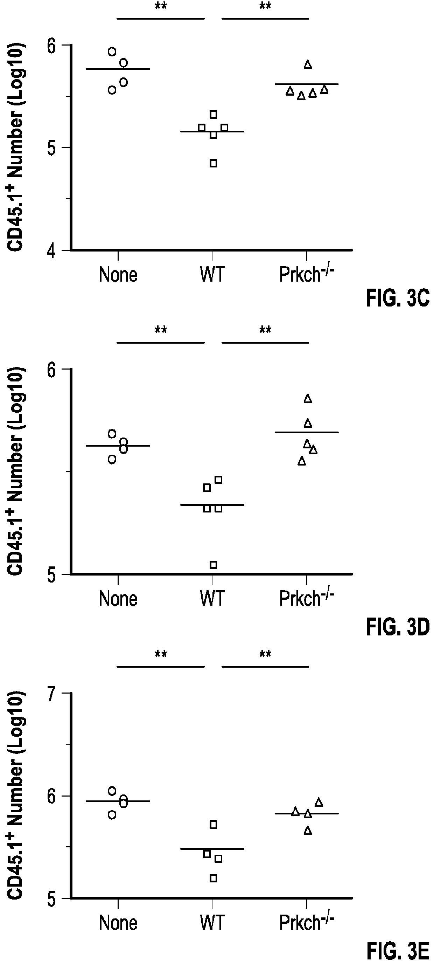

**Figures 3A-3F** **show that contact-dependent suppression of Tregs depends on PKCη.** (A) CD4+GFP+ Treg cells from FIG and Prkch-/--FIG mice were stimulated with plate-bound anti-CD3 mAb and B7-Fc overnight in the presence of IL-2, and the production of IL-10 was measured by ELISA. (B) Treg cells from the same mice were cocultured with CD4+CD25-Teff cells, anti-CD3 mAb and splenic DCs for 3 days. Percentages of CellTrace Violet-diluting Teff cultures at the indicated Treg:Teff ratios were calculated. (C to D) B6.SJL CD45.1+ naive T cells were transferred i.v. alone, or cotransferred with FACS-sorted CD4+GFP+ Tregs from FIG or Prkch-/--FIG mice into Rag1-/- mice. CD45.1+ cells were enumerated in spleens (C), peripheral lymph nodes (D) or mesenteric lymph node (E). Each data point represents a single mouse. (F) CD25-depleted splenic cells (1.5 x 10⁷) from B6 mice were cotransferred without or with 0.5 x 10⁶ CD4+GFP+ Treg cells from FIG or Prkch-/--FIG mice, and inoculated with 2 x 10⁵ B16- F10 cells one day later. Tumor diameters were along two perpendicular axes were measured 2-3 times/week. **p < 0.05.

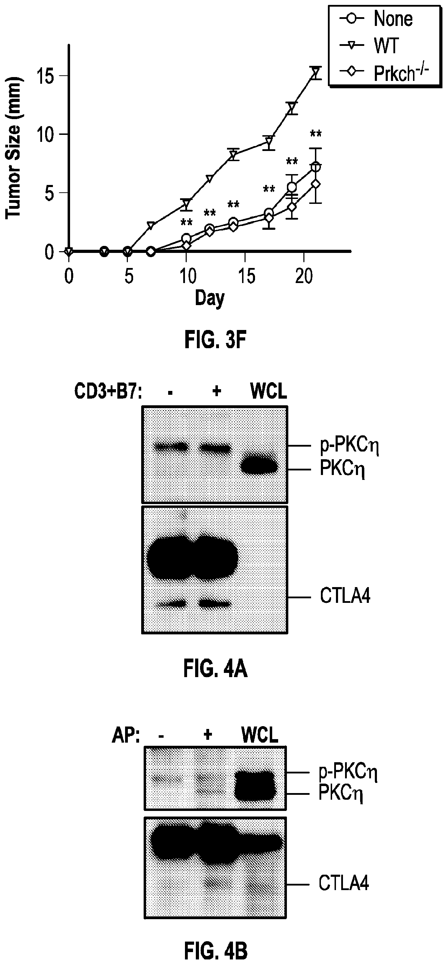

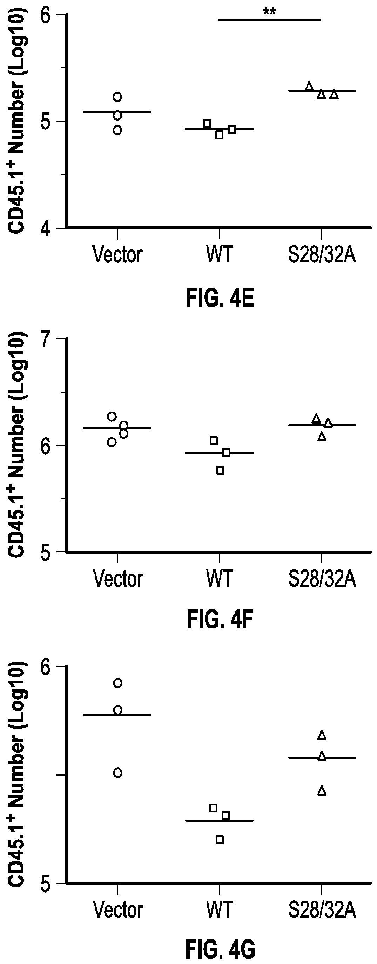

**Figures 4A-4H** **show interaction of phosphorylated PKCη with CTLA-4.** (A) MCC-specific T hybridoma cells were left unstimulated or stimulated for 5 min with anti-CD3 mAb plus B7-Fc. Lysates were immunoprecipitated with anti-CTLA-4, and immunoblotted with the indicated Abs. The two PKCη species are marked by arrowheads. (B) Immunoprecipitated samples were treated with alkaline phosphatase (AP) prior to immunoblotting. (C) Equal numbers of CD4+CD62L+GFP- and CD4+GFP+ cells from FIG mice were FACS-sorted, and cytosol or nuclear fractions were immunoblotted with the indicated Abs. (D) JTAg cells were cotransfected with the indicated PKCη vectors plus WT CTLA-4 and stimulated as in (A) prior to lysis, immunoprecipitation, and immunoblotting. Bottom row shows expression of transfected PKCη proteins in lysates. Schematic representation of human PKCη and phosphorylation sites is shown at the top. (E to G) BM cells from Prkch-/--FIG mice were reconstituted with WT PKCη or a CTLA-4 non-interacting mutant (PKCη-S28/32A) using retroviral pMIG-IRESrCD2 vector. Transduced Tregs (GFP+rCD2+) were FACS-sorted and cotransferred with CD45.1+ naive T cells into Rag1-/- recipients. CD45.1+ cells were enumerated 10 days post-transfer in pLN (E), mLN (F), and spleens (G). Each data point represents a single mouse. **p < 0.05. (H) JTAg cells were cotransfected with the indicated CTLA-4 vectors plus WT PKCη, and processed as in (D). Schematic representation of mouse CTLA-4 is shown at the top.

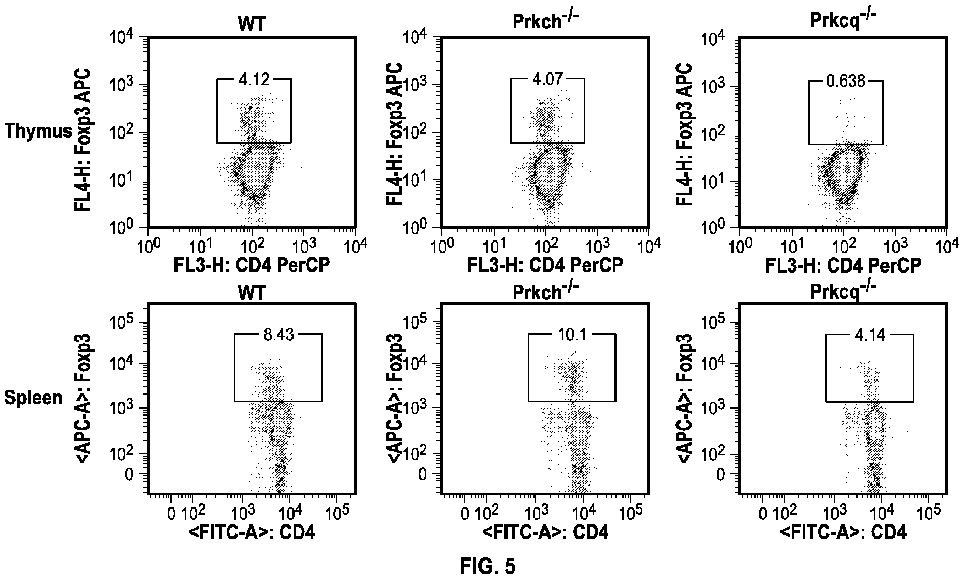

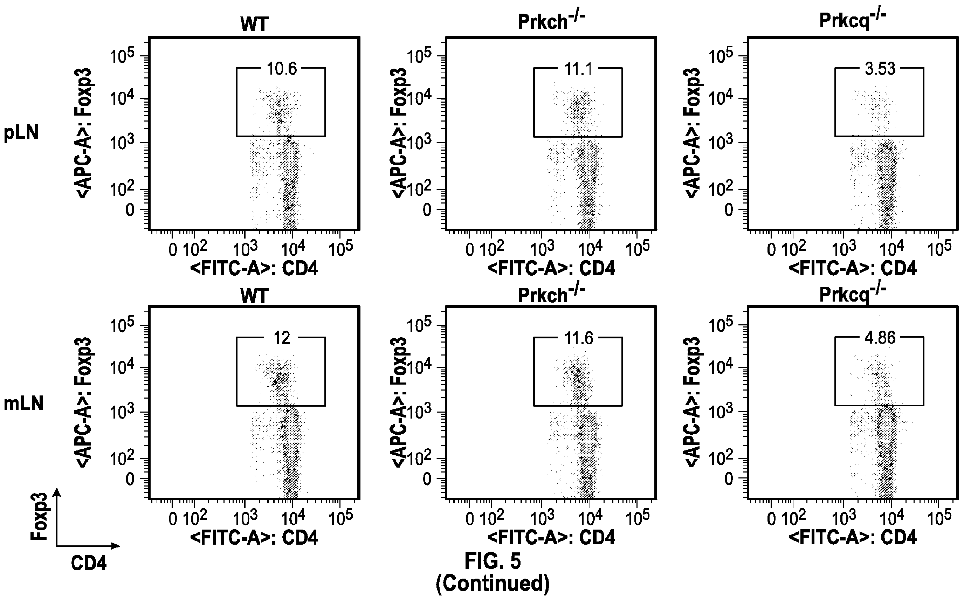

**Figure 5** **shows frequency of Treg population in WT, PKCη- and PKCθ-deficient** mice. The CD4+Foxp3+ cell population from thymi, spleens, peripheral lymph nodes and mesenteric lymph nodes of 8- to 12-week-old WT, Prkcq-/- and Prkch-/- mice were determined by intracellular staining of Foxp3.

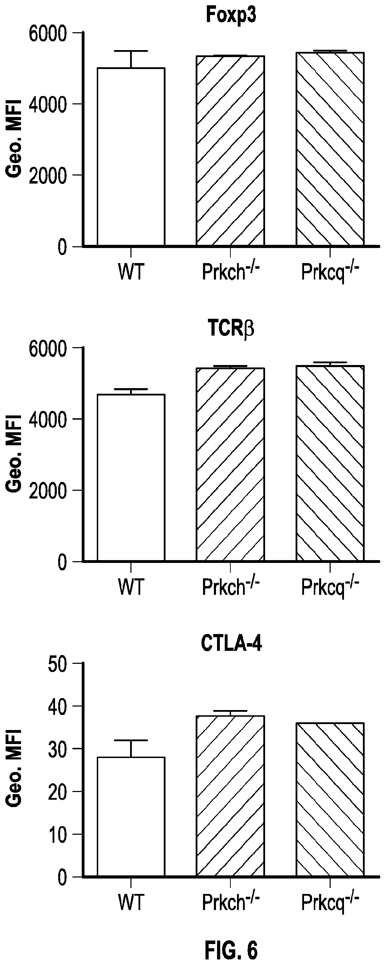

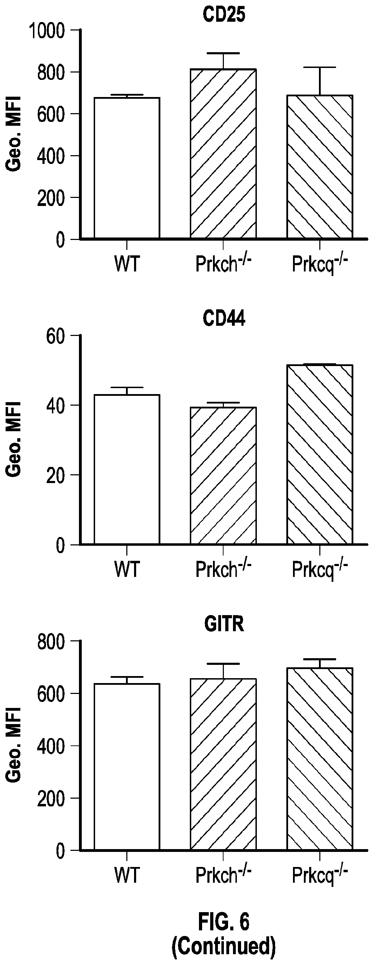

**Figure 6** **shows expression of Foxp3, TCRβ, CTLA-4, CD25, CD44 and GITR among CD4+Foxp3+ cells in WT, PKCη- and PKCθ-deficient mice.** The mean fluorescent intensities (MFI) of Treg markers were determined on gated CD4+Foxp3+ cell population from peripheral lymph nodes of 8- to 12-week-old WT, Prkcq-/- and Prkch-/- mice. Similar data were obtained for CD4+Foxp3+ cells from thymi, spleens and mesenteric lymph nodes.

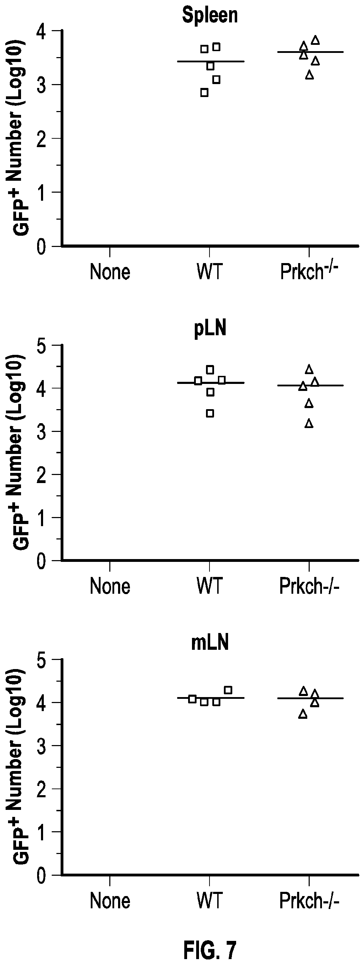

**Figure 7** **shows the number of GFP+ cells recovered from the homeostasis expansion model.** In this model, naive T cells from congenically marked CD45.1+ B6.SJL mice were transferred alone (None), or co-transferred with FACS-sorted CD4+GFP+ Tregs from FIG (WT) or Prkch-/--FIG mice (Prkch-/-) into RAG1-/- mice. The numbers of GFP+ cells were enumerated from spleens, peripheral lymph nodes and mesenteric lymph node. Each data point represents a single mouse.

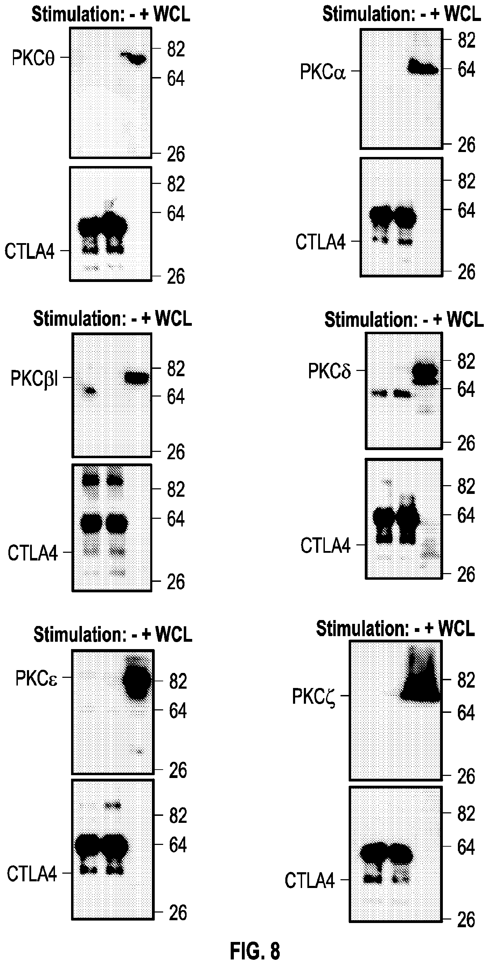

**Figure 8** **shows specific interaction between CTLA4 and PKOCη, but not other PKC isoforms.** MCC-specific T hybridoma cells were left unstimulated or stimulated for 5 min. Cell lysates were prepared, immunoprecipitated with anti-CTLA4 mAb, resolved by SDS-PAGE, and immunoblotted with the indicated PKC Abs.

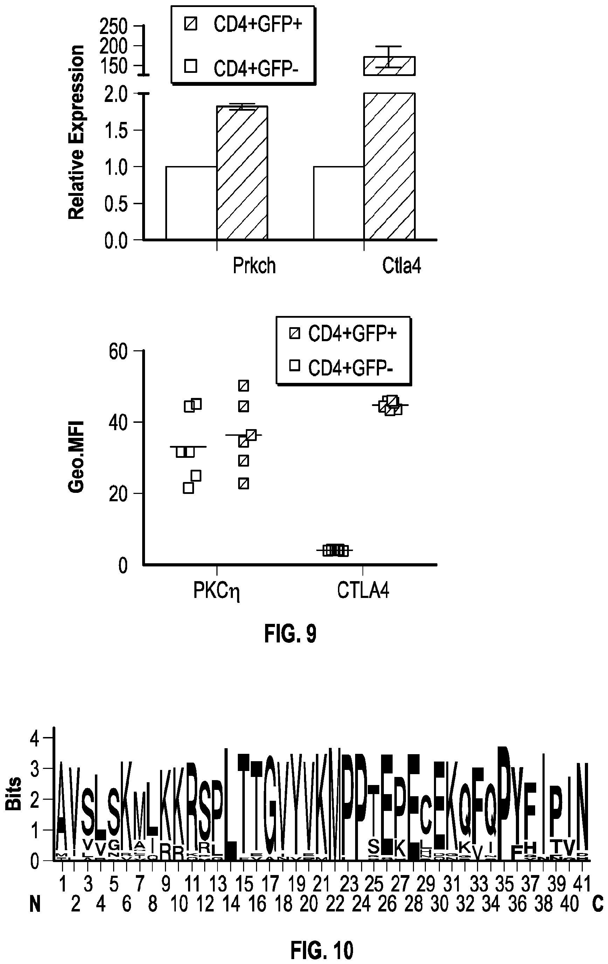

**Figure 9** **shows transcriptional and translational levels of PKCη and CTLA4 in Treg vs. non-Treg cells.** CD4+ T cells were purified from spleens of FIG mice and then FACS-sorted for GFP+ (Tregs) and GFP+ (non-Tregs) populations. Equal number of sorted cells were subjected to RNA purification, reverse transcription and then quantitative PCR to determine the mRNA levels of Prkch and Ctla4 gene. Intracellular staining of PKCη and CTLA4 was performed to determine their respective protein levels.

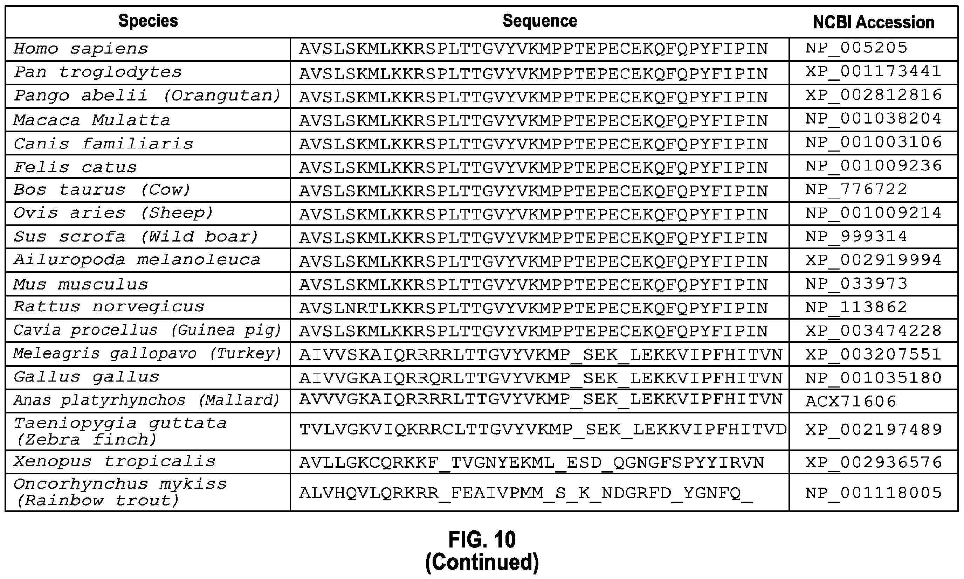

**Figure 10** **shows evolutionary conservation of the positively charged region in the cytoplamic tail of CTLA4.** Protein sequence of putative CTLA4 from indicated organisms were aligned with human CTLA4. Consensus sequence was generated using Weblogo (www.weblogo.berkeley.edu). Membrane proximal positively charged motif (K or R) is bolded.

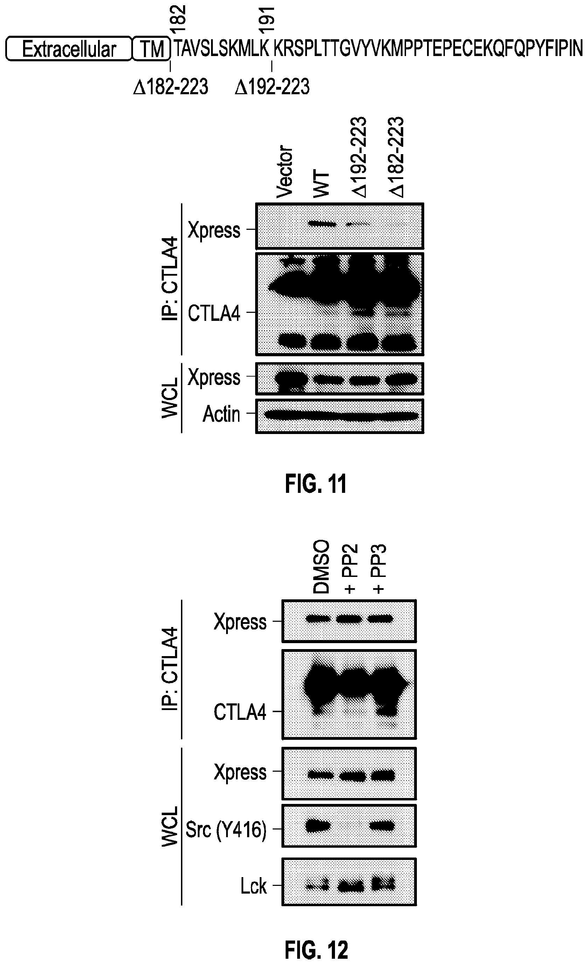

**Figure 11** **shows the effects of CTLA4 tail length in its ability to interact with PKCη Schematic representation of mouse CTLA4 and its cytoplasmic tail.** The cytoplasmic tail of CTLA4 was truncated partially (Δ192-223) or fully (Δ182-223). WT or truncated Ctla4 were cotransfected with Xpress-tagged WT PKCη into JTAg cells. Cells were stimulated with anti-CD3 mAbs + B7-Fc recombinant protein for 5 min and immunoprecipitated with CTLA-4 mAb prior to immunoblotting.
**Figure 12** **shows that interaction between CTLA4-PKCη is independent of Src kinases.** WT Ctla4 were cotransfected with Xpress-tagged WT PKCη into JTAg cells. Cells were treated with vehicle (DMSO), PP2 (10 µM) or PP3 (10 µM) for 30 min prior to stimulation with anti-CD3 mAbs + B7-Fc recombinant protein for 5 min. Cell lysates were immunoprecipitated with CTLA-4 mAb and then immunoblotted with the indicated Abs.

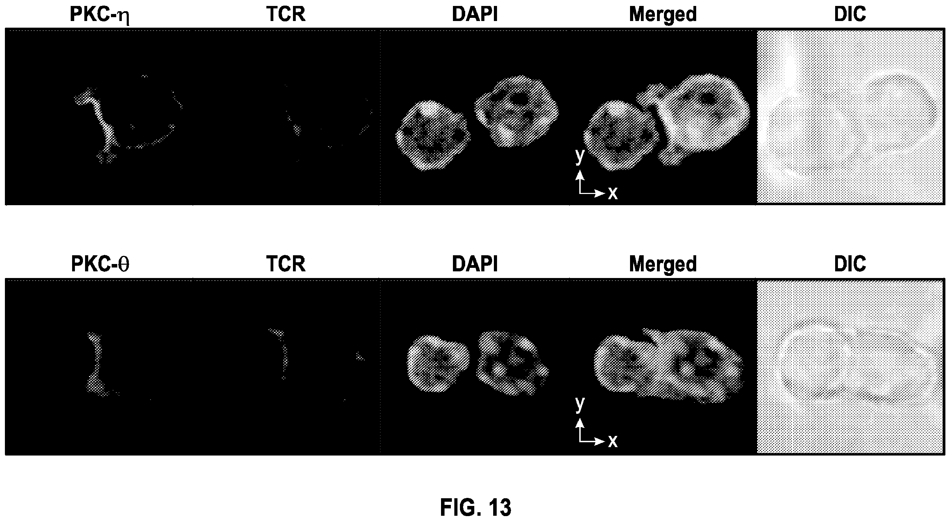

**Figure 13** **shows IS recruitment and CTLA-4 interaction of PKC-η in Treg cells.** Confocal imaging of PKC-η (top) or PKC-θ (bottom) and TCRβ (TCR) localization in in vitro differentiated iTreg cells from AND TCR-Tg Rag2-/- mice, which were retrovirally transduced with eGFP-tagged mouse PKC-θ or PKC-η 1 d after anti-CD3 and anti-CD28 stimulation. Sorted GFP+ T cells (∼90% FoxP3+ by intracellular staining) obtained on day 4 and stimulated for 5-10 min by conjugation with mouse cytochrome C-pulsed lipopolysaccharide-stimulated B cells were fixed and analyzed. eGFP-tagged PKC, TCR and nuclear DAPI staining are shown in green, red and blue, respectively. DIC, differential interference contrast. Data are representative of at least four studies.

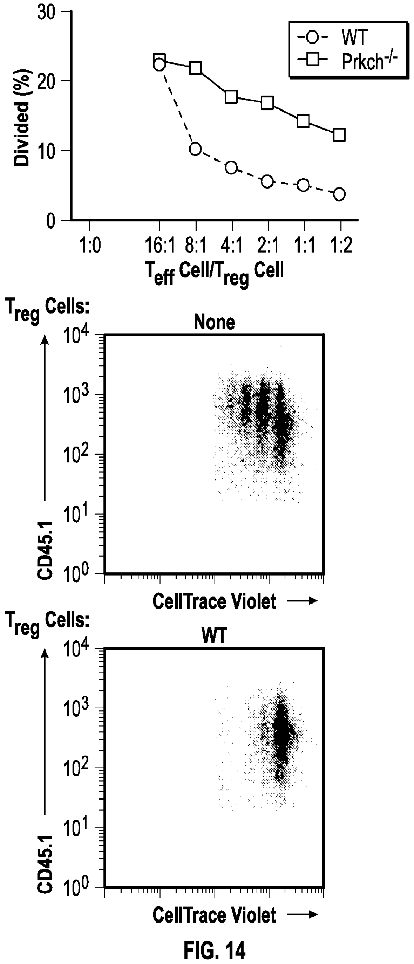

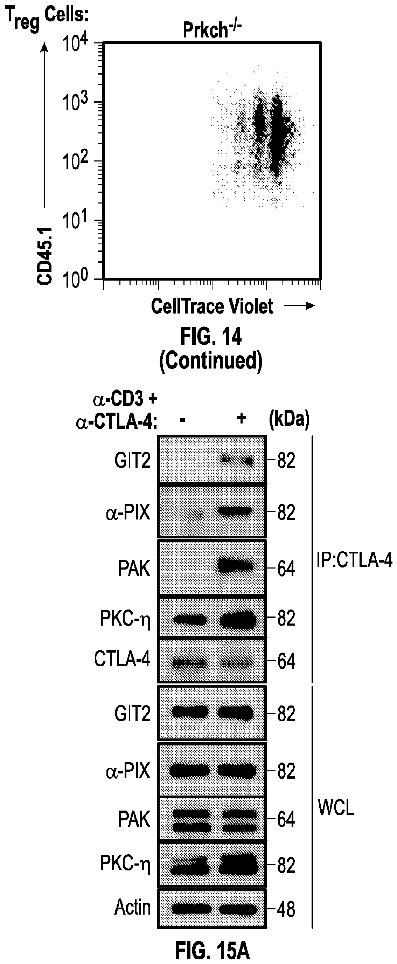

**Figures 14A-14B** **show contact-dependent suppression by Treg cells depends on PKC-η.** In vitro suppression assay measuring the proliferation of CellTrace Violet-labeled naive B6 CD4+CD25- Teff cells cocultured in the absence (1:0) or presence of Foxp3+ Treg cells from Prkch+/+ or Prkch-/- FIG mice at the indicated Teff/Treg cell ratios and stimulated with anti-CD3 mAb and splenic DCs for 3 d. (A) Percentages of CellTrace Violet-diluting Teff cells were calculated. (B) Flow cytometry dot plots of dye-diluting Teff cells cultured with Treg cells at a 1:1 ratio are shown.
**Figures 15A-15E** **show that CTLA-4-PKC-η recruits GIT2-αPIX-PAK complex and modulates Treg cell-APC interaction.** (A) Immunoblot analysis of CTLA-4 IPs or WCL from flow cytometry-sorted Prkch+/+ FIG CD4+GFP+ Treg cells, which were left unstimulated (-) or stimulated (+) with anti-CD3ε plus anti-CTLA-4 mAbs for 5 min. (B) Expression of phospho-PAK1, phospho-PAK2 and total PAK2 in lysates of CD4+GFP+ Treg cells from Prkch+/+ or Prkch-/- FIG mice determined by immunoblotting with antibodies to indicated proteins. (C) Immunoblot analysis of cytosolic (C) and nuclear (N) fractions from cell lysates of flow cytometry-sorted GFP+ Treg cells derived from Prkch+/+ (FIG) or Prkch-/- FIG mice, which were stimulated with anti-CD3 plus anti-CTLA-4 antibodies for 5 min. (D) Conjugation assay measuring formation of cell doublets between flow cytometry-sorted CD4+GFP+ Prkch+/+or Prkch-/- FIG Treg and CellTrace Violet-labeled splenic DCs at different times during a 3 d coculture period in the presence of anti-CD3 mAb and IL-2. Percentages of GFP+ and Violet+ double-positive doublets are shown. *P < 0.05; **P < 0.001. (E) CD86 depletion from APCs cocultured in the absence (DC) or presence of flow cytometry-sorted CD4+GFP+ Prkch+/+ (DC + WT GFP+) or Prkch-/- (DC + Prkch-/- GFP+) FIG Treg cells. A first set of CD45.2+ splenic DCs was cultured for 9 h in the presence of anti-CD3 mAb and IL-2 before the addition of a second set of CD45.1+ splenic DCs. The geometric mean fluorescence intensity (MFI) of CD86 was determined on gated CD11c+ Annexin V- CD45.2+ (left panel) and CD11c+ Annexin V- CD45.1+ (right) cells, respectively. The t_{1/2} values of CD86 decay curves were calculated using the GraphPad Prism program. This study is representative of three independent studies, ns, not significant; *P < 0.05.

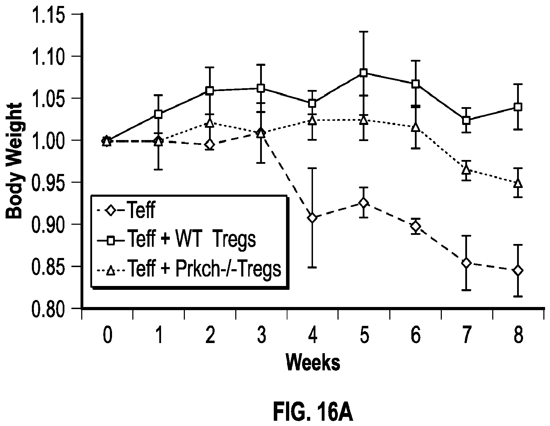

**Figures 16A-16C** **show that Prkch-/- Treg cells protect mice in a T cell transfer model of colitis.** (A) Sorted CD4+CD62L+ naive T cells (Teff) in the absence or presence of WT or Prkch-/- GFP+ Treg cells were cotransferred into Rag1-/- mice and weight was monitored over time as indicated. Mice were sacrificed 10 weeks post-transfer. (B, C) The infiltrating T cell populations in spleens, peripheral lymph nodes (pLN) and mesenteric lymph nodes (mLN) were analyzed by flow cytometry and enumerated. **P < 0.05

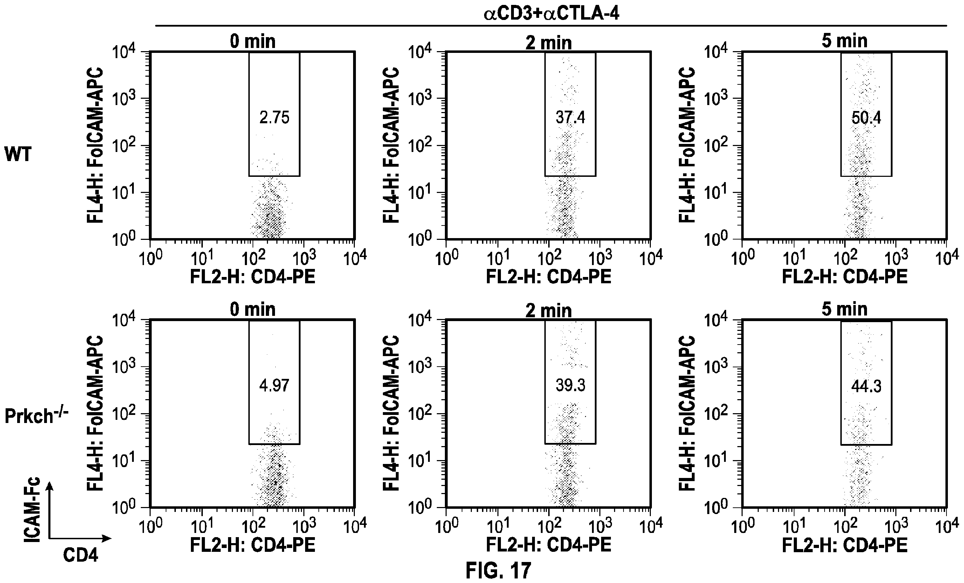

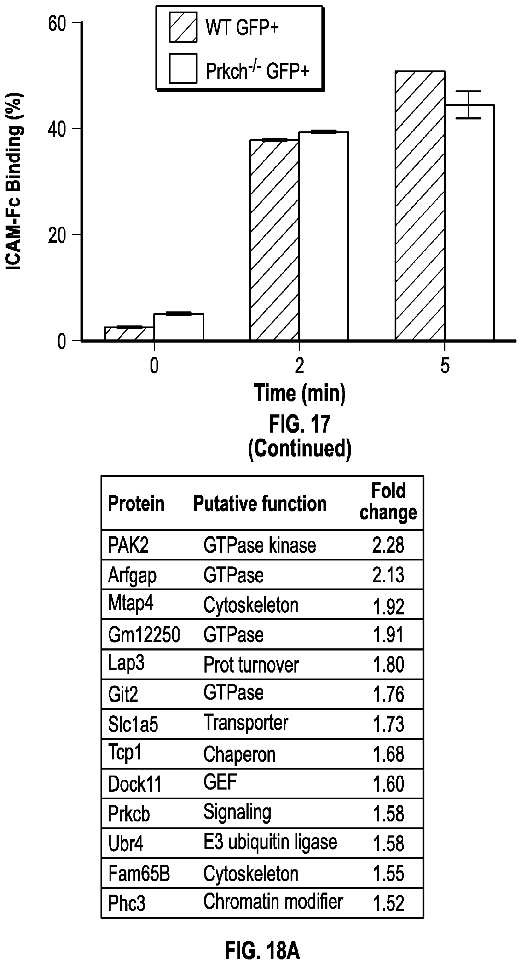

**Figure 17** **shows the effect of Prkch deletion on LFA-1 function.** Purified CD4+ cells from WT or Prkch-/- FIG mice were stimulated with anti-CD3 plus anti-CTLA-4 Abs for the indicated times. The function of LFA-1 was measured by its ability to bind to ICAM1-Fc. Cells were stained with fluorophore-conjugated anti-CD4 and anti-Fc antibodies. Shown are representative data gated on GFP+ cells (top panel) and cumulative data of 2 independent studies (bottom panel).
**Figures 18A-18B** **shows phosphoproteome analysis of CD3- plus CTLA-4-costimulated in vitro-induced Treg cells.** (A) Purified CD4+ T cells from WT or Prkch-/- FIG mice were differentiated for 6 days into iTregs in the presence of TGF-β and IL-2 in standard SILAC media. The cells were stimulated with anti-CD3 plus anti-CTLA-4 mAbs for 5 min before cell lysis and sample preparation for phosphoproteomic analysis. Shown are representative hypophosphorylated proteins in Prkch-/- Tregs as compared to WT Tregs with a fold-change of >1.5. (B) Recruitment of GIT-PIX-PAK complex to CTLA-4-PKC-η complex is dependent on CTLA-4, but not CD28. MCC-specific T hybridoma cells were left unstimulated or stimulated with anti-CD3 and anti-B7, anti-CD3 and anti-CD28, or anti-CD3 and anti-CTLA-4 for 5 min prior to CTLA-4 immunoprecipitation. Immunoblotting was carried out with indicated antibodies.

### DETAILED DESCRIPTION

This disclosure is based, at least in part, on the discovery that diacylglycerol, the PKC-activating second messenger generated by active PLCγ1, is produced locally upon IS formation in Tregs and that this leads to the IS recruitment and activation of a PKC family member, which plays a role in Treg function.

CD4+Foxp3+ regulatory T cells (Tregs) maintain immune homeostasis and self-tolerance during the process of self/nonself discrimination. Formation of an immunological synapse (IS) between Tregs and antigen-presenting cells is a prominent feature of contact-dependent suppression. However, signaling events at the Treg IS remain unknown. Disclosed herein is data showing that protein kinase C-η (PKCη) is recruited to IS of Tregs. Unlike PKCθ, deletion of PKCη did not affect Treg development. However, PKCη-deficient Tregs were grossly impaired in their suppressive functions, including the ability to suppress tumor immunity. Phosphorylated PKCη physically associated with a conserved membrane-proximal motif in CTLA-4, a costimulatory receptor recruited to the IS of Tregs and necessary for their function. Tregs expressing a CTLA-4 non-interacting PKCη mutant were devoid of suppressive activity. These results reveal a unique and novel CTLA-4-PKCη signaling axis for Treg-mediated contact-dependent suppression. Accordingly, the data demonstrate that interfering with CTLA-4-PKCη signaling is a strategy for providing beneficial effects in boosting immune responses.

As disclosed herein, it has been surprisingly discovered that PKCη is a modulator of immune response, including Treg activity. Thus in various examples there are provided methods of modulating immune response, regulatory T cell function and of treatment comprising modulating PKCη and/or the interaction of PKCη with CTLA-4. In further examples, there also are provided agents for modulating PKCη and/or the interaction of PKCη with CTLA-4, including proteins, peptides, antibodies and small molecules and compositions comprising the same, including for example therapeutic compositions.

Accordingly, the disclosure provides, *inter alia,* PKCη polypeptides, subsequences and inhibitors of binding between PKCη and CTLA-4, compositions thereof, and methods and uses of PKCη polypeptides, subsequences and inhibitors of binding between PKCη and CTLA-4. Methods and uses include, for example, modulation and/or treatment of immune responses such as undesirable or aberrant, immune responses, disorders, inflammatory responses, and inflammation. Methods and uses also include, for example, modulation and/or treatment of autoimmune responses, disorders and diseases. Methods and uses further include, for example, modulation (e.g., reducing, inhibiting, suppressing, limiting; or increasing, inducing, stimulating, or promoting) of binding of protein kinase C (PKC) eta (PKCη) and CTLA-4. Methods and uses additionally include, for example, modulation (e.g., increasing, inducing, stimulating, promoting) of regulatory T cell (Tregs) differentiation or function. Methods and uses moreover include, for example, in a subject, such as a mammal (e.g., a primate such as a human).

Compositions, methods and uses include PKCη and CTLA-4 polypeptides, and subsequences and fragments thereof. In one example, a PKCη polypeptide subsequence or fragment is characterized as including or consisting of a subsequence of PKCη (e.g., not full length PKCη) which inhibits or reduces PKCη binding to CTLA-4 (in solution, in solid phase, in a cell, *in vitro, ex vivo,* or *in vivo*). In another example, a CTLA-4 polypeptide subsequence or fragment is characterized as including or consisting of a subsequence of CTLA-4 (e.g., not full length CTLA-4 which inhibits, reduces or decreases PKCη binding to CTLA-4 (in solution, in solid phase, in a cell, *in vitro, ex vivo,* or *in vivo*). Such PKCη and CTLA-4 polypeptide sequences, subsequences/fragments, variants and derivatives, and polymorphisms as set forth herein, are also included as compositions, methods and uses.

In further examples, a subsequence or fragment of a PKCη or CTLA-4 polypeptide includes or consists of one or more amino acids less than full length PKCη and CTLA-4 polypeptides, respectively, and optionally that inhibit, reduce or decrease binding of PKCη to CTLA-4. The term "subsequence" or "fragment" means a portion of the full length molecule. A subsequence of a polypeptide sequence, such as a PKCη and/or CTLA-4 sequence, has one or more amino acids less than a full length PKCη and/or CTLA-4 (*e.g.* one or more internal or terminal amino acid deletions from either amino or carboxy-termini). Subsequences therefore can be any length up to the full length native molecule, provided said length is at least one amino acid less than full length native molecule.

Subsequences can vary in size, for example, from a polypeptide as small as an epitope capable of binding an antibody (*i*.*e*., about five to about eight amino acids) up to a polypeptide that is one amino acid less than the entire length of a reference polypeptide such as PKCη or CTLA-4. In various examples, a polypeptide subsequence is characterized as including or consisting of a PKCη sequence with less than 683 amino acids in length identical to PKCη and a CTLA-4 sequence with less than 223 amino acids in length identical to CTLA-4. Non-limiting exemplary subsequences less than full length PKCη sequence include, for example, a subsequence from about 5 to 10, 10 to 20, 20 to 30, 30 to 50, 50 to 100, 100 to 150, 150 to 200, 200 to 250, 250 to 300, 300 to 400, 400 to 500, 500 to 600, or 600 to 682 amino acids in length. Non-limiting exemplary subsequences less than full length CTLA-4 sequence include, for example, a subsequence from about 5 to 10, 10 to 20, 20 to 30, 30 to 50, 50 to 100, 100 to 150, 150 to 200, 200 to 222 amino acids in length.

As used herein, subsequences may also include or consist of one or more amino acid additions or deletions, wherein the subsequence does not comprise full length native/wild type PKCη or CTLA-4 sequence. Accordingly, total subsequence lengths can be greater than the length of full length native/wild type PKCη or CTLA-4 polypeptide, for example, where a PKCη or CTLA-4 subsequence is fused or forms a chimera with another polypeptide.

PKCη and CTLA-4 polypeptides include mammalian sequences, such as human, gorilla, chimpanzee, orangutan, or macaque PKCη and CTLA-4 sequences. Non-limiting exemplary full length mammalian PKCη polypeptide sequence is as follows (SEQ ID NO:1):

Non-limiting exemplary full length CTLA-4 sequence showing is as follows (SEQ ID NO:2):

As used herein, a "polypeptide" or "peptide" refers to two, or more, amino acids linked by an amide or equivalent bond. A polypeptide can also be referred to herein, *inter alia,* as a protein, or an amino acid sequence, or simply a sequence. Polypeptides include L- and D-isomers, and combinations of L- and D-isomers. Polypeptides can form intra or intermolecular disulfide bonds. Polypeptides can also form higher order structures, such as multimers or oligomers, with the same or different polypeptide, or other molecules. The polypeptides can include modifications typically associated with post-translational processing of proteins, for example, cyclization (*e.g*., disulfide bond), phosphorylation, glycosylation, carboxylation, ubiquitination, myristylation, acetylation (N-terminal), amidation (C-terminal), or lipidation. Polypeptides described herein further include compounds having amino acid structural and functional analogues, for example, peptidomimetics having synthetic or non-natural amino acids or amino acid analogues, so long as the mimetic has one or more functions or activities of a native polypeptide set forth herein. Non-natural and non-amide chemical bonds, and other coupling means can also be included, for example, glutaraldehyde, N-hydoxysuccinimide esters, bifunctional maleimides, or N, N'-dicyclohexylcarbodiimide (DCC). Non-amide bonds can include, for example, ketomethylene aminomethylene, olefin, ether, thioether and the like (see, *e.g*., Spatola (1983) in Chemistry and Biochemistry of Amino Acids, Peptides and Proteins, Vol. 7, pp 267-357, "Peptide and Backbone Modifications," Marcel Decker, NY).

As set forth herein and in particular aspects, a PKCη or CTLA-4 sequence can inhibit, reduce or decrease binding between PKCη and CTLA-4. The term "bind," or "binding," when used in reference to an interaction between PKCη and CTLA-4 means that there is a physical interaction at the molecular level or functional interaction between PKCη and CTLA-4. A functional interaction need not require physical binding. Thus, an inhibitor of binding between PKCη and CTLA-4 partially or completely inhibits, reduces or decreases a physical interaction or a functional interaction between PKCη and CTLA-4. Binding inhibition can be due to steric hinderance, occupation or obstruction or blocking of the site of physical or functional interaction or alteration of a modification or another factor that participates in binding between PKCη and CTLA-4. Accordingly, inhibitors of binding between PKCη and CTLA-4 can act directly or indirectly upon PKCη and/or CTLA-4. For example, a peptide comprising the CTLA-4 binding region of PKCη can be an inhibitor by binding to CTLA-4, or the PKCη binding region of CTLA-4 can be an inhibitor that binds to PKCη, thereby inhibiting binding between PKCη and CTLA-4. Accordingly inhibitors can inhibit, decrease or reduce binding between PKCη and CTLA-4 by interference of a physical or functional interaction of either of these two motifs, for example.

In accordance with particular embodiments, a PKCη sequence includes or consists of a serine at position 28, a serine at position 32 or a serine at position 317. In further particular embodiments, a PKCη amino acid sequence includes or consists of serine at positions 28, 32 and 317, or a subsequence thereof. In still further particular embodiments, a PKCη amino acid sequence includes or consists of residues 28-317 of PKCη amino acid sequence, or a subsequence thereof.

In accordance with particular examples, a CTLA-4 sequence includes or consists of a lysine at position 188, a lysine at position 191, a lysine at position 192, or a lysine at position 193. In further particular examples, a CTLA-4 amino acid sequence includes or consists of lysine at positions 188, 191, 192 and 193, or a subsequence thereof. In still further particular examples, a CTLA-4 amino acid sequence includes or consists of residues 182-223 of CTLA-4 amino acid sequence, or a subsequence thereof.

Accordingly, PKCη and CTLA-4 sequences, subsequences and fragments, and substitutions, variants, derivatives and polymorphisms, as well as methods and uses including PKCη and CTLA-4 sequences, subsequences and fragments, amino acid substitutions, variants, derivatives and polymorphisms include but are not limited to the aforementioned sequence residues or regions. Such forms can be conveniently referred to as variants or derivatives of PKCη and CTLA-4.

As set forth herein, variant and derivative forms also include, for example, in addition to subsequences and fragments, deletions, substitutions, additions, and insertions of the amino acid sequences set forth herein, such as PKCη and CTLA-4. Exemplary sequence deletions, substitutions, additions, and insertions include a full length sequence or a subsequence with one or more amino acids deleted, substituted, added or inserted.

Subsequences, and fragments, variants and derivatives, and polymorphisms can be considered functional as long as they retain at least a partial function or activity of a reference molecule. For example, a functional PKCη subsequence, variant, derivative, or polymorphism would retain at least a partial function or activity of full-length PKCη; a functional CTLA-4 subsequence, variant or derivative, or polymorphism would retain at least a partial function or activity of full-length CTLA-4 (e.g., binding to CTLA-4 or PKCη).

A "functional sequence" or "functional variant," or "functional polymorphism," as used herein refers to a sequence, subsequence, variant or derivative, or polymorphism that possesses at least one partial function or activity characteristic of a native wild type or full length counterpart polypeptide. For example, PKCη or CTLA-4 polypeptide subsequence, variant or derivative, or polymorphism, as disclosed herein, can function to modulate (e.g., inhibit, reduce or decrease) binding between PKCη and CTLA-4. Examples herein include PKCη and CTLA-4 sequences, subsequences, and fragments, variants and derivatives, and polymorphisms that typically retain, at least a part of, one or more functions or activities of a corresponding reference or an unmodified native wild type or full length counterpart PKCη or CTLA-4 sequence. Compositions, methods and uses therefore include PKCη and CTLA-4 polypeptide sequences, subsequences, variants and derivatives, and polymorphisms, having one or more functions or activities of wild type native PKCη and/or CTLA-4.

As disclosed herein, inhibition of binding between PKCη and CTLA-4 polypeptide can lead to various effects on one or more PKCη and/or CTLA-4 functions or activities. Particular non-limiting examples include modulating, such as decreasing, reducing, inhibiting, suppressing, limiting or controlling an undesirable or aberrant immune response, immune disorder, inflammatory response, or inflammation; modulating, such as decreasing, reducing, inhibiting, suppressing, limiting or controlling an autoimmune response, disorder or disease; and modulating, such as increasing, inducing, stimulating, or promoting regulatory T cell (Tregs) differentiation or function. Accordingly, functional sequences therefore include subsequences, variants and derivatives, and polymorphisms, such as PKCη and CTLA-4 sequences that, may have one or more of functions or biological activities described herein or known to one of skill in the art (e.g., ability to modulate binding between PKCη and CTLA-4; modulation of undesirable or aberrant immune responses, immune disorders, inflammatory responses, or inflammation; modulation of autoimmune responses, disorders or diseases; modulation of regulatory T cell (Tregs) differentiation or function, etc.).

PKCη and CTLA-4 sequences, subsequences, variants and derivatives, and polymorphisms may have an activity or function substantially the same or greater or less than 2-5, 5-10, 10-100, 100-1000 or 1000-10,000-fold activity or function than a comparison PKCη and CTLA-4 sequence. For example, a PKCη or CTLA-4 sequence, subsequence or a variant or derivative could have a function or activity greater or less than 2-5, 5-10, 10-100, 100-1000 or 1000-10,000-fold function or activity of a reference PKCη or CTLA-4 to modulate (e.g., decrease, reduce, or inhibit) binding between PKCη and CTLA-4, or to modulate an undesirable or aberrant immune response, immune disorder, inflammatory response, or inflammation; modulate an autoimmune response, disorder or disease; or modulate regulatory T cell (Tregs) differentiation or function.

In particular examples, a functional sequence shares at least 50% identity with a reference sequence, for example, a PKCη or CTLA-4 polypeptide sequence that is capable of modulating (e.g., inhibiting, reducing or decreasing) binding of PKCη to CTLA-4, or modulating an activity, function or expression of PKCη and/or CTLA-4. In other examples, the sequences have at least 60%, 70%, 75% or more identity (e.g., 80%, 85% 90%, 95%, 96%, 97%, 98%, 99% or more identity) to a reference sequence, e.g., PKCη or CTLA-4.

The term "identity" and grammatical variations thereof, mean that two or more referenced entities are the same. Thus, where two polypeptide (e.g., PKCη or CTLA-4) sequences are identical, they have the same amino acid sequence, at least within the referenced region or portion. Where two nucleic acid sequences are identical, they have the same polynucleotide sequence, at least within the referenced region or portion. The identity can be over a defined area (region or domain) of the sequence. An "area of identity" refers to a portion of two or more referenced entities that are the same. Thus, where two protein or nucleic acid sequences are identical over one or more sequence regions they share identity within that region.

The percent identity can extend over the entire sequence length of the polypeptide (e.g., PKCη or CTLA-4). In particular aspects, the length of the sequence sharing the percent identity is 5 or more contiguous amino acids, e.g., 5, 6, 7, 8, 9, 10, 11, 12, 13, 14, 15, 16, 17, 18, 19, 20, 21, 22, 23, 24, 25, 26, 27, 28, 29, 30, etc. contiguous amino acids. In additional particular aspects, the length of the sequence sharing the percent identity is 31 or more contiguous amino acids, e.g., 32, 33, 34, 35, 36, 37, 38, 39, 40, etc. contiguous amino acids. In further particular aspects, the length of the sequence sharing the percent identity is 41 or more contiguous amino acids, e.g., 42, 43, 44, 45, 45, 47, 48, 49, 50, etc., contiguous amino acids. In yet additional particular aspects, the length of the sequence sharing the percent identity is 50 or more contiguous amino acids, e.g., 50-55, 55-60, 60-65, 65-70, 70-75, 75-80, 80-85, 85-90, 90-95, 95-100, 100-110, 110-120, 120-130, 130-140, 140-150, 150-175, 175-200, etc. contiguous amino acids.

The terms "homologous" or "homology" mean that two or more referenced entities share at least partial identity over a given region or portion. "Areas, regions or domains" of homology or identity mean that a portion of two or more referenced entities share homology or are the same. Thus, where two sequences are identical over one or more sequence regions they share identity in these regions. "Substantial homology" means that a molecule is structurally or functionally conserved such that it has or is predicted to have at least partial structure or function of one or more of the structures or functions (*e.g*., a biological function or activity) of the reference molecule, or relevant/corresponding region or portion of the reference molecule to which it shares homology. A PKCη or CTLA-4 sequence, or a subsequence, variant or derivative, or polymorphism with substantial homology has or is predicted to have at least partial activity or function as the reference sequence.

The extent of identity (homology) between two sequences can be ascertained using a computer program and mathematical algorithm known in the art. Such algorithms that calculate percent sequence identity (homology) generally account for sequence gaps and mismatches over the comparison region or area. For example, a BLAST (*e.g*., BLAST 2.0) search algorithm (see, *e.g.,* Altschul et al., J. Mol. Biol. 215:403 (1990), publicly available through NCBI) has exemplary search parameters as follows: Mismatch -2; gap open 5; gap extension 2. For polypeptide sequence comparisons, a BLASTP algorithm is typically used in combination with a scoring matrix, such as PAM100, PAM 250, BLOSUM 62 or BLOSUM 50. FASTA (e.g., FASTA2 and FASTA3) and SSEARCH sequence comparison programs are also used to quantitate extent of identity (Pearson et al., Proc. Natl. Acad. Sci. USA 85:2444 (1988); Pearson, Methods Mol Biol. 132:185 (2000); and Smith et al., J. Mol. Biol. 147:195 (1981)). Programs for quantitating protein structural similarity using Delaunay-based topological mapping have also been developed (Bostick et al., Biochem Biophys Res Commun. 304:320 (2003)).

Variant and derivative polypeptides include, for example, non-conservative and conservative substitutions of PKCη and/or CTLA-4 sequences. In particular examples, a variant protein has one or a few (e.g., 1-5%, 5-10%, 10-20%) of the residues of total protein length, or 1-2, 2-3, 3-4, 5-10, 10-20, 20-50 residues substituted, with conservative or non-conservative substitutions or conservative and non-conservative amino acid substitutions. A "conservative substitution" denotes the replacement of an amino acid residue by another, chemically or biologically similar residue. Biologically similar means that the substitution does not destroy a biological activity or function. Structurally similar means that the amino acids have side chains with similar length, such as alanine, glycine and serine, or a similar size. Chemical similarity means that the residues have the same charge or are both hydrophilic or hydrophobic.

Particular examples of conservative substitutions include the substitution of a hydrophobic residue such as isoleucine, valine, leucine or methionine for another, the substitution of a polar residue for another, such as the substitution of arginine for lysine, glutamic for aspartic acids, or glutamine for asparagine, and the like. A "conservative substitution" also includes the use of a substituted amino acid in place of an unsubstituted parent amino acid.

Variant and derivative proteins also include one or more D-amino acids substituted for L-amino acids (and mixtures thereof), structural and functional analogues, for example, peptidomimetics having synthetic or non-natural amino acids or amino acid analogues and derivatized forms. Variant and derivative proteins further include "chemical derivatives," in which one or more amino acids have a side chain chemically altered or derivatized. Such derivatized polypeptides include, for example, amino acids in which free amino groups form amine hydrochlorides, p-toluene sulfonyl groups, carobenzoxy groups; the free carboxy groups form salts, methyl and ethyl esters; free hydroxl groups that form O-acyl or O-alkyl derivatives as well as naturally occurring amino acid derivatives, for example, 4-hydroxyproline, for proline, 5-hydroxylysine for lysine, homoserine for serine, ornithine for lysine etc. Also included are amino acid derivatives that can alter covalent bonding, for example, the disulfide linkage that forms between two cysteine residues that produces a cyclized polypeptide.

Additions and insertions include, for example, heterologous domains. An addition (e.g., heterologous domain) can be a covalent or non-covalent attachment of any type of molecule to a composition, such as a protein (*e.g*. PKCη or CTLA-4) or other chemical entity (e.g. organic or inorganic compound). Typically additions and insertions (e.g., a heterologous domain) confer a complementary or a distinct function or activity.

Additions and insertions include chimeric and fusion sequences, which is a protein sequence having one or more molecules not normally present in a reference native wild type sequence covalently attached to the sequence. The terms "fusion" or "chimeric" and grammatical variations thereof, when used in reference to a molecule, such as PKCη or CTLA-4, means that a portions or part of the molecule contains a different entity distinct (heterologous) from the molecule (e.g., PKCη or CTLA-4) as they do not typically exist together in nature. That is, for example, one portion of the fusion or chimera, such as PKCη, includes or consists of a portion that does not exist together in nature, and is structurally distinct. A particular example is a molecule, such as amino acid residues or a polypeptide sequence of another protein *(e.g.,* cell penetrating moiety or protein such as HIV tat) attached to PKCη and/or CTLA-4 subsequence to produce a chimera, or a chimeric polypeptide, to impart a distinct function (e.g., increased cell penetration).

In particular examples, additions and insertions include a cell-penetrating moiety (CPM), or a cell-penetrating peptide (CPP). As used herein, a "cell-penetrating moiety (CPM)" is a molecule that penetrates or passes through cell membranes, typically without a need for binding to a cell membrane receptor. A cell penetrating peptide (CPP) can penetrate membranes, and is typically a peptide sequence of less that 25-50 (more typically, 30) amino acid residues in length. In particular non-limiting examples, a CPM or CPP includes HIV Tat, Drosophila antennapedia (RQIKIWFQNRRMKWKK (SEQ ID NO: 4)), polyarginine (RRRRRRRRR (SEQ ID NO: 5)), polylysine (KKKKKKKKK (SEQ ID NO: 6)), PTD-5 (RRQRRTSKLMKR (SEQ ID NO:7)), or a Transportan (GWTLNSAGYLLGKINLKALAALAKKIL (SEQ ID NO: 8)), or KALA (WEAKLAKALAKALAKHLAKALAKALKACEA (SEQ ID NO:9)) sequence.

Additions and insertions further include labels and tags, which can be used to provide detection or that is useful for isolating the tagged entity (e.g., PKCη or CTLA-4 sequence). A detectable label can be attached (e.g., linked or conjugated), for example, to a PKCη or CTLA-4 sequence, or be within or comprise one or more atoms that comprise the molecule.

Non-limiting exemplary detectable labels include a radioactive material, such as a radioisotope, a metal or a metal oxide. Radioisotopes include radionuclides emitting alpha, beta or gamma radiation, such as one or more of: ³H, ¹⁰B, ¹⁸F, ¹¹C, ¹⁴C, ¹³N, ¹⁸O, ¹⁵O, ³²P, P³³, ³⁵S, ³⁵Cl, ⁴⁵Ti, ⁴⁶SC, ⁴⁷Sc, ⁵¹Cr, ⁵²Fe,⁵⁹Fe, ^{.57}Co, ⁶⁰Cu, ⁶¹Cu, ⁶²Cu, ⁶⁴Cu, ⁶⁷Cu, ⁶⁷Ga, ⁶⁸Ga, ⁷²As ⁷⁶Br, ⁷⁷Br, ^{81m}Kr, ⁸²Rb, ⁸⁵Sr, ⁸⁹Sr, ⁸⁶Y, ⁹⁰Y, ⁹⁵Nb, ^{94m}Tc, ^{99m}Tc, ⁹⁷Ru, ¹⁰³Ru, ¹⁰⁵Rh, ¹⁰⁹Cd, ¹¹¹In, ¹¹³Sn, ^{113m}In, ¹¹⁴In, I¹²⁵, I¹³¹, ¹⁴⁰La, ¹⁴¹Ce, ¹⁴⁹Pm, ¹⁵³Gd, ¹⁵⁷Gd, ¹⁵³Sm, ¹⁶¹Tb, ¹⁶⁶Dy, ¹⁶⁶Ho, ¹⁶⁹Er, ¹⁶⁹Y, ¹⁷⁵Yb, ¹⁷⁷LU, ¹⁸⁶Re, ¹⁸⁸Re, ²⁰¹Tl, ²⁰³Pb, ²¹¹At, ²¹²Bi or ²²⁵Ac. Additional non-limiting exemplary detectable labels include a metal or a metal oxide, such as gold, silver, copper, boron, manganese, gadolinium, iron, chromium, barium, europium, erbium, praseodynium, indium, or technetium.

Further non-limiting exemplary detectable labels include contrast agents (e.g., gadolinium; manganese; barium sulfate; an iodinated or noniodinated agent; an ionic agent or nonionic agent); magnetic and paramagnetic agents (e.g., iron-oxide chelate); nanoparticles; an enzyme (horseradish peroxidase, alkaline phosphatase, -galactosidase, or acetylcholinesterase); a prosthetic group (e.g., streptavidin/biotin and avidin/biotin); a fluorescent material (e.g., umbelliferone, fluorescein, fluorescein isothiocyanate, rhodamine, dichlorotriazinylamine fluorescein, dansyl chloride or phycoerythrin); a luminescent material (e.g., luminol); or a bioluminescent material (e.g., luciferase, luciferin, aequorin).

Still further non-limiting tags and/or detectable labels include enzymes (horseradish peroxidase, urease, catalase, alkaline phosphatase, beta-galactosidase, chloramphenicol transferase); enzyme substrates; ligands (*e.g*., biotin); receptors (avidin); GST-, T7-, His-, myc-, HA- and FLAG-tags; electron-dense reagents; energy transfer molecules; paramagnetic labels; fluorophores (fluorescein, fluorscamine, rhodamine, phycoerthrin, phycocyanin, allophycocyanin); chromophores; chemi-luminescent (imidazole, luciferase, acridinium, oxalate); and bio-luminescent agents.

As set forth herein, a detectable label or tag can be linked or conjugated (e.g., covalently) to the molecule (e.g., PKCη and/or CTLA-4 sequence). In various embodiments a detectable label, such as a radionuclide or metal or metal oxide can be bound or conjugated to the agent, either directly or indirectly. A linker or an intermediary functional group can be used to link the molecule to a detectable label or tag. Linkers include amino acid or peptidomimetic sequences inserted between the molecule and a label or tag so that the two entities maintain, at least in part, a distinct function or activity. Linkers may have one or more properties that include a flexible conformation, an inability to form an ordered secondary structure or a hydrophobic or charged character which could promote or interact with either domain. Amino acids typically found in flexible protein regions include Gly, Asn and Ser. The length of the linker sequence may vary without significantly affecting a function or activity.

Linkers further include chemical moieties, conjugating agents, and intermediary functional groups. Examples include moieties that react with free or semi-free amines, oxygen, sulfur, hydroxy or carboxy groups. Such functional groups therefore include mono and bifunctional crosslinkers, such as sulfo-succinimidyl derivatives (sulfo-SMCC, sulfo-SMPB), in particular, disuccinimidyl suberate (DSS), BS3 (Sulfo-DSS), disuccinimidyl glutarate (DSG) and disuccinimidyl tartrate (DST). Non-limiting examples include diethylenetriaminepentaacetic acid (DTPA) and ethylene diaminetetracetic acid.

Modifications can be produced using methods known in the art (*e.g.,* PCR based site-directed, deletion and insertion mutagenesis, chemical modification and mutagenesis, crosslinking, etc.), or may be spontaneous or naturally occurring (e.g. random mutagenesis). For example, naturally occurring allelic variants can occur by alternative RNA splicing, polymorphisms, or spontaneous mutations of a nucleic acid encoding PKCη or CTLA-4 sequence. Further, deletion of one or more amino acids can also result in a modification of the structure of the resultant polypeptide without significantly altering a biological function or activity. Deletion of amino acids can lead to a smaller active molecule. For example, removal of PKCη and CTLA-4 amino acids does not destroy the ability of such a variant PKCη or CTLA-4 to inhibit binding between PKCη and CTLA-4.

The term "isolated," when used as a modifier of a composition (e.g., PKCη or CTLA-4 sequences, subsequences, variant and derivatives, etc.), means that the compositions are made by the hand of man or are separated, completely or at least in part, from their naturally occurring *in vivo* environment. Generally, isolated compositions are substantially free of one or more materials with which they normally associate with in nature, for example, one or more protein, nucleic acid, lipid, carbohydrate, cell membrane. The term "isolated" does not exclude alternative physical forms, such as fusions/chimeras, multimers/oligomers, modifications (e.g., phosphorylation, glycosylation, lipidation) or derivatized forms, or recombinant or other forms expressed in vitro, in host cells, or in an animal and produced by the hand of man.

An "isolated" composition (e.g., a PKCη or CTLA-4 sequence) can also be "substantially pure" or "purified" when free of most or all of the materials with which it typically associates with in nature. Thus, an isolated sequence that also is substantially pure or purified does not include polypeptides or polynucleotides present among millions of other sequences, such as antibodies of an antibody library or nucleic acids in a genomic or cDNA library, for example. Typically, purity can be at least about 50%, 60% or more by mass. The purity can also be about 70% or 80% or more, and can be greater, for example, 90% or more. Purity can be determined by any appropriate method, including, for example, UV spectroscopy, chromatography (*e.g*., HPLC, gas phase), gel electrophoresis and sequence analysis (nucleic acid and peptide), and is typically relative to the amount of impurities, which typically does not include inert substances, such as water.

A "substantially pure" or "purified" composition can be combined with one or more other molecules. Thus, "substantially pure" or "purified" does not exclude combinations of compositions, such as combinations of PKCη or CTLA-4 sequences, subsequences, variants and derivatives, and other molecular entities such as agents, drugs or therapies.

As used herein, the term "recombinant," when used as a modifier of sequences such as polypeptides and polynucleotides, means that the compositions have been manipulated (*i.e.,* engineered) in a fashion that generally does not occur in nature (*e.g*., *in vitro*)*.* A particular example of a recombinant polypeptide would be where a PKCη or CTLA-4 polypeptide is expressed by a cell transfected with a polynucleotide encoding the PKCη or CTLA-4 sequence. A particular example of a recombinant polynucleotide would be where a nucleic acid (*e.g.,* genomic or cDNA) encoding PKCη or CTLA-4 cloned into a plasmid, with or without 5', 3' or intron regions that the gene is normally contiguous with in the genome of the organism. Another example of a recombinant polynucleotide or polypeptide is a hybrid or fusion sequence, such as a chimeric PKCη or CTLA-4 sequence comprising a second sequence, such as a heterologous functional domain.

The disclosure also provides polynucleotides encoding PKCη and CTLA-4 sequences that modulate binding between PKCη and CTLA-4. In one example, a polynucleotide sequence has about 65% or more identity (e.g., 70%, 75%, 80%, 85%, 90%, 95%, 96%, 97%, 98%, 99% or more) to a sequence encoding a PKCη or CTLA-4 subsequence that modulates binding between PKCη and CTLA-4. In particular examples, a nucleic acid encodes amino acids of a PKCη or CTLA-4 subsequence. Such polynucleotides can therefore encode any subsequence of PKCη or CTLA-4 sequence that includes or consists of a region that binds to PKCη or CTLA-4, or that modulates binding between PKCη and CTLA-4.

As used herein, the terms "polynucleotide" and "nucleic acid" are used interchangeably to refer to all forms of nucleic acid, oligonucleotides, primers, and probes, including deoxyribonucleic acid (DNA) and ribonucleic acid (RNA). Polynucleotides include genomic DNA, cDNA and antisense DNA, and spliced or unspliced mRNA, rRNA tRNA and antisense RNA *(e.g.,* RNAi). Polynucleotides include naturally occurring, synthetic, and intentionally altered or modified polynucleotides as well as analogues and derivatives. Alterations can result in increased stability due to resistance to nuclease digestion, for example. Polynucleotides can be double, single or triplex, linear or circular, and can be of any length.

Polynucleotides include sequences that are degenerate as a result of the genetic code. There are 20 natural amino acids, most of which are specified by more than one codon. Degenerate sequences may not selectively hybridize to other nucleic acids; however, they are nonetheless included as they encode PKCη and CTLA-4 sequences, subsequences, variants and derivatives, and polymorphisms thereof. Thus, in another example, degenerate nucleotide sequences that encode PKCη and CTLA-4 sequences, subsequences, variants and derivatives, and polymorphisms, as set forth herein, are provided.

Polynucleotide sequences include sequences having 15-20, 20-30, 30-40, 50-75, 75-100, 100-150, 150-200, or more contiguous nucleotides. In additional examples, the polynucleotide sequence includes a sequence having 200 or more, 250 or more, 300 or more, 400 or more, 500 or more, 600 or more, 700 or more contiguous nucleotides, up to the full length coding sequence.

Polynucleotide sequences include complementary sequences (*e.g*., antisense to all or a part of PKCη and/or CTLA-4). Antisense may be encoded by a nucleic acid and such a nucleic acid may be operatively linked to an expression control element for sustained or increased expression of the encoded antisense in cells or *in vivo.*

Polynucleotides can be obtained using various standard cloning and chemical synthesis techniques. Purity of polynucleotides can be determined through sequencing, gel electrophoresis and the like. For example, nucleic acids can be isolated using hybridization as set forth herein or computer-based database screening techniques known in the art. Such techniques include, but are not limited to: (1) hybridization of genomic DNA or cDNA libraries with probes to detect homologous nucleotide sequences; (2) antibody screening to detect polypeptides having shared structural features, for example, using an expression library; (3) polymerase chain reaction (PCR) on genomic DNA or cDNA using primers capable of annealing to a nucleic acid sequence of interest; (4) computer searches of sequence databases for related sequences; and (5) differential screening of a subtracted nucleic acid library.

PKCη and CTLA-4 polynucleotides can include an expression control element distinct from an endogenous *PKCη* or *CTLA-4* gene (e.g., a non-native element), or exclude a control element from the native *PKCη* or *CTLA-4* gene to control expression of an operatively linked nucleic acid. Such polynucleotides containing an expression control element controlling expression of a nucleic acid can be modified or altered as set forth herein, so long as the modified or altered polynucleotide has one or more functions or activities.

For expression in cells, polynucleotides, if desired, may be inserted into a vector. Accordingly, compositions and methods further include polynucleotide sequences inserted into a vector. The term "vector" refers to a plasmid, virus or other vehicle known in the art that can be manipulated by insertion or incorporation of a polynucleotide. Such vectors can be used for genetic manipulation (*i.e.,* "cloning vectors") or can be used to transcribe or translate the inserted polynucleotide (*i*.*e*., "expression vectors"). A vector generally contains at least an origin of replication for propagation in a cell and a promoter. Control elements, including expression control elements as set forth herein, present within a vector are included to facilitate proper transcription and translation (*e.g*., splicing signal for introns, maintenance of the correct reading frame of the gene to permit in-frame translation of mRNA and, stop codons etc.).

Compositions, methods and uses include PKCη and/or CTLA-4 sequences which can include any amount or dose of PKCη or CTLA-4 sequence, subsequence, variant or derivative, or polymorphism. In particular examples, PKCη or CTLA-4 is in a concentration range of about 10 µg/ml to 100 mg/ml, or in a range of about 100 µg/ml to 1,000 mg/ml, or at a concentration of about 1 mg/ml. In further particular examples, PKCη or CTLA-4 is in an amount of 10-1,000 milligrams, or an amount of 10-100 milligrams.

As disclosed herein, methods and uses include modulating (e.g., reducing, inhibiting, suppressing, or limiting) binding between PKCη and CTLA-4. An agent for use in a method of treating cancer and an *in vitro* method of modulating regulatory T cell function comprising modulating interaction of PKCη with CTLA-4 are defined in the claims. The disclosure also describes by way of example only methods and uses that can be performed *in vivo,* such as in a subject, *ex vivo,* in a cell, in solution, in solid phase or in silica. In one example, a method or use includes contacting an inhibitor of binding between PKCη and CTLA-4 thereby reducing, inhibiting, decreasing, suppressing, or limiting binding between PKCη and CTLA-4.

As used herein, the term "modulate," means an alteration or effect of the term modified. For example, the term modulate can be used in various contexts to refer to an alteration or effect of an activity, a function, or expression of a polypeptide, gene or signaling pathway, or a physiological condition or response of an organism. Methods and uses include modulating (e.g., decrease, reduce, inhibit, suppress, limit or control) one or more functions, activities or expression of PKCη or CTLA-4 in solution, in solid phase, in a cell, *in vitro, ex vivo* or *in vivo.* Thus, where the term "modulate" is used to modify the term "PKCη" or "CTLA-4" this means that a PKCη or CTLA-4 activity, function, or expression is altered or affected (e.g., decreased, reduced, inhibited, suppressed, limited, controlled or prevented, etc.). Detecting an alteration or an effect on PKCη or CTLA-4 activity, function or expression can be determined as set forth herein using cell based, *in vitro* or *in vivo* assays, such as an animal model.

As disclosed herein, inhibition of binding between PKCη and CTLA-4 polypeptide can lead to various consequences, such as effects on a PKCη and/or CTLA-4 function or activity. Accordingly, PKCη and CTLA-4 sequences, subsequences, variants and derivatives, and polymorphisms as disclosed herein, including compositions including PKCη and/or CTLA-4, are useful in various methods and uses such as modulation and treatment methods and uses, including, for example, treatment of numerous responses, disorders and diseases, both chronic and acute. In one example, a method of treating a PKCη mediated or dependent response, disorder, or disease, includes administering an inhibitor of binding between PKCη and CTLA-4 to a subject in an amount that treats the PKCη mediated or dependent response, disorder, or disease.

Responses, disorders and diseases include, without limitation, aberrant or undesirable cell proliferation or a cell proliferative or hyperproliferative disorder. A "hyperproliferative disorder" refers to any undesirable or aberrant cell survival (e.g., failure to undergo programmed cell death or apoptosis), growth or proliferation. Such disorders include benign hyperplasias, non-metastatic tumors and metastatic tumors. Such disorders can affect any cell, tissue, organ in a subject. Such disorders can be present in a subject, locally, regionally or systemically.

As used herein, the terms "tumor," "cancer," "malignancy," and "neoplasia" are used interchangeably and refer to a cell or population of cells whose growth, proliferation or survival is greater than growth, proliferation or survival of a normal counterpart cell, e.g. a cell proliferative or differentiative disorder. Such disorders can affect virtually any cell or tissue type, e.g., carcinoma, sarcoma, melanoma, neural, and reticuloendothelial or haematopoietic neoplastic disorders (e.g., myeloma, lymphoma or leukemia). A tumor can arise from a multitude of tissues and organs, including but not limited to breast, lung, thyroid, head and neck, brain, lymphoid, gastrointestinal (mouth, esophagus, stomach, small intestine, colon, rectum), genito-urinary tract (uterus, ovary, cervix, bladder, testicle, penis, prostate), kidney, pancreas, liver, bone, muscle, skin, which may or may not metastasize to other secondary sites.

The tumor, cancer or malignancy may be in any stage, e.g., early or advanced, such as a stage I, II, III, IV or V tumor, cancer or malignancy. The tumor, cancer or malignancy may have been subject to a prior treatment or be stabilized (non-progressing) or in remission.

Cells comprising a tumor, cancer or malignancy may be aggregated in a cell mass or be dispersed. A "solid tumor" refers to neoplasia or metastasis that typically aggregates together and forms a mass. Specific non-limiting examples include visceral tumors such as melanomas, breast, pancreatic, uterine and ovarian cancers, testicular cancer, including seminomas, gastric or colon cancer, hepatomas, adrenal, renal and bladder carcinomas, lung, head and neck cancers and brain tumors/cancers.

Carcinomas, which refer to malignancies of epithelial or endocrine tissue, include respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from the uterine cervix, lung, prostate, breast, head and neck, colon, pancreas, testes, adrenal, kidney, esophagus, stomach, liver and ovary. The term also includes carcinosarcomas, *e.g*., which include malignant tumors composed of carcinomatous and sarcomatous tissues. Adenocarcinoma includes a carcinoma of a glandular tissue, or in which the tumor forms a gland like structure.

Melanoma, which refers to malignant tumors of melanocytes and other cells derived from pigment cell origin that may arise in the skin, the eye (including retina), or other regions of the body, include the cells derived from the neural crest that also gives rise to the melanocyte lineage. A pre-malignant form of melanoma, known as dysplastic nevus or dysplastic nevus syndrome, is associated with melanoma development.

Sarcomas refer to malignant tumors of mesenchymal cell origin. Exemplary sarcomas include for example, lymphosarcoma, liposarcoma, osteosarcoma, chondrosarcoma, leiomyosarcoma, rhabdomyosarcoma and fibrosarcoma.

Neural neoplasias include glioma, glioblastoma, meningioma, neuroblastoma, retinoblastoma, astrocytoma, oligodendrocytoma
A "liquid tumor," which refers to neoplasia that is diffuse in nature, as they do not typically form a solid mass. Particular examples include neoplasia of the reticuloendothelial or haematopoetic system, such as lymphomas, myelomas and leukemias. Non-limiting examples of leukemias include acute and chronic lymphoblastic, myeolblastic and multiple myeloma. Typically, such diseases arise from poorly differentiated acute leukemias, *e.g*., erythroblastic leukemia and acute megakaryoblastic leukemia. Specific myeloid disorders include, but are not limited to, acute promyeloid leukemia (APML), acute myelogenous leukemia (AML) and chronic myelogenous leukemia (CML). Lymphoid malignancies include, but are not limited to, acute lymphoblastic leukemia (ALL), which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Specific malignant lymphomas include, non-Hodgkin lymphoma and variants, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

Responses, disorders and diseases also include, without limitation, immune responses, disorders and diseases, inflammatory responses, disorders and diseases, and inflammation. Responses, disorders and diseases also include, without limitation, autoimmune responses, disorders and diseases. Responses additionally include regulatory T cell (Tregs) differentiation or function. Responses, disorders and diseases further include, without limitation, graft vs. host disease (GVHD), or host rejection of a cell, tissue or organ transplant (such as heart, liver, lung, bone marrow, etc.).

Accordingly, the disclosure describes methods and uses of modulating and treatment of all the foregoing immune responses, disorders and disease. In one example, a method includes administering an inhibitor of binding between PKCη and CTLA-4 to a subject in an amount to decrease, reduce, inhibit, suppress, limit or control the undesirable or aberrant immune responses, disorders or diseases, inflammatory responses, disorders or diseases or inflammation in the subject. In another example, a method includes administering an inhibitor of binding between PKCη and CTLA-4 to a subject in an amount to decrease, reduce, inhibit, suppress, limit or control an autoimmune response, disorder or disease in the subject. In an additional example, a method includes contacting an inhibitor of binding between PKCη and CTLA-4 in an amount effective for increasing, inducing, stimulating, or promoting regulatory T cell differentiation or function. In a further example, a method includes administering an inhibitor of binding between PKCη and CTLA-4 to a subject in an amount to decrease, reduce, inhibit, suppress, limit or control GVHD, or host rejection of a cell, tissue or organ transplant (such as heart, liver, lung, bone marrow, etc.).

Responses, disorders and diseases treatable in accordance with examples include, but are not limited to, treatment of acute and chronic undesirable or aberrant immune responses, disorders or diseases, inflammatory responses, disorders or diseases or inflammation. Responses, disorders and diseases treatable in accordance with examples herein also include, but are not limited to treatment of acute and chronic autoimmune responses, disorders and diseases. Such responses, disorders and diseases may be antibody or cell mediated, or a combination of antibody and cell mediated.

As used herein, an "undesirable immune response" or "aberrant immune response" refers to any immune response, activity or function that is greater or less than desired or physiologically normal response, activity or function including, acute or chronic responses, activities or functions. "Undesirable immune response" is generally characterized as an undesirable or aberrant increased or inappropriate response, activity or function of the immune system. However, an undesirable immune response, function or activity can be a normal response, function or activity. Thus, normal immune responses so long as they are undesirable, even if not considered aberrant, are included within the meaning of these terms. An undesirable immune response, function or activity can also be an abnormal response, function or activity. An abnormal (aberrant) immune response, function or activity deviates from normal.

One non-limiting example of an undesirable or aberrant immune response is where the immune response is hyper-responsive, such as in the case of an autoimmune disorder or disease. Another non-limiting example of an undesirable or aberrant immune response is where an immune response leads to acute or chronic inflammatory response or inflammation in any tissue or organ, such as an allergy, Crohn's disease, inflammatory bowel disease (IBD) or ulcerative colitis, or a transplant, as in GVHD (graft vs. host disease) or host rejection of a cell, tissue or organ transplant.

Undesirable or aberrant immune responses, inflammatory responses, or inflammation are characterized by many different physiological adverse symptoms or complications, which can be humoral, cell-mediated or a combination thereof. Responses, disorders and diseases that can be treated in accordance with examples herein include, but are not limited to, those that either directly or indirectly lead to or cause cell or tissue/organ damage in a subject. At the whole body, regional or local level, an immune response, inflammatory response, or inflammation can be characterized by swelling, pain, headache, fever, nausea, skeletal joint stiffness or lack of mobility, rash, redness or other discoloration. At the cellular level, an immune response, inflammatory response, or inflammation can be characterized by one or more of T cell activation and/or differentiation, cell infiltration of the region, production of antibodies, production of cytokines, lymphokines, chemokines, interferons and interleukins, cell growth and maturation factors (e.g., proliferation and differentiation factors), cell accumulation or migration and cell, tissue or organ damage. Thus, methods and uses include treatment of and an ameliorative effect upon any such physiological symptoms or cellular or biological responses characteristic of immune responses, inflammatory response, or inflammation.

Autoimmune responses, disorders and diseases are generally characterized as an undesirable or aberrant response, activity or function of the immune system characterized by increased or undesirable humoral or cell-mediated immune responsiveness or memory, or decreased or insufficient tolerance to self-antigens. Autoimmune responses, disorders and diseases that may be treated in accordance with examples herein include but are not limited to responses, disorders and diseases that cause cell or tissue/organ damage in the subject. The terms "immune disorder" and "immune disease" mean an immune function or activity, which is characterized by different physiological symptoms or abnormalities, depending upon the disorder or disease.

In particular embodiments, a method or use according to examples herein decreases, reduces, inhibits, suppresses, limits or controls an undesirable or aberrant immune response, immune disorder, inflammatory response, or inflammation in a subject. In additional particular examples, a method or use decreases, reduces, inhibits, suppresses, limits or controls an autoimmune response, disorder or disease in a subject. In further particular examples, a method or use decreases, reduces, inhibits, suppresses, limits or controls an adverse symptom of the undesirable or aberrant immune response, immune disorder, inflammatory response, or inflammation, or an adverse symptom of the autoimmune response, disorder or disease.

In additional particular examples, methods and uses according to embodiments herein can result in a reduction in occurrence, frequency, severity, progression, or duration of a symptom of the condition (e.g., undesirable or aberrant immune response, immune disorder, inflammatory response, or inflammation). For example, methods disclosed herein can protect against or decrease, reduce, inhibit, suppress, limit or control progression, severity, frequency, duration or probability of an adverse symptom of the undesirable or aberrant immune response, immune disorder, inflammatory response, or inflammation, or an autoimmune response, disorder or disease.

Examples of adverse symptoms of an undesirable or aberrant immune response, immune disorder, inflammatory response, or inflammation, or an adverse symptom of the autoimmune response, disorder or disease include swelling, pain, rash, discoloration, headache, fever, nausea, diarhea, bloat, lethargy, skeletal joint stiffness, reduced muscle or limb mobility or of the subject, paralysis, a sensory impairment, such as vision or tissue or cell damage. Examples of adverse symptoms occur in particular tissues, or organs, or regions or areas of the body, such as in skin, epidermal or mucosal tissue, gut, gastrointestinal, bowel, genito-urinary tract, pancreas, thymus, lung, liver, kidney, muscle, central or peripheral nerves, spleen, skin, a skeletal joint (e.g., knee, ankle, hip, shoulder, wrist, finger, toe, or elbow), blood or lymphatic vessel, or a cardio-pulmonary tissue or organ. Additional examples of adverse symptoms of an autoimmune response, disorder or disease include T cell production, survival, proliferation, activation or differentiation, and/or production of auto-antibodies, or pro-inflammatory cytokines or chemokines (e.g., TNF-alpha, IL-6, etc.).

Exemplary inhibitors inhibit binding between PKCη and CTLA-4. Accordingly, inhibitors include any molecule that binds to a PKCη and CTLA-4 amino acid sequence, and inhibits binding or interaction between PKCη and CTLA-4, e.g., binding or interaction between native or endogenous PKCη and CTLA-4. Accordingly, exemplary inhibitors of binding between PKCη and CTLA-4 include all PKCη and CTLA-4sequences, subsequences, variants and derivatives, and polymorphisms set forth herein.

More specifically, for example, inhibitors include PKCη amino acid subsequences that include regions that bind to or interact with CTLA-4. By way of example, a PKCη amino acid sequence comprises, consists or consists essentially of from about residue 28 to residue 317 of PKCη or a subsequence, portion, homologue, variant or derivative thereof. Additional examples of a PKCη amino acid sequence comprises, consists or consists essentially of from about residue 28 to residue 32 or from about residue 32 to residue 317 of PKCη or a subsequence, portion, homologue, variant or derivative thereof. Additional examples of a PKCη amino acid sequence comprises, consists or consists essentially of a serine at one or more of residues 28, 32 and/or 317 of of PKCη or a subsequence, portion, homologue, variant or derivative thereof.

By way of additional examples, a CTLA-4 amino acid sequence comprises, consists or consists essentially of from about residue 182 to residue 223 of CTLA-4 or a subsequence, portion, homologue, variant or derivative thereof. Additional examples of a CTLA-4 amino acid sequence comprises, consists or consists essentially of from about residue 188 to residue 193 or from about residue 191 to residue 193 of CTLA-4 or a subsequence, portion, homologue, variant or derivative thereof. In more particular examples, a CTLA-4 amino acid sequence comprises, consists or consists essentially of a lysine at one or more of residues 188, 191, 192 and/or 193 of CTLA-4.

Such PKCη and CTLA-4 sequences, as set forth herein, can be included within a larger sequence. For example, a PKCη subsequence with a length from 5 to about 682 amino acids, where the 5 to about 682 amino acid sequence includes all or portion of a PKCη amino acid sequence, or does not include all or a portion of a PKCη amino acid sequence. In another example, a CTLA-4 subsequence with a length from 5 to about 222 amino acids, where the 5 to about 222 amino acid sequence includes all or portion of a CTLA-4 amino acid sequence, or does not include all or a portion of a CTLA-4 amino acid sequence.

In addition to the foregoing inhibitors of binding between PKCη and CTLA-4, additional inhibitors include small molecules. Example, of small molecule inhibitors include organic molecules that bind to PKCη or CTLA-4, such as in a respective sequence region that includes or consists of a binding region of PKCη or CTLA-4 that binds to CTLA-4 and PKCη, respectively. Particular non-limiting examples include Rottlerin ((E)-1-[6-[(3-acetyl-2,4,6-trihydroxy-5-methylphenyl)methyl]-5,7-dihydroxy-2,2-dimethylchromen-8-yl]-3-phenylprop-2-en-1-one); Midostaurin ((9S,10R,11R,13R)-2,3,10,11,12,13-Hexahydro-10-methoxy-9-methyl-11-(methylamino)-9,13-epoxy-1H,9H-diindolo[1,2,3-gh:3',2',1'-lm]pyrrolo[3,4-j][1,7]benzodiamzonine-1-one) and a peptide pseudosubstrate sequence set forth as: Thr-Arg-Lys-Arg-Gln-Arg-Ala-Met-Arg-Arg-Arg-Val-His-Gln-Ile-Asn-Gly (SEQ ID NO: 3).

The term "contacting" means direct or indirect interaction between two or more entities (e.g., between PKCη or CTLA-4 and an inhibitor). A particular example of direct interaction is binding. A particular example of an indirect interaction is where one entity acts upon an intermediary molecule, which in turn acts upon the second referenced entity. Contacting as used herein includes in solution, in solid phase, *in vitro, ex vivo,* in a cell and *in vivo.* Contacting *in vivo* can be referred to as administering, or administration, or delivery.

In methods and uses described herein, an inhibitor, such as a PKCη or CTLA-4 sequence, can be administered prior to, substantially contemporaneously with or following an undesirable or aberrant immune response, immune disorder, inflammatory response, or inflammation, or an autoimmune response, disorder or disease, GVHD, or host rejection of a cell, tissue or organ transplant (such as heart, liver, lung, bone marrow, etc.), or one or more adverse symptoms, disorders, illnesses, pathologies, diseases, or complications caused by or associated with the foregoing. Thus, methods and uses may be practiced prior to (i.e. prophylaxis), concurrently with or after evidence of the response, disorder or disease begins, or one or more adverse symptoms, disorders, illnesses, pathologies, diseases, or complications caused by or associated with the undesirable or aberrant immune response, immune disorder, inflammatory response, inflammation or an autoimmune response, disorder or disease, GVHD or host rejection of a cell, tissue or organ transplant (such as heart, liver, lung, bone marrow, etc.). Administering a PKCη or CTLA-4 sequence prior to, concurrently with or immediately following development of an adverse symptom may decrease, reduce, inhibit, suppress, limit or control the occurrence, frequency, severity, progression, or duration of one or more adverse symptoms, disorders, illnesses, pathologies, diseases, or complications caused by or associated with the undesirable or aberrant immune response, immune disorder, inflammatory response, inflammation or autoimmune response, disorder or disease, or GVHD, or host rejection of a cell, tissue or organ transplant (such as heart, liver, lung, bone marrow, etc.).

The disclosure also describes combination compositions, methods and uses, such as a PKCη or CTLA-4 sequence and a second agent or drug. PKCη or CTLA-4 sequence or a composition thereof can be formulated and/or administered in combination with a second agent, drug or treatment, such as an anti-cell proliferative, anti-cancer, anti-tumor, anti-metastatic, an immunosuppressive, anti-inflammatory, or palliative agent, drug or treatment. Accordingly, PKCη or CTLA-4, or a composition thereof can be formulated as a combination and/or administered prior to, substantially contemporaneously with or following administering a second agent, drug or treatment,such as an anti-cell proliferative, anti-cancer, anti-tumor, anti-metastatic, immunosuppressive, anti-inflammatory, or palliative agent, drug or treatment.

In one example, a composition, method or use includes a PKCη or CTLA-4 sequence and an anti-cell proliferative, anti-cancer, anti-tumor, anti-metastatic, or anti-inflammatory agent or drug. Such agents and drugs useful in combinations, methods and uses include drugs and agents for treatment of cell proliferative, cancer, tumor, metastasis, an undesirable or aberrant immune response, disorder or disease, an inflammatory response, disorder or disease, inflammation, an autoimmune response, disorder or disease, GVHD, or host rejection of a cell, tissue or organ transplant.

Anti-cell proliferative, anti-tumor, anti-cancer, anti-neoplastic treatments, protocols and therapies include any other composition, treatment, protocol or therapeutic regimen that inhibits, decreases, retards, slows, reduces or prevents aberrant or undesirable cell proliferation, a hyperproliferative disorder, such as tumor, cancer or neoplastic growth, progression, metastasis, proliferation or survival, in vitro or *in vivo.* Particular non-limiting examples of an anti-proliferative (e.g., tumor) therapy include chemotherapy, immunotherapy, radiotherapy (ionizing or chemical), local thermal (hyperthermia) therapy and surgical resection. Any composition, treatment, protocol, therapy or regimen having an anti-cell proliferative activity or effect can be used in combination with a composition or method disclosed herein.

Anti-proliferative or anti-tumor compositions, therapies, protocols or treatments can operate by biological mechanisms that prevent, disrupt, interrupt, inhibit or delay cell cycle progression or cell proliferation; stimulate or enhance apoptosis or cell death, inhibit nucleic acid or protein synthesis or metabolism, inhibit cell division, or decrease, reduce or inhibit cell survival, or production or utilization of a necessary cell survival factor, growth factor or signaling pathway (extracellular or intracellular). Non-limiting examples of chemical agent classes having anti-cell proliferative and anti-tumor activities include alkylating agents, anti-metabolites, plant extracts, plant alkaloids, nitrosoureas, hormones, nucleoside and nucleotide analogues. Specific examples having anti-cell proliferative and anti-tumor activities include cyclophosphamide, azathioprine, cyclosporin A, prednisolone, melphalan, chlorambucil, mechlorethamine, busulphan, methotrexate, 6-mercaptopurine, thioguanine, 5-fluorouracil, cytosine arabinoside, AZT, 5-azacytidine (5-AZC) and 5-azacytidine related compounds such as decitabine (5-aza-2'deoxycytidine), cytarabine, 1-beta-D-arabinofuranosyl-5-azacytosine and dihydro-5-azacytidine, bleomycin, actinomycin D, mithramycin, mitomycin C, carmustine, lomustine, semustine, streptozotocin, hydroxyurea, cisplatin, mitotane, procarbazine, dacarbazine, taxanes such as taxol, vinblastine, vincristine, doxorubicin and dibromomannitol.

Additional agents that are applicable with the compositions and methods are known in the art and can be employed. For example, monoclonal antibodies that bind tumor cells or oncogene products, such as Rituxan® and Herceptin (Trastuzumab)(anti-Her-2 neu antibody), Bevacizumab (Avastin), Zevalin, Bexxar, Oncolym, 17-1A(Edrecolomab), 3F8 (anti-neuroblastoma antibody), MDX-CTLA4 (Ipilimumab, Medarex, NJ), Campath®, Mylotarg, IMC-C225 (Cetuximab), aurinstatin conjugates of cBR96 and cAC10 (Doronina et al. (2003). Nat Biotechnol 21:778) can be used in combination with an agent that binds to a cis complex in accordance with examples herein.

Additional non-limiting examples of second agents and drugs include anti-inflammatory agents, such as steroidal and non-steroidal anti-inflammatory drugs (NSAIDs) to limit or control inflammatory symptoms. Second agents and drugs also include immunosuppressive corticosteroids (steroid receptor agonists) such as budesonide, prednisone, flunisolide; anti-inflammatory agents such as flunisolide hydrofluoroalkane, estrogen, progesterone, dexamethasone and loteprednol; beta-agonists (e.g., short or long-acting) such as bambuterol, formoterol, salmeterol, albuterol; anticholinergics such as ipratropium bromide, oxitropium bromide, cromolyn and calcium-channel blocking agents; antihistamines such as terfenadine, astemizole, hydroxyzine, chlorpheniramine, tripelennamine, cetirizine, desloratadine, mizolastine, fexofenadine, olopatadine hydrochloride, norastemizole, levocetirizine, levocabastine, azelastine, ebastine and loratadine; antileukotrienes (e.g., anti-cysteinyl leukotrienes (CysLTs)) such as oxatomide, montelukast, zafirlukast and zileuton; phosphodiesterase inhibitors (e.g., PDE4 subtype) such as ibudilast, cilomilast, BAY 19-8004, theophylline (e.g., sustained-release) and other xanthine derivatives (e.g., doxofylline); thromboxane antagonists such as seratrodast, ozagrel hydrochloride and ramatroban; prostaglandin antagonists such as COX-1 and COX-2 inhibitors (e.g., celecoxib and rofecoxib), aspirin; and potassium channel openers. Additional non-limiting examples of classes of other agents and drugs include anti-inflammatory agents that are immunomodulatory therapies, such as pro-inflammatory cytokine antagonists, such as TNFα antagonists (e.g. etanercept, aka Enbrel™) and the anti-IL-6 receptor tocilizumab; immune cell antagonists, such as the B cell depleting agent rituximab and the T cell costimulation blocker abatacept, which have been used to treat rheumatoid arthritis, and antibodies that bind to cytokines, such as anti-IgE (e.g., rhuMAb-E25 omalizumab), and anti-TNF, IFN, IL-1, IL-2, IL-5, IL-6, IL-9, IL-13, IL-16, and growth factors such as granulocyte/macrophage colony-stimulating factor.

As disclosed herein, compositions, methods and uses, such as treatment methods and uses, can provide a detectable or measurable therapeutic benefit or improvement to a subject. A therapeutic benefit or improvement is any measurable or detectable, objective or subjective, transient, temporary, or longer-term benefit to the subject or improvement in the response, disorder or disease, or one or more adverse symptoms, disorders, illnesses, pathologies, diseases, or complications caused by or associated with the undesirable or aberrant response, disorder or disease, etc. Therapeutic benefits and improvements include, but are not limited to, decreasing, reducing, inhibiting, suppressing, limiting or controlling the occurrence, frequency, severity, progression, or duration of an adverse symptom of undesirable or aberrant response, disorder or disease, etc. Therapeutic benefits and improvements also include, but are not limited to, decreasing, reducing, inhibiting, suppressing, limiting or controlling amounts or activity of T cells, auto-antibodies, pro-inflammatory cytokines or chemokines. Compositions, methods and uses described herein therefore include providing a therapeutic benefit or improvement to a subject.

Compositions, methods and uses described herein by way of example only, can be administered in a sufficient or effective amount to a subject in need thereof. An "effective amount" or "sufficient amount" refers to an amount that provides, in single or multiple doses, alone or in combination, with one or more other compositions (therapeutic agents such as a drug), treatments, protocols, or therapeutic regimens agents, a detectable response of any duration of time (long or short term), an expected or desired outcome in or a benefit to a subject of any measurable or detectable degree or for any duration of time (e.g., for minutes, hours, days, months, years, or cured).

The doses of an "effective amount" or "sufficient amount" for treatment (e.g., to ameliorate or to provide a therapeutic benefit or improvement) typically are effective to provide a response, disorder or disease, of one, multiple or all adverse symptoms, consequences or complications of the response, disorder or disease, one or more adverse symptoms, disorders, illnesses, pathologies, diseases, or complications, for example, caused by or associated with an undesirable or an undesirable or aberrant immune response, disorder or disease, an inflammatory response, disorder or disease, inflammation, an autoimmune response, disorder or disease, GVHD, or host rejection of a cell, tissue or organ transplant, to a measurable extent, although decreasing, reducing, inhibiting, suppressing, limiting or controlling progression or worsening of the response, disorder or disease, or GVHD, or host rejection of a cell, tissue or organ transplant, or an adverse symptom thereof, is a satisfactory outcome.

An effective amount or a sufficient amount can, but need not, be provided in a single administration, but may require multiple administrations, and, can but need not be, administered alone or in combination with another composition (e.g., agent), treatment, protocol or therapeutic regimen. For example, the amount may be proportionally increased as indicated by the need of the subject, type, status and severity of the response, disorder, or disease treated or side effects (if any) of treatment. In addition, an effective amount or a sufficient amount need not be effective or sufficient if given in single or multiple doses without a second composition (e.g., another drug or agent), treatment, protocol or therapeutic regimen, since additional doses, amounts or duration above and beyond such doses, or additional compositions (e.g., drugs or agents), treatments, protocols or therapeutic regimens may be included in order to be considered effective or sufficient in a given subject. Amounts considered effective also include amounts that result in a reduction of the use of another treatment, therapeutic regimen or protocol.

An effective amount or a sufficient amount need not be effective in each and every subject treated, prophylactically or therapeutically, nor a majority of treated subjects in a given group or population. An effective amount or a sufficient amount means effectiveness or sufficiency in a particular subject, not a group or the general population. As is typical for such methods, some subjects will exhibit a greater response, or less or no response to a given treatment method or use.

Thus, appropriate amounts will depend upon the condition treated, the therapeutic effect desired, as well as the individual subject (e.g., the bioavailability within the subject, gender, age, etc.).

The term "ameliorate" means a detectable or measurable improvement in a subject's condition or an underlying cellular response. A detectable or measurable improvement includes a subjective or objective decrease, reduction, inhibition, suppression, limit or control in the occurrence, frequency, severity, progression, or duration of the response, disorder or disease, such as undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant, or one or more adverse symptoms, disorders, illnesses, pathologies, diseases, or complications caused by or associated with the response, disorder or disease, such as an undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant, or a reversal of the response, disorder or disease, such as undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant. Such improvements can also occur at the cellular level.

Thus, a successful treatment outcome can lead to a "therapeutic effect," or "benefit" of decreasing, reducing, inhibiting, suppressing, limiting, controlling or preventing the occurrence, frequency, severity, progression, or duration of undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant, or one or more adverse symptoms or underlying causes or consequences of the undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant in a subject. Treatment methods affecting one or more underlying causes of the response, disorder or disease or adverse symptom are therefore considered to be beneficial. A decrease or reduction in worsening, such as stabilizing an undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant, or an adverse symptom thereof, is also a successful treatment outcome.

A therapeutic benefit or improvement therefore need not be complete ablation of the undesirable or aberrant cell proliferation or hyperproliferation, a neoplasia, tumor or cancer, or the undesirable or aberrant immune response, disorder or disease, an inflammatory response, disorder or disease, inflammation, an autoimmune response, disorder or disease, GVHD, or host rejection of a cell, tissue or organ transplant, or any one, most or all adverse symptoms, complications, consequences or underlying causes associated with the undesirable or aberrant cell proliferation or hyperproliferation, a neoplasia, tumor or cancer, or the undesirable or aberrant immune response, disorder or disease, an inflammatory response, disorder or disease, inflammation, an autoimmune response, disorder or disease, GVHD, or host rejection of a cell, tissue or organ transplant. Thus, a satisfactory endpoint is achieved when there is an incremental improvement in a subject's response, disorder or disease, or a partial decrease, reduction, inhibition, suppression, limit, control or prevention in the occurrence, frequency, severity, progression, or duration, or inhibition or reversal, of the response, disorder or disease (e.g., stabilizing one or more symptoms or complications), such as the undesirable or aberrant cell proliferation or hyperproliferation, a neoplasia, tumor or cancer, or the undesirable or aberrant immune response, disorder or disease, an inflammatory response, disorder or disease, inflammation, an autoimmune response, disorder or disease, GVHD, or host rejection of a cell, tissue or organ transplant, or one or more adverse symptoms, disorders, illnesses, pathologies, diseases, or complications caused by or associated with the undesirable or aberrant cell proliferation or hyperproliferation, a neoplasia, tumor or cancer, or the undesirable or aberrant immune response, disorder or disease, an inflammatory response, disorder or disease, inflammation, an autoimmune response, disorder or disease, GVHD, or host rejection of a cell, tissue or organ transplant, over a short or long duration of time (hours, days, weeks, months, etc.).

Effectiveness of a method or use, such as a treatment that provides a potential therapeutic benefit or improvement of a response, disorder or disease, such as undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant, can be ascertained by various methods. Such methods include, for example, measuring cell numbers, proliferation and types, tumor or cancer size, cell, cell apoptosis/necrosis, scores of swelling, pain, rash, headache, fever, nausea, diarrhea, bloat, lethargy, skeletal joint stiffness, lack of mobility, rash, or tissue or cell damage. Measuring T cell activation and/or differentiation, cell infiltration of a region, cell accumulation or migration to a region, production of antibodies, cytokines, lymphokines, chemokines, interferons and interleukins, cell growth and maturation factors using various immunological assays, such as ELISA. Determining cell numbers, proliferation and types, tumor or cancer size, metastasis, cell apoptosis/necrosis, or the degree of cell, tissue or organ damage can be ascertained by imaging techniques such as CT scanning, MRI, ultrasound, molecular contrast imaging, or molecular ultrasound contrast imaging. For gastrointestinal tract, inflammation can be assessed by endoscopy (colonoscopy, gastroscopy, ERCP), for example. For inflammation of the central nervous system (CNS), cells and cytokines in spinal tap reflect inflammation, for example. CNS inflammation (Multiple sclerosis, Parkinson's, Alzheimer's) may be reflected in the corresponding clinical function scores known in the art, for example. Peripheral nerve inflammation can include functional assessment (motor and sensor), for example.

The term "subject" refers to animals, typically mammalian animals, such as humans, non human primates (e.g., apes, gibbons, chimpanzees, orangutans, macaques), domestic animals (e.g., dogs and cats), farm animals (e.g., horses, cows, goats, sheep, pigs) and experimental animals (e.g., mouse, rat, rabbit, guinea pig). Subjects include animal disease models, for example, animal models of hyperproliferation, cancers, tumors and metastases for *in vivo* analysis of a composition herein. Other animal models described herein include models of infectious pathogens (viral, bacterial, etc.) an undesirable or aberrant immune response, disorder or disease, an inflammatory response, disorder or disease, inflammation, an autoimmune response, disorder or disease (e.g., CIA, BXSB, EAE and SCID mice), GVHD, or host rejection of a cell, tissue or organ transplant GVHD and host rejection of a cell, tissue or organ transplant, for *in vivo* analysis of a compositions herein.

Subjects appropriate for treatment include those having undesirable or aberrant cell proliferation or hyperproliferation, or infected with a pathogen (viral, bacterial, etc.). Subjects appropriate for treatment also include those having, or at risk of having a neoplasia, tumor or cancer, as well as those who are undergoing or have undergone anti-tumor therapy, including subjects where the neoplasia, tumor or cancer is in remission. Embodiments are therefore applicable to treating a subject who is at risk of a neoplasia, tumor or cancer or a complication associated with a neoplasia, tumor or cancer, for example, due to neoplasia, tumor or cancer reappearance or regrowth following a period of remission.

Other subjects appropriate for treatment, described herein by way of example, include those having an undesirable or aberrant immune response, disorder or disease, an inflammatory response, disorder or disease, inflammation, an autoimmune response, disorder or disease, GVHD, or host rejection of a cell, tissue or organ transplant, those undergoing treatment for an undesirable or aberrant immune response, disorder or disease, an inflammatory response, disorder or disease, inflammation, an autoimmune response, disorder or disease, GVHD, or host rejection of a cell, tissue or organ transplant, as well as those who have undergone treatment or therapy for an undesirable or aberrant immune response, disorder or disease, an inflammatory response, disorder or disease, inflammation, an autoimmune response, disorder or disease, GVHD, or host rejection of a cell, tissue or organ transplant, including subjects where theundesirable or aberrant immune response, disorder or disease, inflammatory response, disorder or disease, inflammation, an autoimmune response, disorder or disease, GVHD, or host rejection of a cell, tissue or organ transplant, is in remission.

Also described herein are subjects including those that are at increased risk of an undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant. A candidate subject, for example, has undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant, or is being treated with a therapy or drug for an undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant. Candidate subjects also include subjects that would benefit from or are in need of treatment for undesirable or aberrant cell proliferation or hyperproliferation, a neoplasia, tumor or cancer, or an undesirable or aberrant immune response, disorder or disease, an inflammatory response, disorder or disease, inflammation, an autoimmune response, disorder or disease, GVHD, or host rejection of a cell, tissue or organ transplant.

"At risk" subjects typically have risk factors associated with development of undesirable or aberrant cell proliferation or hyperproliferation, a neoplasia, tumor or cancer, or undesirable or aberrant immune response, immune disorder or immune disease, inflammation or an inflammatory response. Risk factors include gender, lifestyle (diet, smoking), occupation (medical and clinical personnel, agricultural and livestock workers), environmental factors (carcinogen exposure), family history (autoimmune disorders, diabetes, etc.), genetic predisposition, etc. For example, subjects at risk for developing melanoma include excess sun exposure (ultraviolet radiation), fair skin, high numbers of naevi (dysplastic nevus), patient phenotype, family history, or a history of a previous melanoma. Subjects at risk for developing neoplasia, tumor or cancer can therefore be identified by lifestyle, occupation, environmental factors, family history, and genetic screens for tumor associated genes, gene deletions or gene mutations. Subjects at risk for developing breast cancer lack *Brca1*, for example. Subjects at risk for developing colon cancer have early age or high frequency polyp formation, or deleted or mutated tumor suppressor genes, such as adenomatous polyposis coli (*APC*), for example. Susceptibility to autoimmune disease is frequently associated with MHC genotype. For example, in diabetes there is an association with HLA-DR3 and HLA-DR4. At risk subjects also include those with risk factors include family history (e.g., genetic predisposition), gender, lifestyle (diet, smoking), occupation (medical and clinical personnel, agricultural and livestock workers), environmental factors (allergen exposure), etc.

"At risk" subjects therefore include those at increased or enhanced risk of aberrant or undesirable cell proliferation, hyperplasia (e.g., a neoplasia, tumor or cancer), undesirable or aberrant immune response, immune disorder or immune disease, inflammation or an inflammatory response, disorder or disease, inflammation, an autoimmune response, disorder or disease, GVHD, or host rejection of a cell, tissue or organ transplant. Particular subjects at increased or enhanced risk include those that have had an undesirable or aberrant immune response, disorder or disease, an inflammatory response, disorder or disease, inflammation, an autoimmune response, disorder or disease, GVHD, or host rejection of a cell, tissue or organ transplant. Particular subjects at risk also include those prescribed a treatment or therapy for treatment of aberrant or undesirable cell proliferation, hyperplasia (e.g., a neoplasia, tumor or cancer), undesirable or aberrant immune response, immune disorder or immune disease, inflammation or an inflammatory response, disorder or disease, inflammation, an autoimmune response, disorder or disease, GVHD, or host rejection of a cell, tissue or organ transplant.

Described by way of example only, all PKCη and CTLA-4 sequences, subsequences, variants and derivatives, polymorphisms and compositions thereof may be contacted or provided *in vitro, ex vivo* or administered or delivered *in vivo* in various doses and amounts, and frequencies, to a subject. For example, a PKCη or CTLA-4 sequence or a composition thereof can be administered or delivered to provide the intended effect, as a single or as multiple dosages, for example, in an effective or sufficient amount. Exemplary doses range from about 25-250, 250-500, 500-1000, 1000-2500, 2500-5000, 5000-25,000, or 5000-50,000 pg/kg; from about 50-500, 500-5000, 5000-25,000 or 25,000-50,000 ng/kg; from about 50-500, 500-5000, 5000-25,000 or 25,000-50,000 g/kg; and from about 25-250, 250-500, 500-1000, 1000-2500, 2500-5000, 5000-25,000, or 5000-50,000 mg/kg, on consecutive days, alternating days or intermittently.

Single or multiple (e.g., 2, 3, 4, 5, 6, 7, 8, 9, 10, or more times) administrations or doses can be delivered on the same or consecutive days, alternating days or intermittently. For example, a PKCη or CTLA-4 sequence, subsequence, variant, derivative, or polymorphisms set forth herein or a composition thereof can be administered one, two, three, four or more times daily, on alternating days, bi-weekly, weekly, monthly, bi-monthly, or annually. PKCη or CTLA-4 sequences and compositions thereof can be administered for any appropriate duration, for example, for period of 1 hour, or less, e.g., 30 minutes or less, 15 minutes or less, 5 minutes or less, or 1 minute or less.

Described by way of example only, an inhibitor of binding , such as a PKCη or CTLA-4 sequence, subsequence, variant, derivative, or polymorphisms set forth herein or a composition thereof can be administered to a subject and methods and uses may be practiced prior to, substantially contemporaneously with, or within about 1-60 minutes, hours (e.g., within 1, 2, 3, 4 , 5, 6, 8, 12, 24 hours), or days of a symptom or onset of an undesirable or aberrant immune response, disorder or disease, an inflammatory response, disorder or disease, inflammation, an autoimmune response, disorder or disease, GVHD, or host rejection of a cell, tissue or organ transplant.

Described by way of example only, a PKCη or CTLA-4 sequence, subsequence, variant, derivative, or polymorphisms set forth herein or a composition thereof can be administered and methods and uses may be practiced via systemic, regional or local administration, by any route. For example, a PKCη or CTLA-4 sequence, subsequence, variant, derivative, or polymorphisms set forth herein or a composition thereof may be administered systemically, regionally or locally, via injection, infusion, orally (e.g., ingestion or inhalation), topically, intravenously, intraarterially, intramuscularly, intraperitoneally, intradermally, subcutaneously, intracavity, intracranially, transdermally (topical), parenterally, e.g. transmucosally or intrarectally (enema) catheter, optically. Compositions, method and uses, including pharmaceutical formulations, can be administered via a (micro)encapsulated delivery system or packaged into an implant for administration.

Compositions, methods and uses include pharmaceutical compositions, which refer to "pharmaceutically acceptable" and "physiologically acceptable" carriers, diluents or excipients. As used herein, the term "pharmaceutically acceptable" and "physiologically acceptable," when referring to carriers, diluents or excipients includes solvents (aqueous or non-aqueous), detergents, solutions, emulsions, dispersion media, coatings, isotonic and absorption promoting or delaying agents, compatible with pharmaceutical administration and with the other components of the formulation, and can be contained in a tablet (coated or uncoated), capsule (hard or soft), microbead, emulsion, powder, granule, crystal, suspension, syrup or elixir.

In various examples, a pharmaceutical composition includes an inhibitor of binding between PKCη or CTLA-4. In a particular example, an inhibitor includes or consists of a PKCη or CTLA-4 sequence, subsequence, variant, derivative, or polymorphism set forth herein.

Exemplary PKCη sequences typically have a length from 5 to about 682 amino acid sequence includes all or portion of a PKCη amino acid sequence, or does not include all or a portion of a PKCη amino acid sequence. In further particular aspects, a PKCη sequence has a length of about 5-10, 10-20, 20-30, 30-40, 40-50, 50-75, 75-100, 100-150, 150-200, 200-250, 250-300, 300-350, 350-400, 400-500, 500-600 or 600-682 amino acid residues.

Exemplary CTLA-4 sequences typically have a length from 5 to about 222 amino acid sequence includes all or portion of a CTLA-4 amino acid sequence, or does not include all or a portion of a CTLA-4 amino acid sequence. Additional examples of a CTLA-4 amino acid sequence comprises, consists or consists essentially of from about residue 188 to residue 193 or 192, or from about residue 191 to residue 193 of CTLA-4 or a subsequence, portion, homologue, variant or derivative thereof. In more particular examples, a CTLA-4 amino acid sequence comprises, consists or consists essentially of a lysine at one or more of residues 188, 191, 192 and/or 193 of CTLA-4. In further particular examples, a CTLA-4 sequence has a length of about 5-10, 10-20, 20-30, 30-40, 40-50, 50-75, 75-100, 100-150, 150-200, 200-222, amino acid residues.

Pharmaceutical compositions can be formulated to be compatible with a particular route of administration. Compositions for parenteral, intradermal, or subcutaneous administration can include a sterile diluent, such as water, saline, fixed oils, polyethylene glycols, glycerine, propylene glycol or other synthetic solvents. The preparation may contain one or more preservatives to prevent microorganism growth (e.g., antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose).

Pharmaceutical compositions for injection include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). The carrier can be a solvent or dispersion medium containing, for example, water, ethanol, polyol (e.g., glycerol, propylene glycol, and polyetheylene glycol), and suitable mixtures thereof. Fluidity can be maintained, for example, by the use of a coating such as lecithin, or by the use of surfactants. Antibacterial and antifungal agents include, for example, parabens, chlorobutanol, phenol, ascorbic acid and thimerosal. Including an agent that delays absorption, for example, aluminum monostearate and gelatin, can prolong absorption of injectable compositions.

For transmucosal or transdermal administration, penetrants appropriate to the barrier to be permeated are used in the formulation. Such penetrants are known in the art, and include, for example, for transmucosal administration, detergents, bile salts, and fusidic acid derivatives. Transmucosal administration can be accomplished through the use of nasal sprays, inhalation devices (e.g., aspirators) or suppositories. For transdermal administration, the active compounds are formulated into ointments, salves, gels, creams or patches.

Additional pharmaceutical formulations and delivery systems are known in the art and are applicable in the methods disclosed herein (see, e.g., Remington's Pharmaceutical Sciences (1990) 18th ed., Mack Publishing Co., Easton, PA; The Merck Index (1996) 12th ed., Merck Publishing Group, Whitehouse, NJ; Pharmaceutical Principles of Solid Dosage Forms, Technonic Publishing Co., Inc., Lancaster, Pa., (1993); and Poznansky, et al., Drug Delivery Systems, R. L. Juliano, ed., Oxford, N.Y. (1980), pp. 253-315).

The compositions, methods and uses in accordance with examples herein, including PKCη and CTLA-4 sequences, subsequences, variants and derivatives, polymorphisms, treatments, therapies, combinations, agents, drugs and pharmaceutical formulations can be packaged in dosage unit form for ease of administration and uniformity of dosage. "Dosage unit form" as used herein refers to physically discrete units suited as unitary dosages treatment; each unit contains a quantity of the composition in association with the carrier, excipient, diluent, or vehicle calculated to produce the desired treatment or therapeutic (e.g., beneficial) effect. The unit dosage forms will depend on a variety of factors including, but not necessarily limited to, the particular composition employed, the effect to be achieved, and the pharmacodynamics and pharmacogenomics of the subject to be treated.

In some examples, there are provided kits including PKCη and/or CTLA-4 sequences, subsequences, variants and derivatives, polymorphisms, combination compositions and pharmaceutical formulations thereof, packaged into suitable packaging material. Kits can be used in various *in vitro, ex vivo* and *in vivo* methods and uses, for example a treatment method or use as disclosed herein.

A kit typically includes a label or packaging insert including a description of the components or instructions for use *in vitro, in vivo,* or *ex vivo,* of the components therein. A kit can contain a collection of such components, e.g., a PKCη or CTLA-4 sequence, alone, or in combination with another therapeutically useful composition (e.g., an immune modulatory drug).

The term "packaging material" refers to a physical structure housing the components of the kit. The packaging material can maintain the components sterilely, and can be made of material commonly used for such purposes (*e.g*., paper, corrugated fiber, glass, plastic, foil, ampules, vials, tubes, etc.).

Kits can include labels or inserts. Labels or inserts include "printed matter," e.g., paper or cardboard, or separate or affixed to a component, a kit or packing material (e.g., a box), or attached to an ampule, tube or vial containing a kit component. Labels or inserts can additionally include a computer readable medium, such as a disk (e.g., hard disk), optical disk such as CD- or DVD-ROM/RAM, DVD, MP3, magnetic tape, or an electrical storage media such as RAM and ROM or hybrids of these such as magnetic/optical storage media, FLASH media or memory type cards.

Labels or inserts can include identifying information of one or more components therein, dose amounts, clinical pharmacology of the active ingredient(s) including mechanism of action, pharmacokinetics and pharmacodynamics. Labels or inserts can include information identifying manufacturer information, lot numbers, manufacturer location and date.

Labels or inserts can include information on a condition, disorder, disease or symptom for which a kit component may be used. Labels or inserts can include instructions for the clinician or for a subject for using one or more of the kit components in a method, treatment protocol or therapeutic regimen. Instructions can include dosage amounts, frequency or duration, and instructions for practicing any of the methods and uses, treatment protocols or therapeutic regimes set forth herein. Exemplary instructions include, instructions for treating an undesirable or aberrant immune response, disorder or disease, an inflammatory response, disorder or disease, inflammation, an autoimmune response, disorder or disease, GVHD, or host rejection of a cell, tissue or organ transplant. Kits therefore can additionally include labels or instructions for practicing any of the methods and uses described herein.

Labels or inserts can include information on any benefit that a component may provide, such as a prophylactic or therapeutic benefit. Labels or inserts can include information on potential adverse side effects, such as warnings to the subject or clinician regarding situations where it would not be appropriate to use a particular composition. Adverse side effects could also occur when the subject has, will be or is currently taking one or more other medications that may be incompatible with the composition, or the subject has, will be or is currently undergoing another treatment protocol or therapeutic regimen which would be incompatible with the composition and, therefore, instructions could include information regarding such incompatibilities.

Kits can additionally include other components. Each component of the kit can be enclosed within an individual container and all of the various containers can be within a single package. Kits can be designed for cold storage. Kits can further be designed to contain PKCη or CTLA-4 sequences, subsequences, variants and derivatives, polymorphisms, or combination compositions or pharmaceutical compositions.

By way of example only, the disclosure also describes cell-free (*e.g*., in solution, in solid phase) and cell-based (*e.g., in vitro* or *in vivo*) methods of screening for, detecting and identifying agents that modulate binding (interaction) between PKCη and CTLA-4, and methods of screening, detecting and identifying agents that modulate an undesirable or aberrant immune response, disorder or disease, an inflammatory response, disorder or disease, inflammation, an autoimmune response, disorder or disease, GVHD, or host rejection of a cell, tissue or organ transplant. The methods can be performed in solution, in solid phase, in silica, *in vitro,* in a cell, and *in vivo.*

In various examples, a method of screening for an agent includes contacting PKCη and/or CTLA-4 sequence, subsequence, variant, derivative, or polymorphism under conditions allowing binding between PKCη and CTLA-4 sequence, subsequence, variant, derivative, or polymorphism in the presence a test agent; and determining if the test agent inhibits or reduces binding between PKCη and CTLA-4 sequence, subsequence, variant, derivative, or polymorphism. In another example, a method of identifying an agent includes contacting PKCη and/or CTLA-4 sequence, subsequence, variant, derivative, or polymorphism under conditions allowing binding between PKCη and CTLA-4 sequence, subsequence, variant, derivative, or polymorphism in the presence a test agent; and determining if the test agent inhibits or reduces binding between PKCη and CTLA-4 sequence, subsequence, variant, derivative, or polymorphism. A reduction or inhibition of binding screens for or identifies the test agent as an agent that decreases, reduces or inhibits interaction of PKCη and CTLA-4.

In a further example, a method of identifying a candidate agent for modulating (e.g., decreasing, reducing, inhibiting, suppressing, limiting or controlling) an undesirable or aberrant immune response, disorder or disease, an inflammatory response, disorder or disease or inflammation, includes contacting a PKCη and/or CTLA-4 sequence, subsequence, variant, derivative, or polymorphism under conditions allowing binding between PKCη and CTLA-4 sequence, subsequence, variant, derivative, or polymorphism in the presence a test agent; and determining if the test agent inhibits or reduces binding between PKCη and CTLA-4 sequence, subsequence, variant, derivative, or polymorphism. If a test agent reduces or inhibits binding, the test agent is a candidate agent for decreasing, reducing, inhibiting, suppressing, limiting or controlling an undesirable or aberrant immune response, disorder or disease, an inflammatory response, disorder or disease or inflammation.

In an additional example, a method of identifying a candidate agent for decreasing, reducing, inhibiting, suppressing, limiting or controlling an autoimmune response, disorder or disease, includes contacting a PKCη and/or CTLA-4 sequence, subsequence, variant, derivative, or polymorphism under conditions allowing binding between PKCη and CTLA-4 sequence, subsequence, variant, derivative, or polymorphism in the presence a test agent; and determining if the test agent inhibits or reduces binding between PKCη and CTLA-4. If the test agent reduces or inhibits binding, the test agent is a candidate agent for decreasing, reducing, inhibiting, suppressing, limiting or controlling an autoimmune response, disorder or disease.

In yet another example, a method of identifying a candidate agent for decreasing, reducing, inhibiting, suppressing, limiting or controlling graft vs. host disease (GVHD), or host rejection of a cell, tissue or organ transplant includes contacting PKCη and/or CTLA-4 sequence, subsequence, variant, derivative, or polymorphism under conditions allowing binding between a PKCη and CTLA-4 sequence, subsequence, variant, derivative, or polymorphism in the presence a test agent; and determining if the test agent inhibits or reduces binding between PKCη and CTLA-4 sequence, subsequence, variant, derivative, or polymorphism. If the test agent reduces or inhibits binding, the test agent is a candidate agent for decreasing, reducing, inhibiting, suppressing, limiting or controlling graft vs. host disease (GVHD), or host rejection of a cell, tissue or organ transplant.

The terms "determining," "assaying" and "measuring" and grammatical variations thereof are used interchangeably herein and refer to either qualitative or quantitative determinations, or both qualitative and quantitative determinations. When the terms are used in reference to measurement or detection, any means of assessing the relative amount, including the various methods set forth herein and known in the art.

Unless otherwise defined, all technical and scientific terms used herein have the same meaning as commonly understood by one of ordinary skill in the art. Although methods and materials similar or equivalent to those described herein can be used in the practice or testing of the present embodiments and examples, suitable methods and materials are described herein.

With respect to all applications, publications, patents and other references, GenBank citations and ATCC citations cited herein, in case of conflict, the specification, including definitions, will control.

As used herein, the singular forms "a", "and," and "the" include plural referents unless the context clearly indicates otherwise. Thus, for example, reference to "a PKCη sequence" or "a CTLA-4 sequence" includes a plurality of such PKCη or CTLA-4 sequences, subsequences, variants and derivatives, polymorphisms, or combination compositions or pharmaceutical compositions, and reference to "a PKCη or CTLA-4 activity or function" can include reference to one or more PKCη or CTLA-4 activities or functions, and so forth.

As used herein, numerical values are often presented in a range format throughout this document. The use of a range format is merely for convenience and brevity and should not be construed as an inflexible limitation on the scope of the embodiments and examples herein. Accordingly, the use of a range expressly includes all possible subranges, all individual numerical values within that range. Furthermore, all numerical values or numerical ranges include integers within such ranges and fractions of the values or the integers within ranges unless the context clearly indicates otherwise. This construction applies regardless of the breadth of the range and in all contexts throughout this patent document. Thus, for example, reference to a range of 90-100% includes 91-99%, 92-98%, 93-95%, 91-98%, 91-97%, 91-96%, 91-95%, 91-94%, 91-93%, and so forth. Reference to a range of 90-100%, includes 91%, 92%, 93%, 94%, 95%, 95%, 97%, etc., as well as 91.1%, 91.2%, 91.3%, 91.4%, 91.5%, etc., 92.1%, 92.2%, 92.3%, 92.4%, 92.5%, etc., and so forth.

In addition, reference to a range of 1-5,000 fold includes 1, 2, 3, 4, 5, 6, 7, 8, 9, 10, 11, 12, 13, 14, 15, 16, 17, 18, 19, 20, fold, etc., as well as 1.1, 1.2, 1.3, 1.4, 1.5, fold, etc., 2.1, 2.2, 2.3, 2.4, 2.5, fold, etc., and any numerical range within such a ranges, such as 1-2, 5-10, 10-50, 50-100, 100-500, 100-1000, 500-1000, 1000-2000, 1000-5000, etc.

As also used herein a series of range formats are used throughout this document. The use of a series of ranges includes combinations of the upper and lower ranges to provide a range. This construction applies regardless of the breadth of the range and in all contexts throughout this patent document. Thus, for example, reference to a series of ranges such as 5 to 10, 10 to 20, 20 to 30, 30, to 50, 50 to 100, 100 to 150, 150 to 200, 200 to 300, or 300 to 400, 400-500, 500-600, or 600-705, includes ranges such as 5-20, 5-30, 5-40, 5-50, 5-75, 5-100, 5-150, 5-171, and 10-30, 10-40, 10-50, 10-75, 10-100, 10-150, 10-171, and 20-40, 20-50, 20-75, 20-100, 20-150, 20-200, 50 to 200, 50 to 300, 50, to 400, 50 to 500, 100 to 300, 100 to 400, 100 to 500, 100 to 600, 200-400, 200-500, 200 to 600, 200 to 700, and so forth.

Embodiments and examples herein are generally disclosed herein using affirmative language to describe the numerous embodiments and examples. Embodiments and examples herein also specifically include those in which particular subject matter is excluded, in full or in part, such as substances or materials, method steps and conditions, protocols, procedures, assays or analysis. Thus, even though embodiments and examples herein are generally not expressed herein in terms of what they do not include aspects that are not expressly included in various embodiments are nevertheless disclosed herein.

The following examples are intended to illustrate but not limit the scope of the embodiments described in the claims.

Embodiments and examples herein are further exemplified by way of the following non-limited examples.

### EXAMPLES

### Example 1: Materials and Methods

### Antibodies (Abs) and Reagents

mAbs specific for mouse CD3 (clone 145-2C11), CD28 (clone 37.51) or CTLA-4 (clone UC10-4B9) were purchased from Biolegend, as were flourophore-conjugated anti-CD4 (clone GK1.5), anti-CD8 (53-6.7), anti-Foxp3 (clone FJK-16s), anti-CD25 (clone PC61), anti-CD44 (clone IM7) and anti-GITR (clone DTA-1) mAbs. Anti-human CD3 mAb (OKT3) was purified inhouse. Polyclonal anti-PKC-θ (sc-212), anti-PKC-η (C-15 and sc-215), anti-PAK (N-20 and sc-882), anti-NFATc1 (7A6), anti-lamin B (M-20) and anti-α-tubulin (TU-02) Abs were obtained from Santa Cruz Biotechnology. Anti-p65 (NF-κB), anti-GIT2 and anti-aPIX Abs were obtained from Cell Signaling Technology. Anti-Foxp3 Abs (clone 150D/E4 for immunoblotting and clone FJK-16s for flow cytometry) were purchased from eBiosciences. Alexa Fluor 647-conjugated anti-mouse Ig and Alexa Fluor 555-conjugated anti-rabbit Ig were obtained from Molecular Probes. Digitonin was obtained from EMD Chemicals. Calf intestinal alkaline phosphatase was purchased from New England Biolabs. Recombinant CD86-Fc was previously described.

### Plasmids

Plasmids of full-length human Prkch and mouse CTLA-4 were generated via PCR amplification and cloned into the pEF4/HisC expression vector and pMIG retroviral vector, respectively. Point mutations on Prkch and CTLA-4 were generated using Quikchange II Site-directed Mutagenesis Kit (Stratagene). CTLA-4 mutants with shortened cytoplasmic tail (amino acid 182-223 and 192-223) were generated via PCR amplification.

### Mice and primary cell cultures

C57BL/6 (B6; CD45.2+), B6.SJL (CD45.1+), Prkcq-/- (CD45.2+) and Prkch-/- (CD45.2+) mice were housed, maintained under specific pathogen-free conditions, and manipulated according to guidelines approved by the LIAI Animal Care Committee and the Animal Care and Use Committee of TSRI. The Prkcq-/- mice are now available from the Jackson Laboratories (B6.Cg- Prkch<tm1.1Gasc>/J). Foxp3-IRES-eGFP (FIG) mice were obtained from Jackson Lab. Prkch-/- x Foxp3-IRES-eGFP (Prkch-/--FIG) mice were generated by crossing Foxp3-IRES-eGFP and Prkch-/- mice. CD4+ T cells were isolated by CD4 positive selection (BD Biosciences), and were cultured in RPMI-1640 medium (Mediatech Inc.) supplemented with 10% heat-inactivated fetal bovine serum, 2 mM glutamine, 1 mM sodium pyruvate, 1 mM MEM nonessential amino acids, and 100 U/ml each of penicillin G and streptomycin (Life Technologies). In vitro differentiation of induced Tregs was performed using naive CD4+CD62L+ T cells in the presence of plate-bound anti-CD3 (1 µg/ml), soluble anti-CD28 (0.5 µg/ml) mAbs and human TGF-β (2.5 ng/ml) and IL-2 (conc.) for 72 hours.

### Immunoprecipitation and Western blotting

Simian virus 40 large T antigen-transfected human leukemic Jurkat T cells (JTAg) and MCC-specific hybridoma T cells were described previously. JTAg cells in logarithmic growth phase were transfected with plasmid DNAs by electroporation and incubated for 48 hours. Transfected cells were stimulated with OKT3 mAb and recombinant B7-Fc in the presence of cross-linking Ab for 5 min. Cell lysis in 1% digitonin lysis buffer (50 mM Tris-HCl, 50 mM NaCl, 5 mM EDTA), immunoprecipitation and Western blotting were carried out as previously described.

### Enzyme-linked Immunosorbent assay (ELISA)

Serum level of IgE was quantified using capture and biotinylated mAbs from CALTAG Laboratories, as previously described. Autoantibodies specific for double-stranded DNA (dsDNA) and histone were determined using plates coated with salmon sperm DNA (Life Technologies, CA) and calf thymus histone (Roche), respectively. Detection was carried out using biotinylated anti-mouse IgG, streptavidin conjugated HRP and ABTS substrates (BioRad, CA). Ig serum levels were calculated by dividing the absorbance value of samples by negative controls.

### Isolation of mRNA, cDNA synthesis and real-time PCR

Total RNA was extracted from sorted CD4+GFP- and CD4+GFP+ cells from FIG mice using the RNeasy kit (Qiagen, CA). RNA was used to synthesize cDNA by the SuperScript III FirstStrand cDNA synthesis kit (Life Technologies, CA). Gene expressions were determined using real-time PCR with iTaq SYBR Green (Bio-Rad, CA) in the presence of the following primer sets for mouse CTLA-4 (Forward: 5' ACTCATGTACCCACCGCCATA 3' (SEQ ID NO: 10); Reverse: 5' GGGCATGGTTCTGGATCAAT 3' (SEQ ID NO: 11)), Prkcq (Forward: 5' AACTTTGACTGTGGGACCTG 3' (SEQ ID NO: 12); Reverse: 5' AAGGTGGTACATGG TTGG 3' (SEQ ID NO: 13)), Prkch (Forward: 5' CAAGCATTTTACCAGGAAGCG 3' (SEQ ID NO: 14); Reverse: 5' TGTTTCCCAAATACTCCCCAG 3' (SEQ ID NO: 15)) and the housekeeping gene β-actin. The relative gene expression levels were determined in triplicates and calculated using the 2-ΔΔCt normalizing to the level of the housekeeping gene β-actin.

### Immunofluorescence microscopy

Planar lipid bilayers were prepared mixing liposomes that contained DOPC + 0.25 mol% biotin-CAP phosphatidylethanolamine (Avanti Polar Lipids, Alabaster, AL) and placed on an acid Piranha solutioncleaned coverslips mounted in an 8-well chamber (Labtek). After blocking and fluorescent conjugated streptavidin (4 µg/ml) were incorporated in the bilayers, a mixture of monobiotinylated anti-CD3ε and anti-CD28 mAbs (5 µg/ml) were added. Unbound Abs were washed away prior to placing purified ovalbumin-specific TCR-transgenic OT-I CD4+CD25+ Treg or CD4+CD25- Teff cells on the bilayers for 6 min at 37°C. Cells were fixed with 4% paraformaldehyde, permeabilized with 0.3% Triton X-100, and blocked with 10% normal goat serum. Cells were incubated with rabbit anti-PKCθ isoforms (sc- 212) or -PKCη (sc-215) Abs followed by fluorescently tagged goat anti-rabbit (Fab')2 Ab (Invitrogen, Carlsbad, CA). Nuclei were counterstained using DAPI (1 µg/ul). Controls included the use of Prkch-/- or Prkcq-/- T cells. Cells were imaged from top to the bottom every 0.3 µm using an inverted Zeiss 200M microscope using the appropiate filters. Images were background-substracted and deconvolved by the nearest neighbors method using Slidebook 5.0 software (3i Intelligent Imaging Innovations).

CD4+ T cells were purified from moth cytochrome C (MCC)-specific mice expressing the transgenic AND TCR crossed with Rag2-/- (AND-Tg x Rag2-/-) mice and stimulated by immobilized anti-CD3ε (145-2C11; 10 µg/ml) and anti-CD28 (PV-1; 1 µg/ml) in the presence of mouse recombinant IL-2 (10 ng/ml) and human recombinant TGFβ (5 ng/ml) for 3 d. The cells were retrovirally transduced with vectors encoding eGFP-tagged mouse PKC-θ or PKC-η for 24 h 1 d after the initial stimulation. On day 4 or later, the cells were sorted on a FACSAria (BD) to obtain purified (≥90%) GFP+ cells, which were maintained in culture. B cells purified from B10.BR mice and stimulated by LPS (Difco; 10 µg/ml) plus MCC (1 µM) were added to the cultured T cells for 1 d. Dead cells were removed by Lympholite-M (Cedarlane), and the CD4+ Treg cells were prestained by DyLight650-labeled anti-TCRβ (H57) Fab, conjugated with MCC-pulsed LPS-stimulated B cells for 5-10 min, fixed with 2% PFA, and imaged by confocal microscopy (Leica SP5) with ProLong gold antifade reagent with DAPI (Molecular Probes).

### In vitro suppression assay

FACS-sorted naive CD4+CD62L+ cells (Tresp) were labeled with 5 µM of CellTrace Violet according to the manufacturer's protocol (Life Technologies, CA). CD11c+ splenic dendritic cells were purified according to the manufacturer's instruction (Miltenyi Biotec). Labeled Teff (2 x 10⁴) and splenic DCs (5 x 10³) were cocultured for 3 days with CD4+GFP+ Tregs (ranging from 0.125-4 x 10⁴) sorted from FIG and Prkch-/--FIG mice. Anti-CD3 mAb (Clone 145-2C11, Biolegend) was added at a final concentration of 2 µg/ml to the cultures.

### Homeostatic Expansion Model

Naive CD4+CD62L+ cells from congenic B6.SJL and CD4+GFP+ Tregs cells from FIG and Prkch-/--FIG mice were sorted using ARIA Cell Sorter (BD Bioscience, San Diego). 2 x 10⁶ naive CD4+CD62L+ cells were transferred alone or cotransferred with 0.5 x 10⁶ of CD4+GFP+ Tregs cells intravenously into Rag1-/- mice. Mice were euthanized 7-10 days post-transfer. Spleens, peripheral lymph nodes and mesenteric lymph nodes were harvested and each population was independently analyzed by flow cytometry. To achieve reasonable power, at least 5 mice/group (15 mice/study) were used. Additional mice were added to the studies as appropriate.

### B16 Melanoma Model

Splenocytes from WT B6 were depleted of CD25+ cells using biotinylated anti-CD25 mAb (Clone PC61, eBiosciences) and strepavidin-conjugated beads (BD Biosciences). CD4+GFP+ Tregs were FACS-sorted from FIG and Prkch-/--FIG mice. 15 x 10⁶ CD25-depleted splenocytes were adoptively transferred alone or together with 0.5 x 10⁶ WT or Prkch-/- CD4+GFP+ Tregs into recipient Rag1-/- mice. 2 x 10⁵ B16-F10 melanoma cells were inoculated intradermally on the right shaved flanks the next day. Tumor size was measured using an electronic dial caliper 2-3 times/week. To achieve reasonable power, at least 5 mice/group (15 mice/study) were used. Additional mice were added to the studies as appropriate.

### BM chimeras

Full-length human Prkch and S28/32A mutant were sub-cloned into a modified pMIG retroviral vector containing IRES and non-signaling rat CD2 gene (lacking the cytoplasmic tail). BM chimeras were produced in irradiated B6 mice as previously described. Briefly, BM cells were flushed from the femurs and tibias of Prkch-/--FIG mice that have been pretreated with 5-fluorouracil to enrich for stem cells. BM cells were cultured in DMEM media (Mediatech Inc, WI) containing 10% FBS, 20 ng/ml of IL-3, 25 ng/ml of IL-6 and 100 ng/ml of SCF. Retroviral infections were carried out for 2 consecutive days. 1 x 10⁶ infected BM cells were intravenously injected into irradiated B6 mice. Analyses were performed 10-12 weeks post-transplant to determine for cells co-expressing GFP (for Foxp3 expression) and rat CD2 (for transgene expression) using anti-rCD2 mAb (Clone OX-34, Biolegend). Cells were pooled from spleens and peripheral lymph nodes of 4-5 BM chimeric mice, and enriched for CD4+ cells. Double positive cells coexpressing GFP and rCD2 were sorted using ARIA Cell Sorter (BD Bioscience, San Diego). To examine their in vivo suppressive function, sorted GFP+rCD2+ reconstituted cells were coinjected i.v. along with naive CD45.1+CD4+CD62L+ cells from B6.SJL mice at a 1:5 ratio into recipient Rag1-/- mice. Spleens, peripheral lymph nodes and mesenteric lymph nodes were harvested 7- 10 days post-transfer and each population was independently analyzed by flow cytometry.

### Treg cell-APC coculture.

GFP+ Treg cells (5 × 104) from Prkch+/+or Prkch-/- FIG mice were cultured with CellTrace Violet-labeled splenic CD11c+ APCs (5 × 104) for the indicated times. Cells were carefully collected with cut tips and immediately assayed on an LSRII flow cytometer to determine double-positive (GFP+Violet+) conjugates. For the CD86 depletion study, Treg cells (5 × 104) were cultured with CD45.2+CD11c+ splenic DCs (2.5 × 104) for 9 h, followed by addition of CD45.1+CD11c+ splenic DCs (2.5 × 104) from B6.SJL mice for another 9 h. Cells were collected at different time points and stained with fluorophore-conjugated Abs specific for CD11c, Annexin V, CD4, CD86, I-Ab, CD45.1 or CD45.2.

### SILAC and phosphoproteomic analysis.

Prkch+/+and Prkch-/- FIG naive CD4+ T cells were differentiated into iTreg cells as described above in regular RPMI-1640 medium or medium supplemented with 13C and 15N-labeled lysine and arginine for SILAC labeling. GFP+ Treg cells sorted by flow cytometry were left unstimulated or stimulated with anti-CD3 plus anti-CTLA-4 mAbs for 5 min. Prkch+/+ and Prkch-/- FIG cell lysates were mixed at a 1:1 ratio, and 300 µg of the protein mixture was precipitated with 5x volume of cold acetone. After centrifugation at 14,000g (10 min at 4 °C), protein pellets were solubilized and reduced with 100 mM Tris-HCl, 8 M urea and 5 mM tris(2-carboxyethyl)phosphine. Cysteine was alkylated with 10 mM iodoacetamide. The solution was diluted 1:4 and digested with 5 µg of trypsin at 37 °C overnight. Digestion was terminated by adding 10% acetonitrile and 2% formic acid, and the resulting peptides were subjected to TiO2 phosphopeptide enrichment as described. Briefly, phosphopeptides were bound to the TiO2 resin, and eluted with 250 mM NH4HCO3, pH 9. Enriched phosphopeptides were analyzed by the MudPIT LC-MS/MS method. MS analysis was performed using an LTQ-Orbitrap Velos mass spectrometer (Thermo Fisher). A cycle of one full-scan mass spectrum (300-1,800 m/z) at a resolution of 60,000 followed by 20 data-dependent MS/MS spectra at a 35% normalized collision energy was repeated continuously throughout each step of the multidimensional separation.

MS data were analyzed by the Integrated Proteomics Pipeline IP2 (Integrated Proteomics Applications; http://www.integratedproteomics.com/). The tandem mass spectra were searched against the European Bioinformatics Institute's International Protein Index mouse target-decoy protein database. Protein false discovery rates were controlled below 1% for each sample. In ProLuCID database search, the cysteine carboxyamidomethylation was set as a stable modification, and phosphorylation on serine, threonine or tyrosine was configured as differential modification. Peptide quantification was performed by Census software, in which the isotopic distributions for both the unlabeled and labeled peptides were calculated and this information was then used to determine the appropriate mass-to-charge ratio (m/z) range from which to extract ion intensities. Phosphopeptides were further evaluated with IP2 phospho analysis module, which computes Ascore and Debunker score.

### Statistical analysis

Statistical analyses were performed using, unless otherwise stated, one-way-ANOVA with post-hoc Bonfferoni's corrections. Unless otherwise indicated, data represent the mean ± SEM, with p < 0.05 considered statistically significant.

### Example 2: PKCη is recruited to the Treg IS

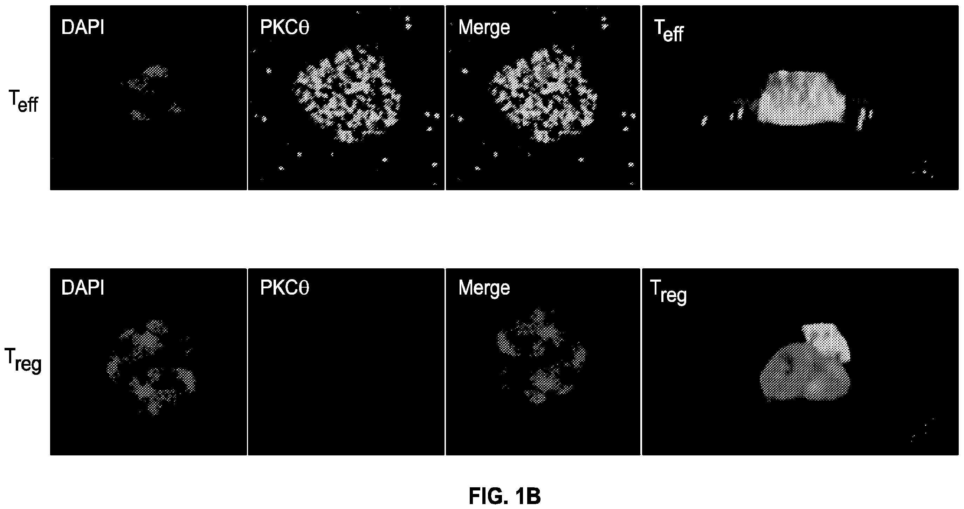

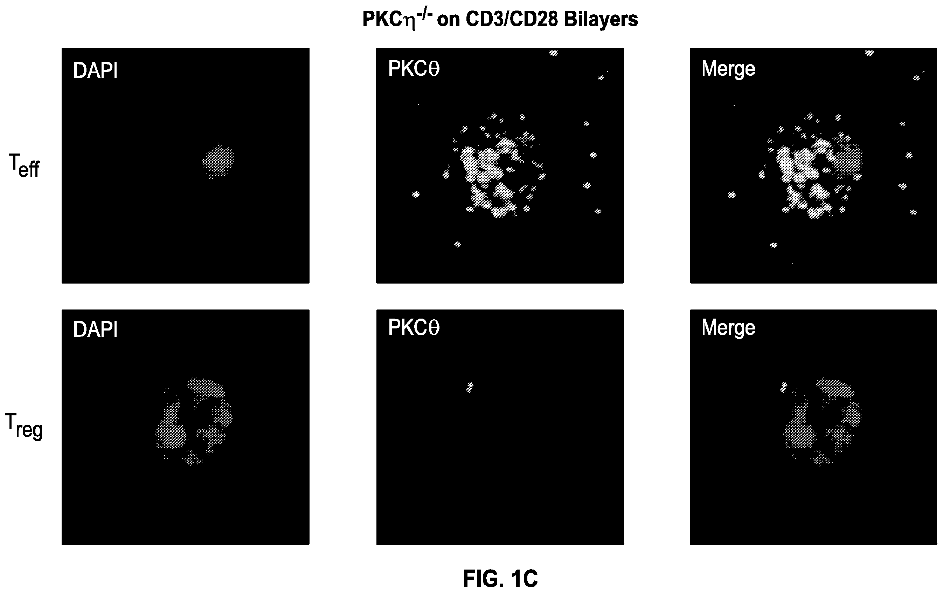

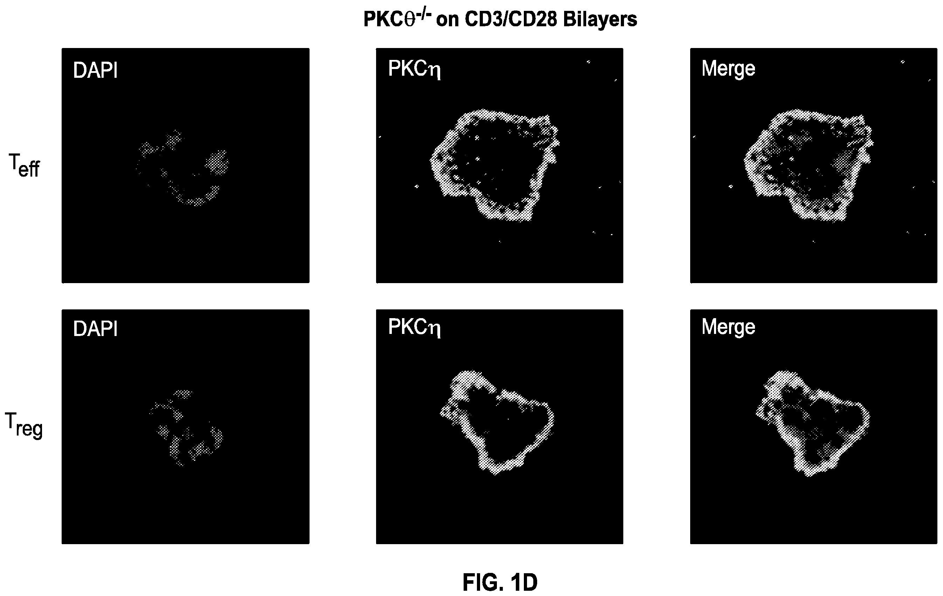

To determine whether a PKC isoform other than PKCθ is recruited to the Treg IS, the PKCη isoform was analyzed for being present in the IS of Tregs. Using a lipid bilayer system and deconvolution microscopy, it was observed that, in addition to primary Teff cells, PKCη was also localized in the Treg IS (Fig. 1A). PKCθ was recruited to the Teff IS, but it was excluded from the IS of Tregs (Fig. 1B). It was also determined that the translocation of the two PKC isoforms to the IS was independent of each other. Thus, the recruitment of PKCθ to the Teff (but not Treg) IS was not affected in cells from PKCη- deficient (Prkch-/-) mice (Fig. 1C) and, vice versa, the localization of PKCη in the IS of Teffs and Tregs was not perturbed in PKCθ-deficient (Prkcq-/-) in T cells (Fig. 1D).

It was investigated whether the loss of PKCη could affect Treg development and/or function. There was a moderate lymphadenopathy in Prkch-/- mice, reflecting increased T and B cell numbers. Furthermore, Prkch-/- mice harbored a higher proportion of CD44hi T cells, characteristic of an activated phenotype, than their wild-type (WT) counterparts. Indeed, Prkch-/- CD44hi T cells secreted significantly elevated amounts of effector cytokines, including IL-2, IFNy, IL-4, and IL-17A (Fig. 2A D), upon in vitro stimulation with anti-CD3 plus -CD28 monoclonal antibodies (mAbs). Consistent with this hyperactive phenotype, Prkch-/- mice displayed elevated serum levels of IgE (Fig. 2E) and autoantibodies against double-stranded DNA and histone (Fig. 2F,G, respectively) at 8-12 weeks of age, implying a deregulated, hyperactive immune system in the absence of PKCη.

### Example 3: Phenotypic and functional characterization of Prkch-/- and Prkcq-/- mice

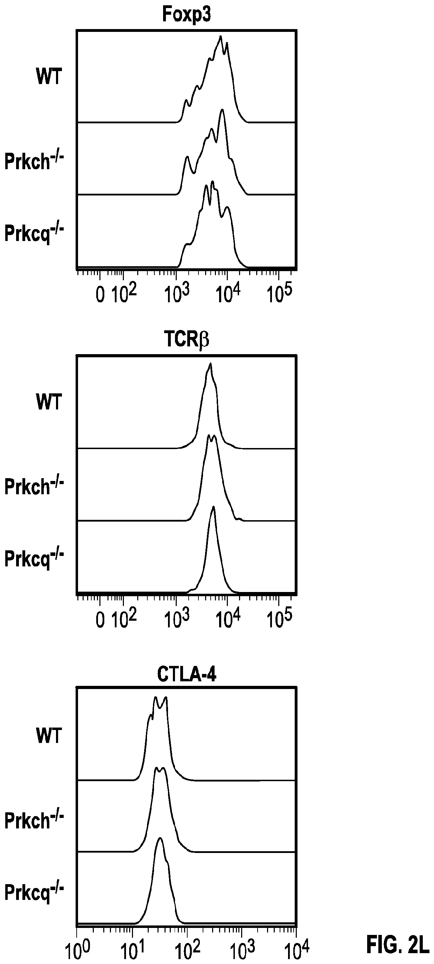

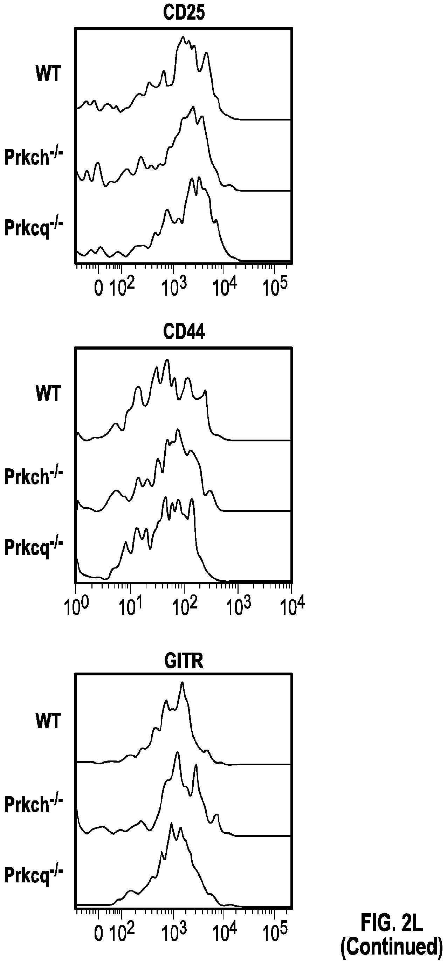

The CD4+Foxp3+ Treg population in the lymphoid organs of WT, Prkch-/-, and Prkcq-/- mice was examined by intracellular Foxp3 staining. The frequency of CD4+Foxp3+ cells was not significantly altered in the thymi and spleens of Prkch-/- mice. However, there was a significant increase in the numbers of CD4+Foxp3+ Treg cells in the peripheral (pLN) and mesenteric (mLN) lymph nodes of these mice (Fig. 2H-K, Fig. 5). On the other hand, the frequency and number of CD4+Foxp3+ Treg cells were significantly reduced in Prkcq-/- mice in all lymphoid organs examined (Fig. 2H-2K, Fig. 5), consistent with the important role of PKCθ in Treg development. Nonetheless, phenotypically, cells that have been "licensed" to become Foxp3+ expressed similar levels of typical Treg markers, including Foxp3, TCR-β chain, CTLA-4, CD25, CD44, and glucocorticoid-induced tumor necrosis factor receptor (GITR) in all three mouse strains (Fig. 2L, Fig. 6).

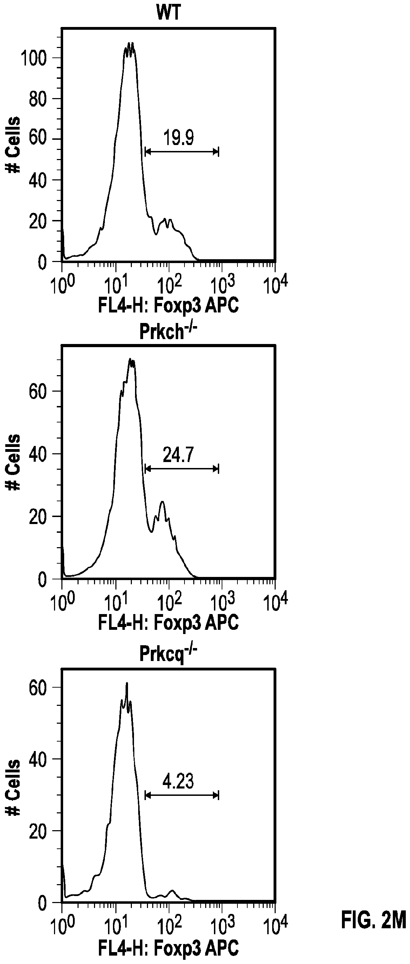

It was determined whether PKCη (or PKCθ) is used in the in vitro differentiation of FoxP3+ T cells. When cultured in the presence of TGF-β, CD4+CD62L+ naive T cells from Prkch-/- mice displayed, a similar proportion of Foxp3+ cells and FoxP3 mean fluorescence intensity (Fig. 2M), as well as similar CTLA-4 expression levels (not shown), to those observed in WT T cell culture, indicating that PKCη is dispensable for the in vitro differentiation of FoxP3+CTLA-4high T cells per se. In contrast, the differentiation of Prkcq-/- naive T cells into Foxp3+ cells was severely impaired, thereby extending an earlier report documenting the importance of this PKC isoform in Treg development in vivo . Despite the paucity of FoxP3+ T cells in Prkcq-/- mice, they show no overt signs of autoimmunity or inflammation, most likely because the differentiation and function of pathogenic Th2, Th17 and, to certain extent, Th1 effector cells are dependent on PKCθ. Hence, in this in vivo environment, the Treg defect is functionally less apparent. Yet, Prkch-/- mice, which do show modest signs of autoimmunity and hyperactivation, have an unaltered (or even increased) FoxP3+ T cell population.

### Example 4: PKC-η is used in Foxp3+ Treg cell suppressive function.

To resolve this apparent discrepancy, it was determined whether PKCη might be required for the suppressive function of Foxp3+ Tregs. To enable definitive identification of Tregs, the Prkch-/- mice were crossed with mice coexpressing Foxp3 and enhanced GFP under the control of the endogenous Foxp3 promoter (Foxp3-IRES-eGFP, hereafter called FIG) to generate Prkch-/--FIG mice. The naturally occurring CD4+GFP+ Tregs from FIG and Prkch-/FIG mice were FACS-sorted, and they produced similar quantities of IL-10 upon stimulation (Fig. 3A). However, in a conventional in vitro Treg suppression assay, which assessed the proliferation of naive T cells (Teff) cocultured with Tregs, the percentage of dividing Teff cells was consistently higher in cultures with Prkch-/- Tregs, compared to Teff cocultured with WT Tregs (Fig. 3B, Fig. 14). Thus, the Prkch-/- Tregs clearly failed to suppress the proliferation of Teff cells even at high Treg:Teff ratios, indicating that PKCη expression by Tregs is important for their function.

To demonstrate the importance of PKCη in Treg function in vivo, two distinct study models were used, namely, homeostatic T cell expansion and tumor growth. Treg cells have been demonstrated to control the homeostatic expansion of Teff cells in a lymphopenic environment. Purified naive CD45.1+CD4+ T cells, either alone or in the presence of FACS-sorted WT or Prkch-/- CD45.2+CD4+GFP+ Treg cells, were adoptively transferred into Rag1-/- mice, and T cell numbers were determined one week post-transfer. In the presence of WT Tregs, CD45.1+ (Teff) T cell expansion was significantly reduced in all secondary lymphoid organs examined; in contrast, minimal or no reduction in Teff cell expansion was observed in the presence of Prkch-/- Tregs (Fig. 3C-E). Both WT and Prkch-/- Tregs populations showed a similar degree of homeostatic proliferation (Fig. 7).

The ability of Prkch-/- Tregs to inhibit the immune response against a growing tumor was investigated. Splenocytes depleted of CD25+ T cells were adoptively transferred into Rag1-/- mice as a source of Teff cells in the absence or presence of Treg cells one day prior to inoculation of B16-F10 melanoma cells. Transfer of CD25+-depleted splenocytes alone resulted in small skin tumors, whereas mice receiving Teff cells together with WT Tregs developed massive B16 tumors, reflecting inhibition of the Teff anti-tumor response by the cotransferred Tregs. Interestingly, cotransfer of Prkch-/- Treg cells resulted in substantially reduced tumor growth similar to that seen in mice receiving only Teff cells (Fig. 3F). Taken together, these results (Figs. 3C-F) indicate that in the absence of PKCη, the in vivo suppressive function of Tregs is attenuated, leading to enhanced homeostatic proliferation and anti-tumor immunity.

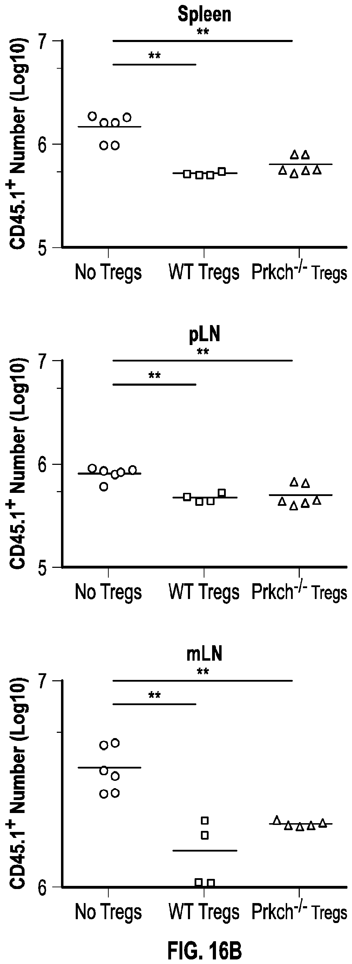

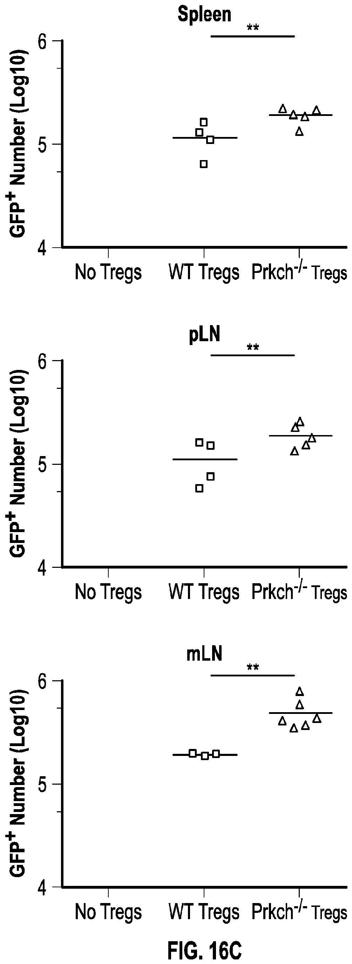

Also assessed was the ability of Prkch+/+ and Prkch-/- Treg cells to inhibit the development of autoimmune colitis in an established T cell transfer model. Although transfer of naive T cells alone into Rag1-/- recipient mice induced weight loss, indicative of the development of chronic inflammatory bowel disease, cotransfer of either Prkch+/+ or Prkch-/- GFP+ Treg cells protected the recipients against weight loss (Fig. 16a) and inhibited the expansion of Teff (CD45.1+) cells (Fig. 16b). Thus, despite the in vitro (Fig. 14) and in vivo (Fig. 3c-f) severe defects in their suppressive function, Prkch-/- Treg cells were still able to protect, albeit perhaps incompletely, recipient mice against the development of colitis. Without being limited to any one theory, this finding indicates that in this particular disease model, Prkch-/-Treg cells use an alternative, PKCη-independent suppressive mechanism(s), e.g., IL-10-mediated suppression (Fig. 3a). Furthermore, increased proliferation (or localization) of Prkch-/- Treg cells in the inflammatory bowel environment may compensate for their defective intrinsic suppressive function. Indeed, there was found greater numbers of cotransferred Prkch-/- Treg cells relative to Prkch+/+ Treg cells in the secondary lymphoid organs of the recipient mice, and this effect tended to be more pronounced in mesenteric lymph nodes, which drain the site of inflammation (Fig. 16C). Together, these findings indicate that PKC-η is not globally required for all forms of Treg cell-induced suppression, and that it may be dispensable for Treg cell-mediated inhibition of colitis.

### Example 5: Interaction of phosphorylated PKCη with CTLA-4

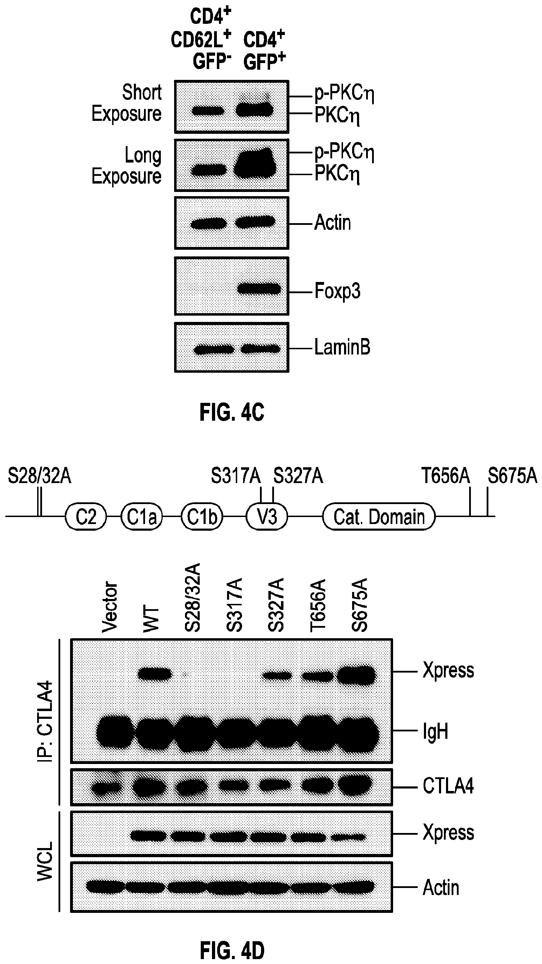

In order to elucidate the molecular basis for the importance of PKCη in the suppressive function of Tregs, and given the localization of both CTLA-4 nd PKCη (Fig. 1B) in the Treg IS, PKCη was anaylzed for interaction with CTLA-4, by analogy with the PKC8-CD28 interaction in the IS of Teff cells. CTLA-4 is highly expressed in Tregs, and it plays an important role in Treg function. However, currently known CTLA-4-associated phosphatases, including SHP1, SHP2 and PP2A, are not recruited to the IS and, thus, how CTLA-4 exerts its effects at the IS of Tregs is unknown. Using a T hybridoma cell line, it was found that CTLA-4 coimmunoprecipitated with a higher molecular weight (MW) species of PKCη (Fig. 4A), but not with any other T cell-expressed PKC isoform (Fig. 8). The MW shift of PKCη may be a result of its phosphorylation. Indeed, alkaline phosphatase treatment partially reversed the shift of the higher MW species of PKCη (Fig. 4B), indicating that CTLA-4 interacts predominantly with the phosphorylated species of PKCη. Consistently, phosphorylated PKCη was found in Foxp3+ Tregs, but not in naive T cells (Fig. 4C), despite the fact that the expression of PKCη was not significantly altered at the transcriptional and translational levels in Tregs (Fig. 9).

To pinpoint the potential phosphorylation site(s) in PKCη for its interaction with CTLA-4, six predicted or documented phosphorylation sites in PKCη were mutated and CTLA-4 coimmunoprecipitations in JTAg, a Jurkat cell derivative that does not express CD28 performed. Upon costimulation with anti-CD3 mAb and B7-Fc recombinant protein, it was found that mutations of amino acid residues S28 and S32 in the C2 domain, or S317 in the V3 domain of PKCη abolished the interaction with CTLA-4; as a control, mutation of three other phosphorylation sites (S327, T656 or S675) did not affect this interaction (Fig. 4D). Thus, phosphorylation of S28/S32 or S317 in PKCη is an important factor for the interaction with CTLA-4.

In order to determine whether the association between PKCη and CTLA-4 is used in the suppressive function of Tregs, bone marrow (BM) chimeric mice on a Prkch-/--FIG background that were transduced retrovirally with WT PKCη or a CTLA-4 non-interacting mutant (PKCη-S28/32A) coexpressing a non-signaling rat CD2 to allow isolation of transduced (rCD2+) T cells were generated. Transduced (rCD2+GFP+CD45.2+) Tregs from these mice were FACS-sorted and assayed for their ability to suppress the in vivo homeostatic proliferation of cotransferred naive CD45.1+ T cells. The results show that, unlike Tregs expressing WT PKCη, Tregs reconstituted with PKCη-S28/32A were incapable of suppressing naive T cell proliferation in lymph nodes (Fig. 4E-F), and only partially inhibited the homeostatic expansion in the spleen (Fig. 4G).

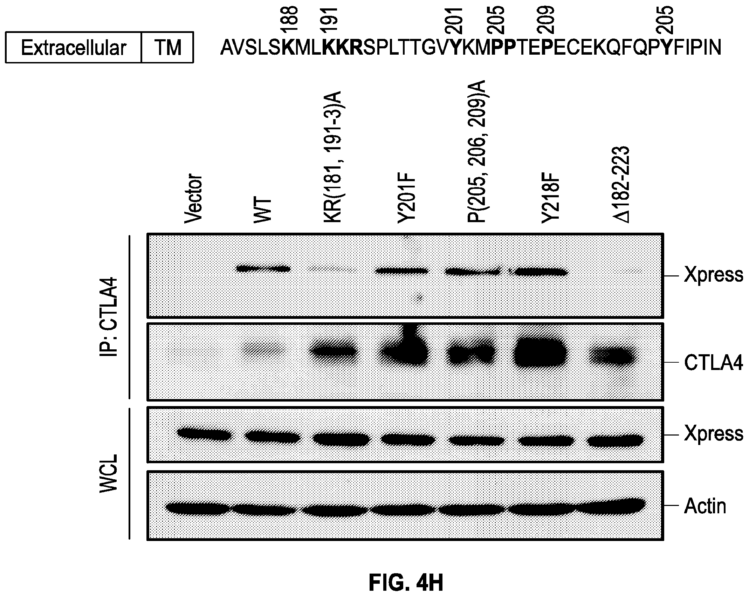

To map the important motif within the cytoplasmic tail of CTLA-4 that is used in the interaction with PKCη, the membrane proximal positively charged motif (K188, 191KKR193), the proline-rich motif (P205, 206, 209), and tyrosine residues (Y201 or Y218) were mutated, and the corresponding mutants examined for their ability to associate with PKCη. Mutation of the positively charged motif as well as complete deletion of the CTLA-4 cytoplasmic tail (Δ182-223) was found to greatly reduce the association with PKCη; in contrast, mutations of the conserved tyrosine residues or the proline-rich motif did not affect the interaction of CTLA-4 with PKCη (Fig. 4H). This positively charged motif is highly conserved throughout evolution from fish to primates (Fig. 10). Partial truncation of the cytoplasmic tail of CTLA-4 (Δ192-223), which left the ten membrane proximal residues, including K188 and K191, resulted in a less severe reduction of the interaction with PKCη (Fig. 11). Interestingly, CTLA-4 with a similar partial truncation has been reported to retain some downstream functions in Tregs. It was also found that the interaction between CTLA-4 and PKCη was not affected by PP2, an inhibitor of Src-family kinases (Fig. 12), consistent with the lack of effect of Y201 or Y218 CTLA-4 tail mutations on this interaction. Taken together, these results indicate that the CTLA-4-PKCη interaction is necessary for the suppressive function of Tregs, thereby implicating PKCη in a signaling axis linking CTLA-4 to Treg-mediated suppression.

### Example 6: Impaired APC dissociation and CD86 depletion by Prkch-/- Treg cells

In comparison to Teff cells, Treg cells preferentially form aggregates with APCs, and this is due in part to the higher expression of adhesion molecules such as LFA-1 (ref. 26) and neuropilin-1 (ref. 27) on Treg cells. Such Treg cell-APC engagement has been implicated as a potential suppression mechanism, as it allows Treg cells to effectively compete with Teff cells in engaging APCs and, thus, inhibit Teff cell activation. However, activation of LFA-1 and its conversion to a high-affinity state, as measured by adhesion to its ligand, ICAM-1, was intact in Prkch-/- Treg cells (Fig. 17). Prkch+/+ and Prkch-/- Treg cells expressed similar levels of neuropilin-1, CD39 and CD73; the latter two are cell-surface molecules that have been implicated in Treg cell-mediated suppression through an adenosine-dependent action28.

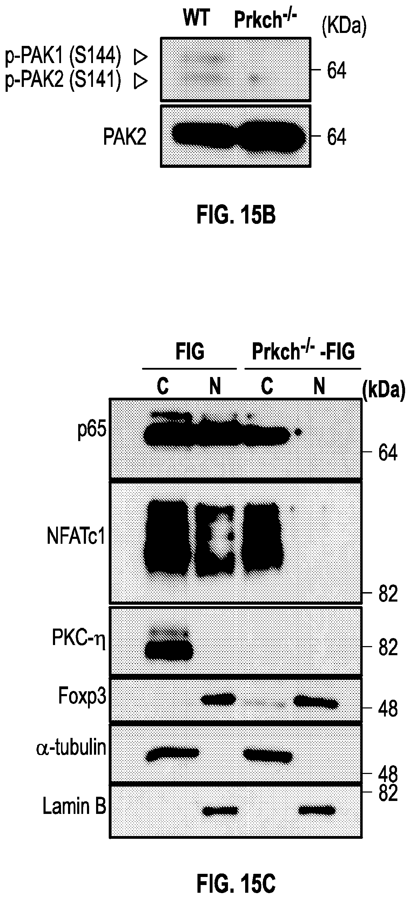

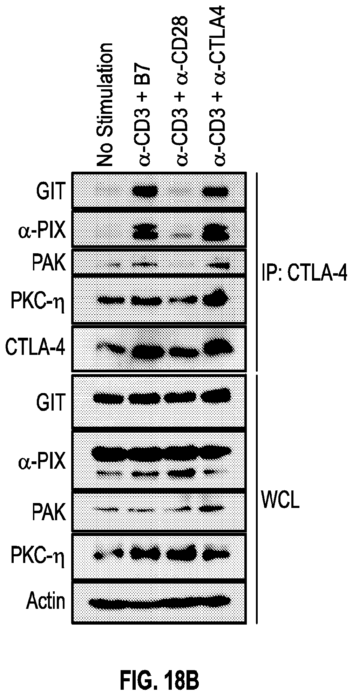

To elucidate the signaling mechanism potentially responsible for the PKC-η-mediated suppression, a phosphoproteomic analysis of Prkch+/+ versus Prkch-/- Treg cells was performed and it was found that PAK2 and GIT2, two components of a focal adhesion complex that promotes focal adhesion disassembly and, hence, cellular motility, were substantially hypophosphorylated in Prkch-/- Treg cells (Fig. 18). A complex of these two proteins together with the guanine nucleotide exchange factors αPIX or βPIX has been found to translocate to the T cell IS and to be needed for optimal Teff cell activation31. Moreover, CTLA-4 immunoprecipitates from anti-CD3- plus anti-CTLA- 4-costimulated Prkch+/+ Treg cells contained not only PKC-η but also GIT2, αPIX and PAK (Fig. 15A). These proteins were also present in PKC-η immunoprecipitates. Of note, recruitment of this complex was unique to CTLA-4 costimulation, because the association of the GIT2-αPIX-PAK complex with CTLA-4 was barely above background level when the cells were costimulated with anti- CD3 plus anti-CD28 mAbs (Fig. 18). Thus, this particular signaling event is not shared between CTLA-4 and CD28. Furthermore, the activating phosphorylation of PAK kinases was dramatically reduced in Prkch-/- Treg cells (Fig. 15B), which indicated impaired activation of this complex.

Given the impaired activation of PAK, and because PAK was found to be needed for TCR induced transcriptional activation of NFAT and the CD28 response element (which includes an NF-κB binding site) in Jurkat T cells the it was also examined whether Prkch-/- Treg cells display impaired activation of these transcription factors after costimulation with anti-CD3ε plus anti-CTLA-4 mAbs. In these conditions, a severe defect in NFATc1 and NF-κB activation in the Prkch-/-Treg cells was observed (Fig. 15C).

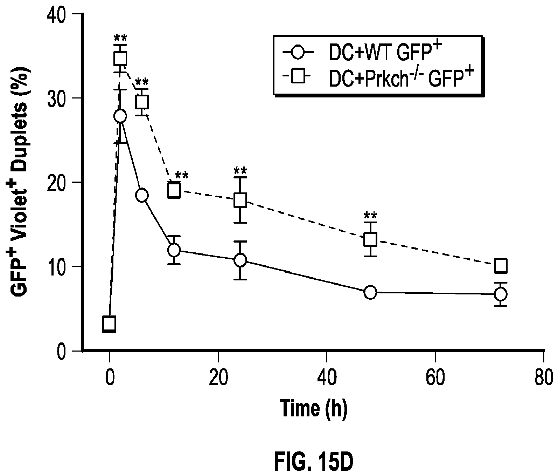

Because the GIT1-PIX-PAK complex promotes cellular motility through focal adhesion disassembly, whether the defective activation of the GIT1-αPIX-PAK complex in Prkch-/- Treg cells might result in a more stable conjugation of Prkch-/- Treg cells with APCs was analyzed. The efficiency of conjugation between Prkch-/- Treg cells and APCs was significantly higher by comparison to APC conjugates of Prkch+/+ Treg cells (Fig. 15D), indicating that in Prkch-/- Treg cells impaired activation of the GIT2-αPIX-PAK complex leads to defective breaking of Treg cell-APC contacts.

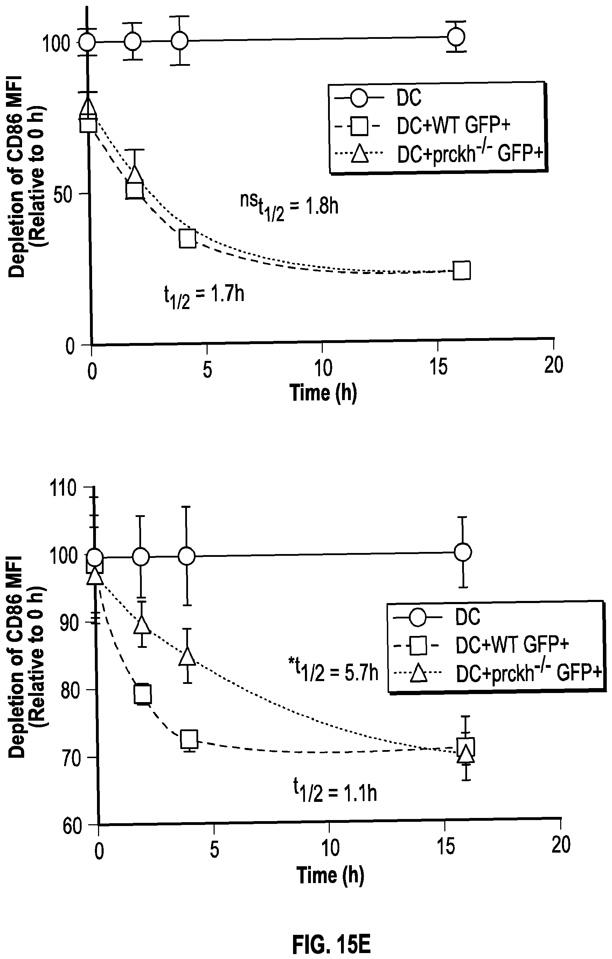

If Prkch-/-Treg cells display impaired dissociation from engaged APCs because of defective activation of the GIT2-αPIX-PAK complex, this defect may be expected to translate into reduced ability of Prkch-/- Treg cells to serially engage new APCs, which, in turn, could result in reduced suppressive activity. This prediction is based on findings that Treg cells can capture CD80 and CD86 from APCs, a process that depletes the ligands used in CD28 costimulation of Teff cells and is thus a potential mechanism of contact-dependent Treg cell-mediated suppression. To address this possibility, it was tested the ability of Prkch+/+ versus Prkch-/- Treg cells to deplete CD86 from cocultured CD45.2+CD11c+ APCs as an indirect measure of APC engagement by the Treg cells. Prkch+/+ and Prkch-/- Treg cells were equally capable of depleting CD86 from these APCs (Fig. 15E). However, upon subsequent addition of a second pool of APCs, distinguished from the first APC population by their CD45.1 expression, Prkch-/- Treg cells had a significant delay in their ability to deplete CD86 from these newly introduced APCs, as indicated by the fact that it took them about fourfold longer (∼16 h versus ∼4 h) to execute the same level of CD86 depletion as that accomplished by Prkch+/+ Treg cells (Fig. 15E). This observation supports the notion that the relative inefficiency of Prkch-/- Treg cells in serially engaging new APCs and, hence, in effectively depleting APC-expressed CD86, could account, at least in part, for their reduced suppressive activity. These findings imply that a Treg cell-intrinsic signaling mechanism dependent on CTLA-4-PKC-η is needed in order to manifest the cell-extrinsic suppressive function of CTLA-4 toward Teff cells.

### Example 7: Discussion

CTLA-4 is a potent regulator that negatively mediates T cell-mediated immune responses. CTLA-4-/- mice display fatal lymphoproliferative disorder characterized by the systemic infiltration of pathogenic self-reactive T cells. However, the basis for the dramatic lethal phenotypes and the mechanisms of action of CTLA-4 are still debatable. This lack of clarity may reflect the fact that most of the earlier studies addressing the inhibitory mechanisms of CTLA-4 did not distinguish between the contributions of CTLA-4 deletion in Teff vs. Treg cells to the overall phenotype. With the conditional deletion of CTLA-4 in Tregs only, it became clear that CTLA-4 expression by Tregs is an important factor for their suppressive function. Despite the modest lymphoproliferative and autoimmune phenotypes, Prkch-/- mice lived into adulthood (∼ 1 year) with no gross signs of pathology. Several important functional disparities could likely account for these differential phenotypes. Firstly, CTLA-4 inhibits negative selection during thymocyte development. As a result, Ctla4-/- mice harbor autoreactive T cells that cause tissue damage. However, the process of thymic selection is intact in the absence of PKCη. Hence, the lack of overt autoreactivity in Prkch-/- mice might limit the self-destructive nature of the hyperactive Prkch-/- T cells. Secondly, deletion of PKCη also affects the in vivo proliferation of potentially pathogenic Prkch-/- Teff cells, counteracting the defective Treg function in these mice. Lastly, the inhibitory effect of CTLA-4 is mediated through two non-mutually exclusive mechanisms, i.e., cell-extrinsic and cell-intrinsic mechanisms, and both these mechanisms, as well as their distinct effects of CTLA-4 in Teff and Treg cells, act cooperatively to dampen T cell responses. CTLA-4 has been demonstrated to capture its ligands from APCs via a process called transendocytosis, depleting the B7 ligands used for CD28 costimulation. This cell-extrinsic mechanism utilizes the Y201 motif in the cytoplasmic tail of CTLA-4, which plays no apparent role in PKCη binding. Hence, the combined loss of the CTLA-4 inhibitory effects via both the extrinsic and intrinsic mechanisms (as is the case in Prkch-/- mice) is likely more profound than the loss of either of these additive (or synergistic) mechanisms alone. This notion is supported by two sets of findings: First, Ctla4-/- mice expressing a tailless Ctla4 transgene exhibit lymphadenopathy and accumulation of activated T cells, whereas a complete rescue is observed in transgenic mice expressing Y201V-mutated CTLA-4; and, second, transgenic expression of a ligand-independent isoform of CTLA-4 can substantially delay the early lethality of Ctla4-/- mice , and protect NOD mice from the development of type 1 diabetes. These data provide strong support to the notion that the intracellular signaling of CTLA-4 contributes significantly to immune homeostasis.

In many tumor tissues, infiltrating Tregs restrict the function of Teff cells and therefore, inhibiting certain Treg signaling molecules that are important for their function could lead to enhanced antitumor responses. Here, it was surprisingly discovered that phosphorylated PKCη is recruited to the Treg IS via an obligatory association with the cytoplasmic tail of CTLA-4, thereby enabling the contact-dependent suppressive activity of Tregs. Hence, without being limited to any particular theory as to mechanisms, it has been discovered that the CTLA-4-PKCη axis is a key therapeutic target for Treg-dependent suppression in controlling cancer.

### REFERENCES

Bennett, C.L. et al. The immune dysregulation, polyendocrinopathy, enteropathy, X-linked syndrome (IPEX) is caused by mutations of FOXP3. Nat. Genet. 27, 20-21 (2001).
Fontenot, J.D., Gavin, M.A. & Rudensky, A.Y. Foxp3 programs the development and function of CD4+CD25+ regulatory T cells. Nat. Immunol. 4, 330-336 (2003).
Hori, S., Nomura, T. & Sakaguchi, S. Control of regulatory T cell development by the transcription factor Foxp3. Science 299, 1057-1061 (2003).
Zanin-Zhorov, A. et al. Protein kinase C-theta mediates negative feedback on regulatory T cell function. Science 328, 372-376 (2010).
Collins, A.V. et al. The interaction properties of costimulatory molecules revisited. Immunity 17, 201-210 (2002).
Yokosuka, T. et al. Spatiotemporal basis of CTLA-4 costimulatory molecule-mediated negative regulation of T cell activation. Immunity 33, 326-339 (2010).
Teft, W.A., Kirchhof, M.G. & Madrenas, J. A molecular perspective of CTLA-4 function. Annu. Rev. Immunol. 24, 65-97 (2006).
Kong, K.F. & Altman, A. In and out of the bull's eye: protein kinase Cs in the immunological synapse. Trends Immunol. 34, 234-242 (2013).
Kong, K.F. et al. A motif in the V3 domain of the kinase PKC-theta determines its localization in the immunological synapse and functions in T cells via association with CD28. Nat. Immunol. 12, 1105-1112 (2011).
Kim, J.K. et al. Impact of the TCR signal on regulatory T cell homeostasis, function, and trafficking. PLoS ONE 4, e6580 (2009).
Park, S.G. et al. T regulatory cells maintain intestinal homeostasis by suppressing gammadelta T cells. Immunity 33, 791-803 (2010).
Chuck, M.I., Zhu, M., Shen, S. & Zhang, W. The role of the LAT-PLC-gamma1 interaction in T regulatory cell function. J. Immunol. 184, 2476-2486 (2010).
Spitaler, M., Emslie, E., Wood, C.D. & Cantrell, D. Diacylglycerol and protein kinase D localization during T lymphocyte activation. Immunity 24, 535-546 (2006).
Wing, K. et al. CTLA-4 control over Foxp3+ regulatory T cell function. Science 322, 271-275 (2008).
Qureshi, O.S. et al. Trans-endocytosis of CD80 and CD86: a molecular basis for the cell extrinsic function of CTLA-4. Science 332, 600-603 (2011).
Haribhai, D. et al. Regulatory T cells dynamically control the primary immune response to foreign antigen. J. Immunol. 178, 2961-2972 (2007).
Tang, Q. & Bluestone, J.A. The Foxp3+ regulatory T cell: a jack of all trades, master of regulation. Nat. Immunol. 9, 239-244 (2008).
Josefowicz, S.Z., Lu, L.F. & Rudensky, A.Y. Regulatory T cells: mechanisms of differentiation and function. Annu. Rev. Immunol. 30, 531-564 (2012).
Matsuda, J.L. et al. Systemic activation and antigen-driven oligoclonal expansion of T cells in a mouse model of colitis. J. Immunol. 164, 2797-2806 (2000).
Collison, L.W. et al. The inhibitory cytokine IL-35 contributes to regulatory T-cell function. Nature 450, 566-569 (2007).
Shimizu, J., Yamazaki, S. & Sakaguchi, S. Induction of tumor immunity by removing CD25+CD4+ T cells: a common basis between tumor immunity and autoimmunity. J. Immunol. 163,5211-5218 (1999).
Fu, G. et al. Protein kinase C eta is required for T cell activation and homeostatic proliferation. Sci. Signal. 4, ra84 (2011).
Tai, X. et al. Basis of CTLA-4 function in regulatory and conventional CD4+ T cells. Blood 119, 5155-5163 (2012).
Kataoka, H. et al. CD25(+)CD4(+) regulatory T cells exert in vitro suppressive activity independent of CTLA-4. Int. Immunol. 17, 421-427 (2005).
Onishi, Y., Fehervari, Z., Yamaguchi, T. & Sakaguchi, S. Foxp3+ natural regulatory T cells preferentially form aggregates on dendritic cells in vitro and actively inhibit their maturation. Proc. Natl. Acad. Sci. USA 105, 10113-10118 (2008).
Tran, D.Q. et al. Analysis of adhesion molecules, target cells, and role of IL-2 in human FOXP3+ regulatory T cell suppressor function. J. Immunol. 182, 2929-2938 (2009).
Sarris, M., Andersen, K.G., Randow, F., Mayr, L. & Betz, A.G. Neuropilin-1 expression on regulatory T cells enhances their interactions with dendritic cells during antigen recognition. Immunity 28, 402-413 (2008).
Borsellino, G. et al. Expression of ectonucleotidase CD39 by Foxp3+ Treg cells: hydrolysis of extracellular ATP and immune suppression. Blood 110, 1225-1232 (2007).
Zhao, Z.S., Manser, E., Loo, T.H. & Lim, L. Coupling of PAK-interacting exchange factor PIX to GIT1 promotes focal complex disassembly. Mol. Cell. Biol. 20, 6354-6363 (2000).
Lucanic, M. & Cheng, H.J.A. RAC/CDC-42-independent GIT/PIX/PAK signalling pathway mediates cell migration in C. elegans. PLoS Genet. 4, e1000269 (2008).
Phee, H., Abraham, R.T. & Weiss, A. Dynamic recruitment of PAK1 to the immunological synapse is mediated by PIX independently of SLP-76 and Vav1. Nat. Immunol. 6, 608-617 (2005).
Ku, G.M., Yablonski, D., Manser, E., Lim, L. & Weiss, A.A. A Pak1-PIX-PKL complex is activated by the T-cell receptor independent of Nck, Slp-76 and LAT . EMBO J. 20, 457-465 (2001).
Uhlig, H.H. et al. Characterization of Foxp3+CD4+CD25+ and IL-10-secreting CD4+CD25+ T cells during cure of colitis. J. Immunol. 177, 5852-5860 (2006).
Dustin, M.L. T-cell activation through immunological synapses and kinapses. Immunol. Rev. 221, 77-89 (2008).
Walker, L.S. & Sansom, D.M. The emerging role of CTLA4 as a cell-extrinsic regulator of T cell responses. Nat. Rev. Immunol. 11, 852-863 (2011).
Read, S., Malmström, V. & Powrie, F. Cytotoxic T lymphocyte-associated antigen 4 plays an essential role in the function of Cd25+Cd4+ regulatory cells that control intestinal inflammation. J. Exp. Med. 192, 295-302 (2000).
Tivol, E.A. et al. Loss of CTLA-4 leads to massive lymphoproliferation and fatal multiorgan tissue destruction, revealing a critical negative regulatory role of CTLA-4. Immunity 3, 541-547 (1995).
Waterhouse, P. et al. Lymphoproliferative disorders with early lethality in mice deficient in Ctla-4. Science 270, 985-988 (1995).
Buhlmann, J.E., Elkin, S.K. & Sharpe, A.H. A role for the B7-1/B7-2:CD28/CTLA-4 pathway during negative selection. J. Immunol. 170, 5421-5428 (2003).
Ise, W. et al. CTLA-4 suppresses the pathogenicity of self antigen-specific T cells by cell-intrinsic and cell-extrinsic mechanisms. Nat. Immunol. 11, 129-135 (2010).
Schneider, H. et al. Reversal of the TCR stop signal by CTLA-4. Science 313, 1972- 1975 (2006).
Rudd, C.E. The reverse stop-signal model for CTLA4 function. Nat. Rev. Immunol. 8, 153-160 (2008).
Lu, Y., Schneider, H. & Rudd, C.E. Murine regulatory T cells differ from conventional T cells in resisting the CTLA-4 reversal of TCR stop-signal. Blood 120, 4560-4570 (2012).
Peggs, K.S., Quezada, S.A. & Allison, J.P. Cancer immunotherapy: co-stimulatory agonists and co-inhibitory antagonists. Clin. Exp. Immunol. 157, 9-19 (2009).
Peggs, K.S., Quezada, S.A., Chambers, C.A., Korman, A.J. & Allison, J.P. Blockade of CTLA-4 on both effector and regulatory T cell compartments contributes to the antitumor activity of anti-CTLA-4 antibodies. J. Exp. Med. 206, 1717-1725 (2009).

## Claims

1. An agent for use in a method of treating cancer in a subject wherein the method comprises decreasing, reducing, inhibiting, suppressing or limiting the direct physical interaction between Protein kinase C eta type (PKCη) and Cytotoxic T-lymphocyte protein 4 (CTLA-4) to decrease, reduce, inhibit, suppress or limit regulatory T cell function;
wherein the agent inhibits the direct physical interaction of PKCη with CTLA-4, further wherein the agent is a protein or peptide comprising or consisting of the PKCη amino acid sequence:
MSSGTMKFNGYLRVRIGEAVGLQPTRWSLRHSLFKKGHQLLDPYLTVSVDQVRVGQTSTKQKTNKPTYNEEFCANVTDGGHLELAVFHETPLGYDHFVANCTLQFQELLRTTGASDTFEGWVDLEPEGKVFVVITLTGSFTEATLQRDRIFKHFTRKRQRAMRRRVHQINGHKFMATYLRQPTYCSHCREFIWGVFGKQGYQCQVCTCVVHKRCHHLIVTACTCQNNINKVDSKIAEQRFGINIPHKFSIHNYKVPTFCDHCGSLLWGIMRQGLQCKICKMNVHIRCQANVAPNCGVNAVELAKTLAGMGLQPGNISPTSKLVSRSTLRRQGKESSKEGNGIGVNSSNRLGIDNFEFIRVLGKGSFGKVMLARVKETGDLYAVKVLKKDVILQDDDVECTMTEKRILSLARNHPFLTQLFCCFQTPDRLFFVMEFVNGGDLMFHIQKSRRFDEARARFYAAEIISALMFLHDKGIIYRDLKLDNVLLDHEGHCKLADFGMCKEGICNGVTTATFCGTPDYIAPEILQEMLYGPAVDWWAMGVLLYEMLCGHAPFEAENEDDLFEAILNDEVVYPTWLHEDATGILKSFMTKNPTMRLGSLTQGGEHAILRHPFFKEIDWAQLNHRQIEPPFRPRIKSREDVSNFDPDFIKEEPVLTPIDEGHLPMINQDEFRNFSYVSPELQP (SEQ ID NO: 1), or subsequence thereof, that binds directly to CTLA-4, and the method comprises contacting CTLA-4 with the agent.

2. An *in vitro* method of modulating regulatory T cell function comprising modulating interaction of PKCη with CTLA-4, the method comprising contacting CTLA-4 with an agent that inhibits the direct physical interaction of CTLA-4 with PKCη wherein the agent is a protein or peptide comprising or consisting of the PKCη amino acid sequence:
MSSGTMKFNGYLRVRIGEAVGLQPTRWSLRHSLFKKGHQLLDPYLTVSVDQVRVGQTSTKQKTNKPTYNEEFCANVTDGGHLELAVFHETPLGYDHFVANCTLQFQE LLRTTGASDTFEGWVDLEPEGKVFVVITLTGSFTEATLQRDRIFKHFTRKRQRAMRRRVHQINGHKFMATYLRQPTYCSHCREFIWGVFGKQGYQCQVCTCVVHKRCHHLIVTACTCQNNINKVDSKIAEQRFGINIPHKFSIHNYKVPTFCDHCGSLLWGIMRQGLQCKICKMNVHIRCQANVAPNCGVNAVELAKTLAGMGLQPGNISPTSKLVSRSTLRRQGKESSKEGNGIGVNSSNRLGIDNFEFIRVLGKGSFGKVMLARVKETGDLYAVKVLKKDVILQDDDVECTMTEKRILSLARNHPFLTQLFCCFQTPDRLFFVMEFVNGGDLMFHIQKSRRFDEARARFYAAEIISALMFLHDKGIIYRDLKLDNVLLDHEGHCKLADFGMCKEGICNGVTTATFCGTPDYIAPEILQEMLYGPAVDWWAMGVLLYEMLCGHAPFEAENEDDLFEAILNDEVVYPTWLHEDATGILKSFMTKNPTMRLGSLTQGGEHAILRHPFFKEIDWAQLNHRQIEPPFRPRIKSREDVSNFDPDFIKEEPVLTPIDEGHLPMINQDEFRNFSYVSPELQP (SEQ ID NO: 1), or subsequence thereof, that binds directly to CTLA-4;
thereby decreasing, reducing, inhibiting, suppressing, limiting or controlling interaction between PKCη and CTLA-4 to decrease, reduce, inhibit, suppress, limit or control regulatory T cell function.

3. The agent for use of claim 1 or *in vitro* method of claim 2, wherein PKCη is phosphorylated at S28, S32 and S317.

4. The agent for use or *in vitro* method of any one of claims 1-3, wherein the agent binds to a CTLA-4 amino acid sequence that comprises, consists or consists essentially of from about residue 182 to residue 223 of CTLA-4.

5. The agent for use or *in vitro* method of any one of claims 1-3, wherein the agent binds to a CTLA-4 amino acid sequence having K188, K191, K192 or R193 of CTLA-4.

6. The agent for use or *in vitro* method of any one of claims 1-3 wherein the peptide:
(i) comprises or consists of from residue 28 to residue 317 of SEQ ID NO: 1; and/or
(ii) is phosphorylated at S28, S32 and S317.

## Patentansprüche

1. Mittel zur Verwendung in einem Verfahren zur Behandlung von Krebs in einem Patienten, wobei das Verfahren die Verminderung, Verringerung, Hemmung, Unterdrückung oder Begrenzung der direkten physischen Wechselwirkung zwischen Proteinkinase vom Typ C eta (PKCη) und zytotoxischem T-Lymphozyten-Protein 4 (CTLA-4) zur Verminderung, Verringerung, Hemmung, Unterdrückung oder Begrenzung der Funktion von regulatorischen T-Zellen umfasst;
wobei das Mittel die direkte physische Wechselwirkung von PKCη mit CTLA-4 hemmt,
wobei das Mittel ferner ein Protein oder Peptid ist, das umfasst oder besteht aus der PKCη-Aminosäuresequenz :
MSSGTMKFNGYLRVRIGEÄVGLQPTRWSLRHSLFKKGHQLLDPYLTVSVDQVRVGQTSTKQKTNKPTYNEEFCANVTDGGHLELAVFHETPLGYDHFVANCTLQFQELLRTTGASDTFEGWVDLEPEGKVFVVlTLTGSFTEATLQRDRIFKHFTRKRQRAMRRRVHQINGHKFMATYLRQPTYCSHCREFIWGVFGKQGYQCQVCTCYVHKRCHHUVTACTCQNNINKVDSKIAEQRFGINIPHKFSIHNYKYPTFCDHCGSLLWGIMRQGLQCKICKMNVHIRCQANVAPNCGVNAVELAKTLAGMGLQPGNISPTSKLVSRSTLRRQGKESSKEGNGJGVNSSNRLGIDNFEFIRVLGKGSFOKVMLARVKETODLYAVKVLKKDVILQDDDVECTMTEKRILSLARNHPFLFQLFCGFQTPDRLFFVMEFVNGGDLMFHIQKSRRFDEARARFYAAEIISALMFLHDKGIIYRDLKLDNVLLDHEGHCKLADFGMCKEGICNGVTTATFCGTPDYIAPEILQEMLYGPAVDWWAMGVLLYEMLCGHAPFEAENEDDLFEAILNDEVVYPTWLFFEDATGILKSFMTKNPTMRLGSLTQGGEHATLRHPFFKEIDWAQLNHRQIEPPFRPRIKSREDVSNFDPDFIKEEPVLTPJDEGHLPMINQDEFRNFSYVSPELQP (SEQ ID NO: 1) oder einer Teilsequenz davon, die direkt an CTLA-4 bindet,
und das Verfahren das Inkontaktbringen von CTLA-4 mit dem Mittel umfasst.

2. In-vitro-Verfahren zum Modulieren der Funktion von regulatorischen T-Zellen, umfassend das Modulieren der Wechselwirkung von PKCη mit CTLA-4, wobei das Verfahren das Inkontaktbringen von CTLA-4 mit einem Mittel umfasst, das die direkte physische Wechselwirkung von CTLA-4 mit PKCη inhibiert, wobei das Mittel ein Protein oder Peptid ist, das umfasst oder besteht aus der PKCη-Aminosauresequenz:
MSSGTMKFNGYLRVRIGEAVGLQPTRWSLRHSLFKKGHQLLDPYLTVSVDQVRVGQTSTKQKTNKPTYNEEFCANVTDGGHLELAVFHETPLGYDHFVANCTLQFQELLRTTGASDTFEGWVDLEPEGKVFVVITLTGSFTEATLQRDRIFKHFTRKRQRAMRRRVHQINGHKPMATYLRQPTYCSHCREFIWGVFGKQGYQCQVCTCVVHKRCHHLIVTACTCQNNINKVDSKIAEQRFGLNIPHKFSIHNYKVPTFCDHCGSLLWGIMRQGLQCKlCKMNVHIRCQAMVAPNCGVNAVELAKTLAGMGLQPGNISPTSKLVSRSTLRRQGKESSKEGNGIGVNSSNRLGIDNFEFIRVLGKGSFGKVMLARVKETGDLYAVKVLKKDVILQDDDVECTMTEKRILSLARNHPFLTQLFCCFQTPDRLFFVMEFVNGGDLMFHfQKSRRFDEARARPYAAEHSALMFLHDKGnYRDLKLDNVLLDHEGHCKLADFGMCKEGICNGVTTATFCGTPDYIAPEILQEMLYGPAVDWWAMGVLLYEMLCGHAPFEAENEDDLFEAILNDEVVYPTWLHEDATGILKSFMTKNPTMRLOSLTQGGEHAILRHPFFKElDWAQLNHRQIEPPFRPRIKSREDVSNFDPDFIKEEPVLTPIDEGHLPMINQDEFRNFSYVSPELQP (SEQ ID NO: 1) oder einer Teilsequenz davon, die direkt an CTLA-4 bindet,
und dadurch die Wechselwirkung zwischen PKCη und CTLA-4 vermindert, reduziert, gesperrt, unterdrückt, begrenzt oder gesteuert wird, um die regulatorische T-Zellen-Funktion zu vermindern, zu reduzieren, zu sperren, zu unterdrücken, zu begrenzen oder zu steuern.

3. Mittel zur Verwendung nach Anspruch 1 oder In-vitro-Verfahren nach Anspruch 2, wobei PKCη an S28, S32 und S317 phosphoryliert ist.

4. Mittel zur Verwendung oder In-vitro-Verfahren nach einem der Ansprüche 1-3, wobei das Mittel an eine CTLA-4-Aminosäuresequenz bindet, die Rest 182 bis Rest 223 von CTLA-4 umfasst, aus diesem besteht oder im wesentlichen aus diesem besteht.

5. Mittel zur Verwendung oder In-vitro-Verfahren nach einem der Ansprüche 1-3, wobei das Mittel an eine CTLA-4-Aminosäuresequenz bindet, die K188, K191, K192 oder R193 von CTLA-4 aufweist.

6. Mittel zur Verwendung oder In-vitro-Verfahren nach einem der Ansprüche 1-3, wobei das Peptid
(i) Rest 28 bis Rest 317 von SEQ ID NO: 1 umfasst oder daraus besteht; und/oder
(ii) bei S28, S32 und S317 phosphoryliert ist.

## Revendications

1. Agent pour utilisation dans un procédé de traitement de cancer chez un sujet, où le procédé consiste à diminuer, réduire, inhiber, supprimer ou limiter l'interaction physique directe entre la protéine kinase C de type êta (PKCη) et la protéine 4 des lymphocytes T cytotoxiques (CTLA-4) pour diminuer, réduire, inhiber, supprimer ou limiter la fonction des cellules T régulatrices ;
où l'agent inhibe l'interaction physique directe de PKCη avec CTLA-4, où en outre l'agent est une protéine ou un peptide comprenant ou consistant en la séquence d'acides aminés de PKCη :
MSSGTMKFNGYLRVRIGEAVGLQPTRWSLRHSLFKKGHQLLDPYLTVSVDQVRVGQTSTKQKTNKPTYNEEFCANVTDGGHLELAVFHETPLGYDHFVANCTLQFQELLRTTGASDTFEGWVDLEPEGKVFVVITLTGSFTEATLQRDRIFKHFTRKRQRAMRRRVHQINGHKFMATYLRQPTYCSHCREFIWGVFGKQGYQCQVCTCVVHKRCHHLIVTACTCQNNINKVDSKIAEQRFGINIPHKFSIHNYKVPTFCDHCGSLLWGIMRQGLQCKICKMNVHIRCQANVAPNCGVNAVELAKTLAGMGLQPGNISPTSKLVSRSTLRRQGKESSKEGNGIGVNSSNRLGIDNFEFIRVLGKGSFGKVMLARVKETGDLYAVKVLKKDVILQDDDVECTMTEKRILSLARNHPFLTQLFCCFQTPDRLFFVMEFVNGGDLMFHIQKSRRFDEARARFYAAEIISALMFLHDKGIIYRDLKLDNVLLDHEGHCKLADFGMCKEGICNGVTTATFCGTPDYIAPEILQEMLYGPAVDWWAMGVLLYEMLCGHAPFEAENEDDLFEAILNDEVVYPTWLHEDATGILKSFMTKNPTMRLGSLTQGGEHAILRHPFFKEIDWAQLNHRQIEPPFRPRIKSREDVSNFDPDFIKEEPVLTPIDEGHLPMINQDEFRNFSYVSPELQP (SEQ ID NO : 1), ou une sous-séquence de celle-ci, qui se lie directement à CTLA-4, et le procédé comprenant l'étape consistant à mettre en contact CTLA-4 avec l'agent.

2. Procédé *in vitro* de modulation de la fonction des cellules T régulatrices consistant à moduler l'interaction de PKCη avec CTLA-4, le procédé comprenant l'étape consistant à mettre en contact CTLA-4 avec un agent qui inhibe l'interaction physique directe de CTLA-4 avec PKCη, où l'agent est une protéine ou un peptide comprenant ou consistant en la séquence d'acides aminés de PKCη :
MSSGTMKFNGYLRVRIGEAVGLQPTRWSLRHSLFKKGHQLLDPYLTVSVDQVRVGQTSTKQKTNKPTYNEEFCANVTDGGHLELAVFHETPLGYDHFVANCTLQFQELLRTTG ASDTFEGWVDLEPEGKVFVVITLTGSFTEATLQRDRIFKHFTRKRQRAMRRRVHQINGHKFMATYLRQPTYCSHCREFIWGVFGKQGYQCQVCTCVVHKRCHHLIVTACTCQNNINKVDSKIAEQRFGINIPHKFSIHNYKVPTFCDHCGSLLWGIMRQGLQCKICKMNVHIRCQANVAPNCGVNAVELAKTLAGMGLQPGNISPTSKLVSRSTLRRQGKESSKEGNGIGVNSSNRLGIDNFEFIRVLGKGSFGKVMLARVKETGDLYAVKVLKKDVILQDDDVECTMTEKRILSLARNHPFLTQLFCCFQTPDRLFFVMEFVNGGDLMFHIQKSRRFDEARARFYAAEIISALMFLHDKGIIYRDLKLDNVLLDHEGHCKLADFGMCKEGICNGVTTATFCGTPDYIAPEILQEMLYGPAVDWWAMGVLLYEMLCGHAPFEAENEDDLFEAILNDEVVYPTWLHEDATGILKSFMTKNPTMRLGSLTQGGEHAILRHPFFKEIDWAQLNHRQIEPPFRPRIKSREDVSNFDPDFIKEEPVLTPIDEGHLPMINQDEFRNFSYVSPELQP (SEQ ID NO : 1), ou une sous-séquence de celle-ci, qui se lie directement à CTLA-4 ;
diminuant, réduisant, inhibant, supprimant, limitant ou régulant ainsi l'interaction entre PKCη et CTLA-4 pour diminuer, réduire, inhiber, supprimer, limiter ou réguler la fonction des cellules T régulatrices.

3. Agent pour utilisation selon la revendication 1 ou procédé *in vitro* de la revendication 2, dans lequel PKCη est phosphorylée au niveau de S28, S32 et S317.

4. Agent pour utilisation ou procédé *in vitro* selon l'une quelconque des revendications 1 à 3, où l'agent se lie à une séquence d'acides aminés de CTLA-4 qui comprend, consiste ou consiste essentiellement en une séquence allant d'à peu près le résidu 182 au résidu 223 de CTLA-4.

5. Agent pour utilisation ou procédé *in vitro* selon l'une quelconque des revendications 1 à 3, où l'agent se lie à une séquence d'acides aminés de CTLA-4 ayant K188, K191, K192 ou R193 de CTLA-4.

6. Agent pour utilisation ou procédé *in vitro* selon l'une quelconque des revendications 1 à 3, où le peptide :
(i) comprend ou consiste en une séquence allant du résidu 28 au résidu 317 de la SEQ ID NO : 1 ; et/ou
(ii) est phosphorylé au niveau de S28, S32 et S317.
